# EUROPEAN PATENT SPECIFICATION

(11) **EP 2 129 653 B1**
(45) Date of publication and mention of the grant of the patent: **17.05.2017**
(21) Application number: 08735068.2
(22) Date of filing: 07.03.2008
(51) Int. Cl.: C07C 229/74, C07C 309/46, C07C 309/53, C07C 323/37, C07D 239/42, C07D 251/42, C07D 251/44, C07D 251/46, C07D 251/50, A61K 31/136, A61P 7/02, A61P 9/10

(54) **NOVEL P2Y12 RECEPTOR ANTAGONISTS**
NEUE P2Y12-REZEPTORANTAGONISTEN
NOUVEAUX ANTAGONISTES DU RÉCEPTEUR P2Y12

(30) Priority: 08.03.2007 EP 07103803
(43) Date of publication of application: 09.12.2009
(73) Proprietor: Rheinische Friedrich-Wilhelms-Universität Bonn, 53113 Bonn (DE)
(72) Inventor: MÜLLER, Christa E., 53113 Bonn (DE); BAQI, Younis, 53121 Bonn (DE)
(74) Representative: Viering, Jentschura & Partner mbB Patent- und Rechtsanwälte
(86) International application number: PCT/EP2008/002748
(87) International publication number: WO 2008/107211

(56) References cited:
- WO-A-2006/108760
- DE-C- 453 769
- GB-A- 1 131 787
- PL-B1- 104 980
- PL-B2- 109 305
- US-A- 2 081 359
- US-A- 2 870 173
- US-A- 3 135 606
- US-A- 3 941 742
- US-A- 4 043 997
- US-A- 4 501 592
- US-A1- 2007 048 247
- US-B2- 7 138 412
- M. GLÄNZEL, ET AL.: "Constitutional isomers of Reactive Blue 2 - selective P2Y-receptor antagonists?" EUROPEAN JOURNAL OF MEDICINAL CHEMISTRY, vol. 38, no. 3, March 2003 (2003-03), pages 303-312, XP004416736 EDITIONS SCIENTIFIQUE ELSEVIER, PARIS, FR ISSN: 0223-5234
- M. GLÄNZEL, ET AL.: "Structure-activity relationships of novel P2-receptor antagonists structurally related to Reactive Blue 2" EUROPEAN JOURNAL OF MEDICINAL CHEMISTRY, vol. 40, no. 12, 8 September 2005 (2005-09-08), pages 1262-1276, XP005163003 EDITIONS SCIENTIFIQUE ELSEVIER, PARIS, FR ISSN: 0223-5234
- DATABASE BEILSTEIN BEILSTEIN INSTITUTE FOR ORGANIC CHEMISTRY, FRANKFURT-MAIN, DE; 27 June 1988 (1988-06-27), XP002453746 Database accession no. Citation Number 4141686 & FISICHELLA, ET AL.: ANNALI DI CHIMICA, vol. 68, 1978, page 503, SOCIETA' CHIMICA ITALIANA, ROME, IT ISSN: 0003-4592
- DATABASE BEILSTEIN BEILSTEIN INSTITUTE FOR ORGANIC CHEMISTRY, FRANKFURT-MAIN, DE; 27 June 1988 (1988-06-27), XP002453747 Database accession no. Citation Number 5120997 & FISICHELLA, ET AL.: CHIMICA E L'INDUSTRIA, vol. 59, 1977, page 618, SOCIETA' CHIMICA ITALIANO, MILANO, IT
- DATABASE BEILSTEIN BEILSTEIN INSTITUTE FOR ORGANIC CHEMISTRY, FRANKFURT-MAIN, DE; 27 June 1988 (1988-06-27), XP002453757 & FISICHELLA, ET AL.: GAZZETTA CHIMICA ITALIANA, vol. 109, 1979, page 155, SOCIETA' CHIMICA ITALIANA, ROME, IT
- DATABASE BEILSTEIN BEILSTEIN INSTITUTE FOR ORGANIC CHEMISTRY, FRANKFURT-MAIN, DE; 27 June 1988 (1988-06-27), XP002453748 Database accession no. Citation Number 5118373 & TOKUMITSU, ET AL.: KOGYO KAGAKU ZASSHI, vol. 67, 1964, page 201, NIPPON KAGAKUKAI, TOKYO, JP
- DATABASE BEILSTEIN BEILSTEIN INSTITUTE FOR ORGANIC CHEMISTRY, FRANKFURT-MAIN, DE; 27 June 1988 (1988-06-27), XP002453759 & BESSUBEZ, ET AL.: ZHURNAL PRIKLADNOI KHIMII, vol. 21, 1948, page 1152, MAIK NAUKA: ROSSIISKAYA AKADEMIYA NAUK, RU
- DENG NANSHENG, ET AL.: "Photodegradation of dyes in aqueous solutions containing Fe(III)-hydroxy Complex II. Solar photodegradation kinetics" CHEMOSPHERE, vol. 34, no. 12, June 1997 (1997-06), pages 2725-2735, XP002453754 ELSEVIER SCIENCE, OXFORD, GB
- YU.V. VINOKUROV, ET AL.: "Transformation of sodium 1-amino-4-arylaminoanthra- quinone-2-sulphonates in an aqueous medium" JOURNAL OF ORGANIC CHEMISTRY OF THE USSR, vol. 24, no. 3, March 1988 (1988-03), pages 566-569, XP002453755 PLENUM PUBLISHING, NEW YORK, NY, US
- D. S. KEMP, ET AL.: "Peptide synthesis by prior thiol capture. 2. Design of templates for intramolecular O,N-acyl transfer. 4,6-Disubstituted dibenzofurans as optimal spacing elements" JOURNAL OF ORGANIC CHEMISTRY, vol. 51, no. 10, 16 May 1986 (1986-05-16), pages 1829-1838, XP002456195 AMERICAN CHEMICAL SOCIETY, WASHINGTON, DC, US
- M. HARFENIST: "The action of lithium aluminum hydride and of Grignard reagents on some heterocyclic beta-ketoamides. Synthesis of 1-aldehydes and 1-ketones of phenothiazine, phenoxazine, and carbazole" JOURNAL OF ORGANIC CHEMISTRY, vol. 27, no. 12, December 1962 (1962-12), pages 4326-4331, XP002456196 AMERICAN CHEMICAL SOCIETY, WASHINGTON, DC, US
- D. S. KEMP, ET AL.: "Synthesis of 1-methoxy-9-mercaptophenoxathiin and the resolved 1-[[N-[(benzyloxy)carbonyl]- L-alanyl]oxy]-9-[(methoxycarbonyl)dithio]- phenoxathiin 10-oxide diastereomers. Comments on improved methods for sulphone reduction" JOURNAL OF ORGANIC CHEMISTRY, vol. 54, no. 15, 21 July 1989 (1989-07-21), pages 3647-3651, XP002456197 AMERICAN CHEMICAL SOCIETY, WASHINGTON, DC, US
- P. POULALLION, ET AL.: "Synthèse et caracterisation d'une série de bis-9,9'-(thio-9-acridinyl)-alpha,omega-al canes" JOURNAL OF HETEROCYCLIC CHEMISTRY, vol. 23, 1986, pages 1141-1149, XP002456198 HETEROCORPORATION, PROVO, US
- V.A. LOSTUTOV, ET AL.: "Synthesis fo anthraoxaza- and anthraoxaphophorine derivatives" RUSSIAN CHEMICAL BULLETIN, vol. 44, no. 1, January 1995 (1995-01), pages 137-139, XP002456199 PLENUM PUBLISHING, NEW YORK, NY, US
- F. ULLMANN, ET AL.: "Untersuchungen in der Thioxanthon- und Benzophenonsulfon-Reihe" BERICHTE DER DEUTSCHEN CHEMISCHEN GESELLSCHAFT, vol. 49, 1916, pages 2487-2514, XP002456200 VERLAG CHEMIE, WEINHEIM, DE
- J.I.G. CADOGAN, ET AL.: "Reduction of nitro- and nitroso-compounds by tervalent phosphorus reagents. Part IX. The 'blocked ortho' effect in reactions of 2,6-disubstituted aryl 2-nitrophenyl and 2,6-disubstituted aryl 2-azidophenyl sulphides: a new series of nitrene-induced aromatic rearrangements" JOURNAL OF THE CHEMICAL SOCIETY, SECTION C: ORGANIC CHEMISTRY, 1971, pages 2621-2632, XP002456201 ROYAL SOCIETY OF CHEMISTRY LETCHWORTH, GB
- F. MAYER, ET AL.: "Umsetzung von o -halogenierten Ketonen mit schwach basischen Aminen und Aufbau von ms -Phenyl-acridin-Abkömmlingen", BERICHTE DER DEUTSCHEN CHEMISCHEN GESELLSCHAFT, vol. 55, no. 7, 8 July 1922 (1922-07-08), pages 2049-2058, XP055082799, Verlag Chemie, Weinheim, DE ISSN: 0009-2940, DOI: 10.1002/cber.19220550711
- W.M. LORD, ET AL.: "New intermediates and dyestuffs for synthetic fibres. Part V. By-products of the Ullmann condensation between 1-chloroanthraquinone and aniline", JOURNAL OF THE CHEMICAL SOCIETY, SECTION C: ORGANIC CHEMISTRY, 1971, pages 3600-3601, XP055082803, Royal Society of Chemistry, Cambridge, GB ISSN: 0022-4952, DOI: 10.1039/j39710003600
- M.G. PELATT, ET AL.: "Photostable anthraquinone pleochroic dyes", MOLECULAR CRYSTALS AND LIQUID CRYSTALS., vol. 59, 1980, pages 299-316, XP009143750, GORDON AND BREACH, LONDON. ISSN: 0026-8941, DOI: 10.1080/00268948008071430
- G. PHILIP, ET AL.: "Arylamination of aminohalogenoanthraquinones", INDIAN JOURNAL OF CHEMISTRY, vol. 22B, no. 8, August 1983 (1983-08), pages 808-811, XP008165244, Scientific Publishers, Jodhpur, IN ISSN: 0376-4699
- F. TULUC, ET AL.: "P2-receptor antagonists: IV. Blockade of P2-receptor subtypes and ecto-nucleotidases by compounds related to reactive blue 2", NAUNYN-SCHMIEDEBERG'S ARCHIVES OF PHARMACOLOGY, vol. 357, no. 2, 1 February 1998 (1998-02-01), pages 111-120, XP002662512, Springer-Verlag, Berlin, DE ISSN: 0028-1298, DOI: 10.1007/pl00005144

## Description

### Field of the Invention

The present invention lies on the field of pharmacology, drug design and organic chemistry and relates to novel P2Y₁₂ receptor antagonists useful for treating, alleviating and/or preventing diseases and disorders related to P2Y₁₂ receptor function as well as pharmaceutical compositions comprising such compounds and methods for preparing such compounds. The present invention is further directed to the use of these compounds, alone or in combination with other therapeutic agents, for the alleviation, prevention and/or treatment of diseases and disorders, especially the use as antithrombotic agents for inhibiting platelet aggregation.

### Background of the Invention

The P2 receptors are a major class of receptors in the human body (Guile, S. D. et al., Prog. Med. Chem., 2001, 38, 115; Lambrecht, G. et al., Curr. Pharm. Des, 2002, 8, 2371; Gao, Z. G., Jacobson, K. A. Curr. Top. Med. Chem., 2004, 4, 855; Burnstock, G. Curr. Top. Med. Chem., 2004, 4, 793-803). They are subdivided into two main groups: G protein-coupled or "metabotropic" P2 receptors, designated P2Y, and ligand-gated ion channels or "ionotropic" receptors, termed P2X (Khakh, B.S. et al., Pharmacol. Rev. 2001, 53, 107-118; Abbracchio, M.P. et al., Pharmacol. Rev. 2006, 58, 281-341; Jacobson, K. A.; Jarvis, M.F.; Williams, M. J. Med. Chem. 2002, 45, 4057-4093; Müller, C.E. Curr. Pharm. Design 2002, 8, 2353-69). Currently, there are seven subtypes in the P2X family and 8 subtypes in the P2Y family (P2Y_{1,2,4,6,11,12,13,14}).

Blood platelets express one P2X receptor subtype, P2X₁, activated by ATP, and two P2Y receptor subtypes, P2Y₁ and P2Y₁₂, both of which are activated by the nucleotide ADP, which induces platelet aggregation (Gachet C., Annu. Rev. Pharmacol. Toxicol. 2006, 46, 277-300).

The combined action of both P2Y receptor subtypes on thrombocytes is required for a full aggregation response following stimulation by ADP. P2Y₁, coupled to the heterotrimeric GTP-binding protein G_{q} and phospholipase C*_{β}* is responsible for the mobilization of calcium ions from internal stores mediating platelet shape change and the initial wave of rapidly reversible platelet aggregation induced by ADP. P2Y₁₂, on the other hand, is coupled to inhibition of adenylate cyclase through Gᵢ protein and mediates a progressive and sustained aggregation not preceded by shape change. The latter receptor also plays an important role in the potentiation of platelet secretion induced by several agonists, and its congenital deficiency has been shown to result in a lifelong bleeding disorder (Leon, C. et al. Thromb. Haemost. 1999, 81: 775-781; Cattaneo, M., Gachet, C. Arterioscler. Thromb. Vasc. Biol. 1999, 19: 2281-2285).

Modulation of P2 receptors in platelets appears to be of paramount importance in regulating platelet function, and, as a consequence, in controlling thrombotic diseases, which are the most common cause of morbidity and mortality in the Western world. The P2Y₁₂ receptor shows a limited expression in the human body. It is expressed in very high density on blood platelets (El-Tayeb, A., Griessmeier, K.J, Mueller, C.E. Bioorg. Med. Chem. Lett. 2005, 15: 5450-52). Apart from that, it shows a limited expression pattern (no or only low expression in peripheral tissues and cells, some expression in the brain) making it an ideal drug target for selective therapeutic intervention (Barnard E.A., Simon, J. Trends Pharmacol. Sci. 2001, 22: 388-391).

The P2Y₁₂ receptor on blood platelets is the target of the antithrombotic drugs ticlopidine and clopidogrel, and the newly developed analog prasugrel (Hollopeter, G. et al., Nature 2001, 409, 202-207; Zangh et al., J. Biol. Chem. 2001, 276: 8608-8615; Takasaki, J. et al., Mol. Pharmacol. 2001, 60: 432-439), representing platelet aggregation inhibitors that are effective in the prevention and treatment of arterial thrombosis. Ticlopidine, clopidogrel and prasugrel are no direct P2Y₁₂ antagonists. They have to be bioactivated by cytochrome P450 enzymes (oxidation) followed by ring-opening to the corresponding highly unstable thiol derivatives, which are believed to covalently react with the receptor protein forming a disulfide bond, and thus presumably act as covalent, possibly allosteric antagonists at P2Y₁₂ receptors (Savi, P. et al., Thromb. Haemost. 2000, 84: 891-6; Sugidachi, A. et al., Br. J. Pharmacol. 2000, 129: 1439-1446).

Major drawbacks of clopidogrel and related thienotetrahydropyridine derivatives are:
(i) slow onset of action (up to several days) due to the required metabolism;
(ii) long duration of action due to irreversible inhibition;
(iii) "drug resistance" in a high percentage of patients (up to 30%);
(iv) moderate potency, therefore high doses required;
(v) difficulties in steering and controlling the effects.

Therefore, it is highly desirable to develop P2Y₁₂ antagonists that are lacking the drawbacks associated with the standard P2Y₁₂ antagonists such as clopidogrel and other thienopyridine derivatives.

Several companies have recently been developing competitive, reversible P2Y₁₂ antagonists that may be superior to clopidogrel and related drugs. Most approaches started from adenine nucleotides (ATP, ADP) as lead compounds. Astra-Zeneca developed a series of ATP analogs (since ATP is an antagonist at P2Y₁₂ receptors), including cangrelor (AR-C66096MX) and AR-C67085XX (Ingall, A.H. et al., J. Med. Chem. 1999, 42: 213-220; Boeynaems, J.-M. et al., Curr. Opin. Invest. Drugs 2005, 6: 275-282). These compounds are very polar since they are negatively charged at a physiologic pH value of 7.4, and therefore cannot be perorally applied. In addition, they are metabolically unstable due to hydrolysis by nucleotide pyrophosphatases and therefore exhibit only a very short half-life in vivo. Furthermore, they are difficult to synthesize and in particular difficult to purify in large amounts that are required for use as drugs. Their selectivity for P2Y₁₂ receptors is unclear, since they may also bind to other nucleotide binding sites.

K.A. Jacobson and colleagues described a series of adenosine bisphosphate derivatives, e.g. MRS-2395, as P2Y₁₂ antagonists (Jacobson, K.A. et al., Curr. Top. Med. Chem. 2004, 4, 805-819), however with very moderate potency. In addition, the ester groups in the molecules may be metabolically unstable.

Inspire Pharmaceuticals Inc. developed nucleoside 5'-monophosphates and related compounds as P2Y₁₂ antagonists for intravenous application, e.g. for the treatment of postoperative bleeding and for blood product transfusion medicinal applications (see, for example, international patent publication WO 2006/119507). INS50589 has been evaluated in initial clinical trials (Johnson, F.L. et al., J. Thromb. Haemost. 2005, 3 (Suppl. 1), P2206). The compounds are again not perorally applicable and have very short half-lives due to enzymatic dephosphorylation leading to inactive nucleosides or nucleoside analogs.

An important progress was achieved by the development of the nucleoside analog AZD6140 and related compounds, which are perorally active P2Y₁₂ antagonists, and AZD6140 is currently developed for clinical application as an antithrombotic agent (Tantry, U.S.; Bliden, K.P.; Gurberl, P.A. Expert Opin. Investig. Drugs 2007, 16, 225-9; van Giezen, J.J.J., Humphries, R.G. Seminars in thrombosis and hemostasis 2005, 31, 195-204). However, the drug molecule is stereochemically sophisticated and requires a demanding, multi-step synthesis. Furthermore, recent studies have indicated dyspnoea as a possible side-effect of AZD6140 as well as other adenosine-related nucleoside and nucleotide derivatives and analogs due to the formation of adenosine receptor-activating metabolites (Serebruany, V.L., Stebbing, J., Atar, D. Int. J. Clin. Pract. 2007, 61, 529-33).

Screening strategies have yielded P2Y₁₂ antagonists that are not structurally related to nucleotides or nucleosides. An example is CT-50547, a benzothiazolo[2,3-c]thiadiazine derivative, which exhibits affinity for the human platelet P2Y₁₂ receptor of greater than 100 nM (Scarborough RM et al. Bioorg. Med. Chem. Lett. 11, 1805-08, 2001; Scarborough RM et al., EP 1734041A2, 2006, and related patents). Two recent patent applications described other groups of non-nucleotide platelet ADP receptor antagonists (US patent application 2005/0065163 and international patent publication WO 2005/000281), however no pharmacological data are given in the US patent application.

The anthraquinone derivative Reactive Blue 2 (RB-2) has been found to bind to a variety of nucleotide-binding proteins in the human body, including a number of different P2 receptor subtypes (Burnstock G. Curr Top. Med. Chem. 2004, 4, 793-803). Several publications indicated that RB-2 was able to antagonize P2Y₁₂ receptor activation and may thus be acting as a direct P2Y₁₂ receptor antagonist (Unterberger, U. et al., Br. J. Pharmacol. 2002, 135, 673-84; Kulick, M.B.; von Kügelgen, I, J. Pharmacol. Exp. Ther. 2002, 303, 520-6).

Hence, a number of RB-2 derivatives were synthesized and investigated in functional studies at P2Y₁ and P2X receptors (Tuluc, F. et al., Naunyn Schmiedebergs Arch. Pharmacol., 1998, 357, 111. Glänzel, M. et al., Eur. J Med. Chem., 2003, 38, 303. Glänzel, M. et al. Eur. J. Med. Chem. 2005, 40, 1262). However, no data were published on their potency at the P2Y₁₂ receptor subtype. In a recent review article RB-2 was described as a P2Y₁₂ antagonist with a Kᵢ value of 1.3 µM (Abbracchio, M.P. et al., Pharmacol. Rev. 2006, 58, 281-341).

Moreover, document US 7,138,412 B2 discloses polycyclic compounds, which are useful in the treatment of thromboembolic disorders such as thrombosis.

However, although one anthraquinone derivative, RB-2, has shown promise as P2Y₁₂ antagonist, the synthesis of analogs and the development of this class of compounds has been hampered to date by the available synthesis procedures that require harsh conditions e.g., high temperatures and long reaction times, and suffer from mostly poor yields.

The efforts aiming in the direction of developing P2Y₁₂ antagonists show that there is still need in the art for compounds that act as specific P2Y₁₂ inhibiting agents and thus may be useful as antithrombotic agents for the inhibition of platelet aggregation. Since the P2Y₁₂ receptor is also expressed in brain, e.g. in microglial cells (Hayes S.E. et al. Nature Neuroscience 2006, 9, 1512-1519), specific P2Y₁₂ antagonists may, in addition to their use as anti-thrombotics, be used to prevent or treat CNS disorders, including neuroinflammatory conditions and neurodegenerative disorders (e.g. Alzheimer's and Parkinson's disease).

Hence, it was an objective of the present invention to provide compounds that act as potent and selective P2Y₁₂ antagonists and thus may be useful as antithrombotics and avoid essentially all of the above-mentioned drawbacks of the known drugs.

Moreover, it was an objective of the present invention to provide methods for preparing said compounds. It was furthermore an objective of the present invention to provide compounds and pharmaceutical formulations for the treatment, alleviation and/or prevention of a host of diseases and disorders connected to P2Y₁₂ function. It was a further objective of the present invention to provide the use of these compounds for alleviating, preventing and/or treating diseases and disorders connected to P2Y₁₂ function, particularly for, but not limited to the use as antithrombotic agents by inhibiting platelet aggregation.

### Summary of the Invention

The present invention is directed to certain or anthracene derivatives or heterocyclic analogs thereof which act as P2Y₁₂ antagonists and are therefore useful for inhibiting platelet aggregation.

In a first aspect, the invention is directed to a compound
(A) according to Formula I: 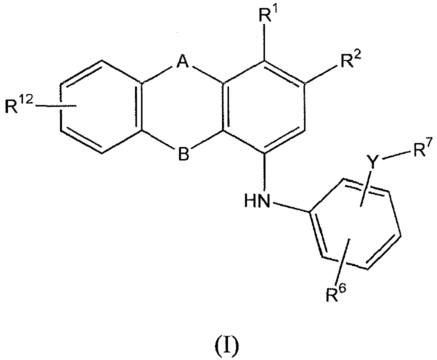 wherein:
   A and B are independently selected from the group consisting of CH₂, O, S, NH, C=O, C=NH, C=S, or C=N-OH;
   R¹ is independently selected from the group consisting of hydrogen, unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -C(O)R, -C(O)OR, -C(O)NRR', -NRR', - S(O)₂R, -S(O)₂OR, and -S(O)₂NRR';
   R² is independently selected from the group consisting of unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -C(O)R, -C(O)OR, -C(O)NRR', -NRR', - S(O)₂R, -S(O)₂OR, and -S(O)₂NRR';
   R⁶ represents substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5-to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR, -C(O)NRR', - S(O)₂R, -S(O)₂OR, and -S(O)₂NRR wherein in the meta position of the aryl ring R⁶ is a tetrazolyl, sulfonic acid or carboxylic acid group or the respective sodium or potassium salt;
   Y is selected from the group consisting of NH, O, S, CH₂, CH₂CH₂, C(O), C(O)O, or S(O)₂O;
   R⁷ is unsubstituted C₆-C₁₄ aryl;
   R¹² represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR, -C(O)NRR', - S(O)₂R, -S(O)₂OR, and -S(O)₂NRR'; and
   R and R' are independently selected from the group consisting of hydrogen and unsubstituted C₁-C₄ alkyl;
   or a pharmaceutically acceptable salt or thereof,
   wherein if the C₁-C₁₀ alkyl is substituted, the substituent group(s) is one or more, for example one or two groups, individually selected from the group consisting of C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰ R¹¹ where R¹⁰ and R¹¹ are independently selected from the group consisting of hydrogen, C₁-C₄ alkyl, C₃-C₈ cycloalkyl, C₆-C₁₄ aryl, carbonyl, acetyl, sulfonyl, amino, and trifluoromethanesulfonyl, or R¹⁰ and R¹¹, together with the nitrogen atom to which they are attached, combine to form a five-or six-membered heteroalicyclic ring,
   wherein if the C₃-C₈ cycloalkyl is substituted, the substituent group(s) is one or more, for example one or two groups, individually selected from C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and NR¹⁰ R¹¹ with R¹⁰ and R¹¹ as defined above,
   wherein if the C₆-C₁₄ aryl is substituted, the substituted group(s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and NR¹⁰ R¹¹ with R¹⁰ and R¹¹ as defined above,
   wherein if the 5- to 10-membered heteroaryl is substituted, the substituted group(s) is one or more, for example one or two substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰R¹¹ with R¹⁰ and R¹¹ as defined above, and
   wherein if the 5- to 10-membered heteroalicyclic ring is substituted the the substituted group (s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰ R¹¹ with R¹⁰ and R¹¹ as defined above;
(B) selected from the group consisting of: 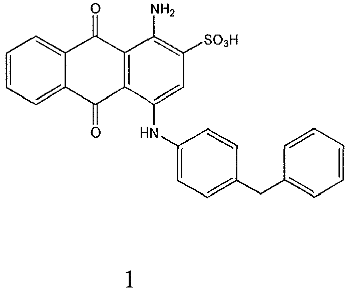 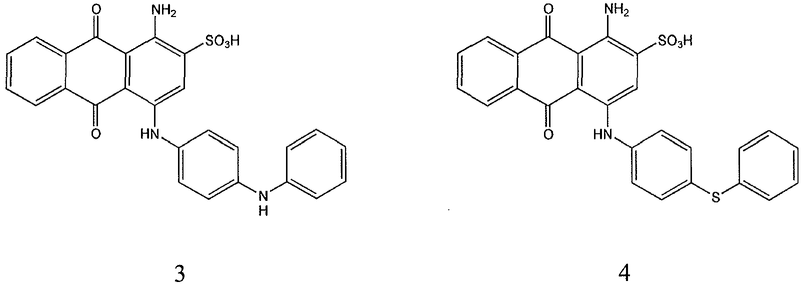 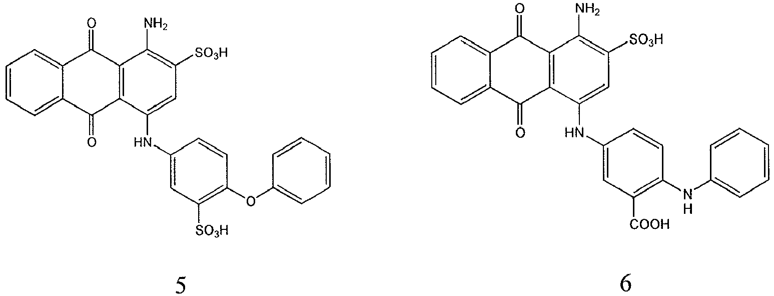 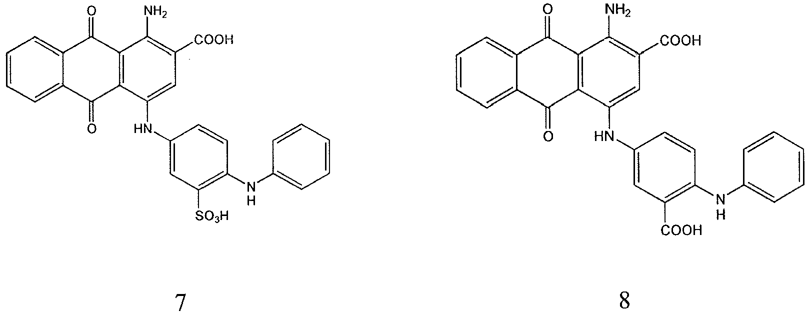 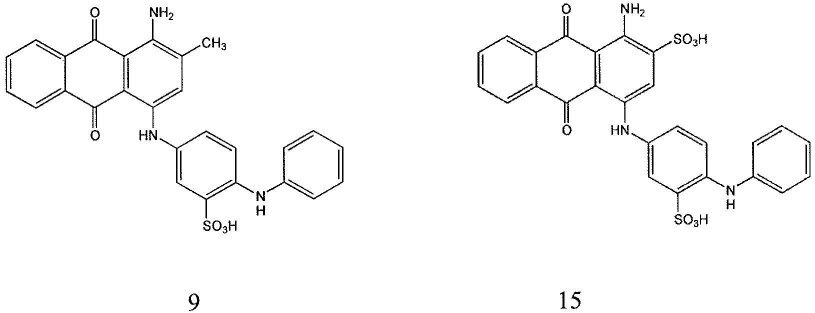 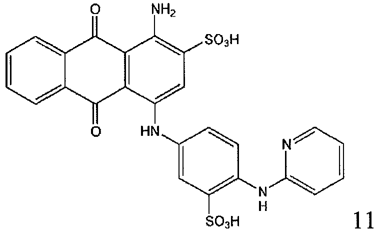 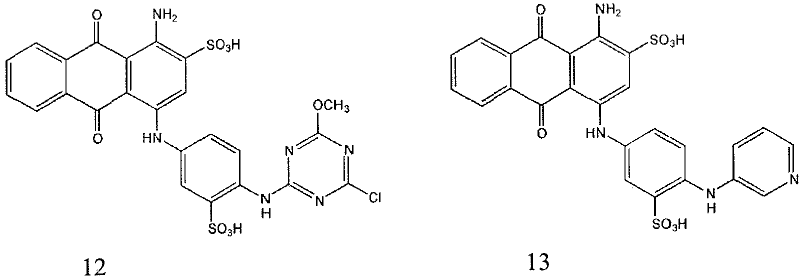 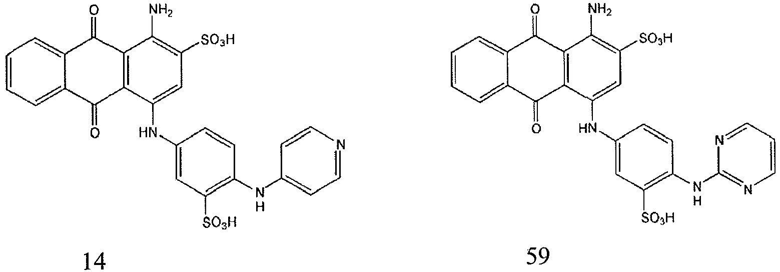 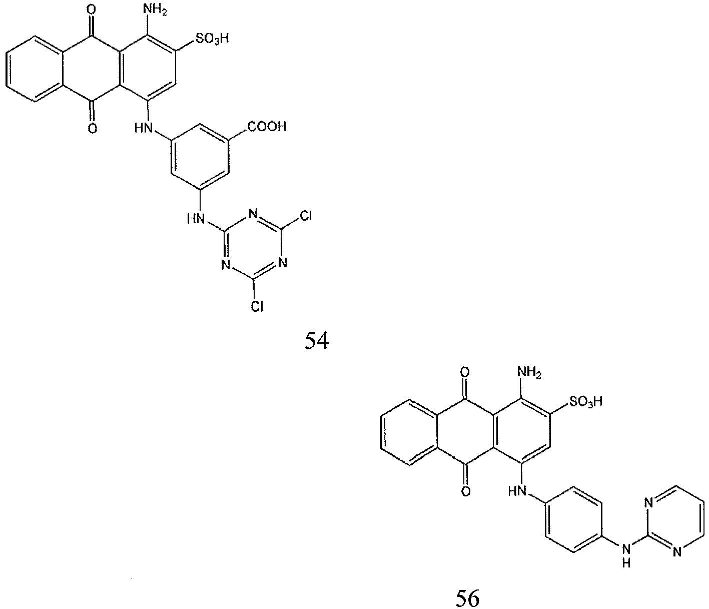 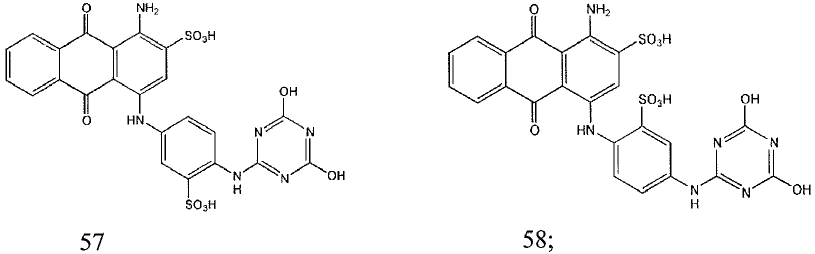 or pharmaceutically acceptable salts thereof; or
(C) selected from the group consisting of: 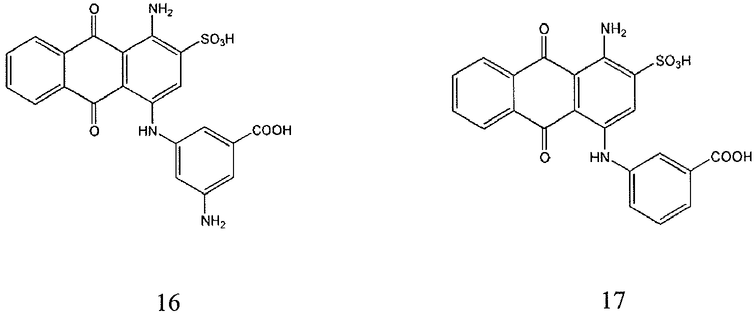 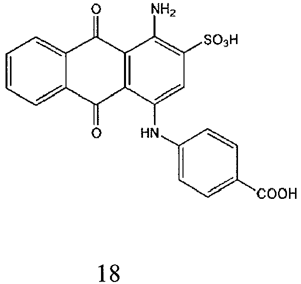 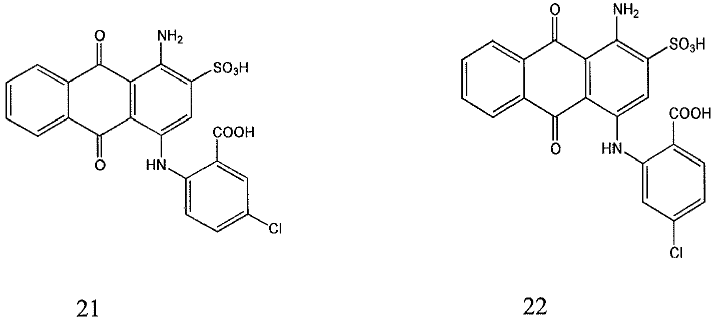 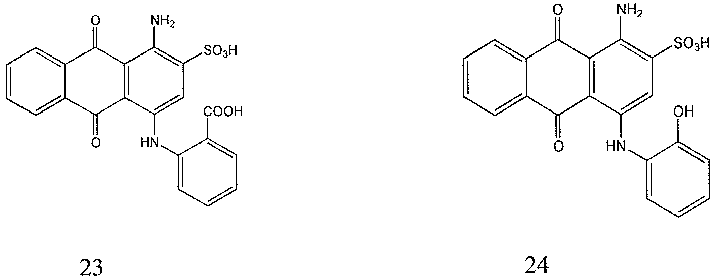 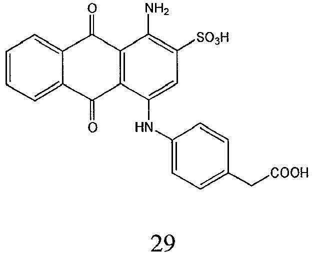 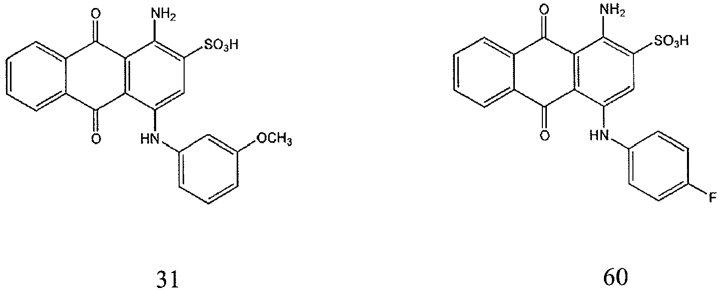 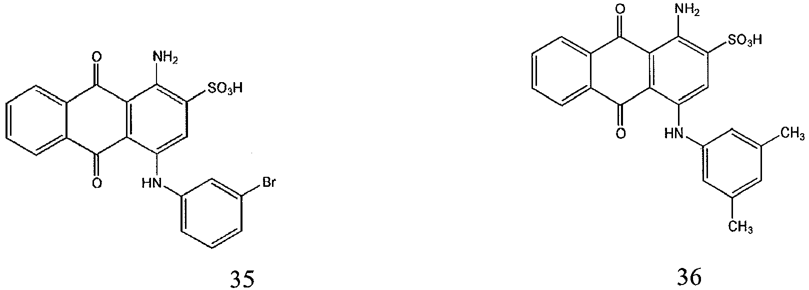 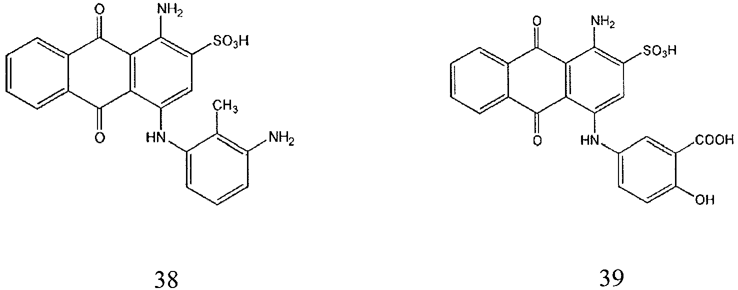 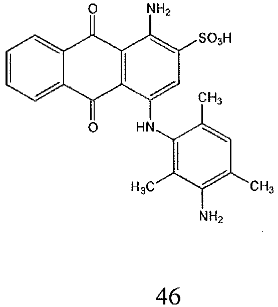 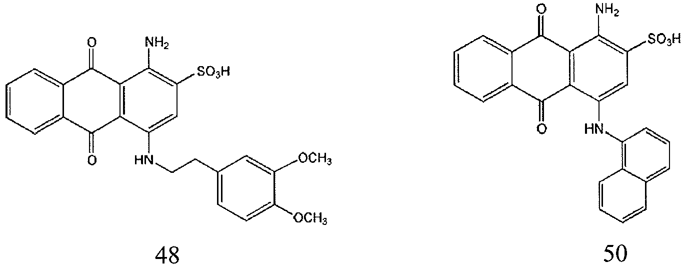 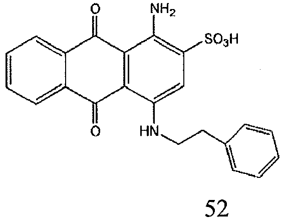
or pharmaceutically acceptable salts thereof.

In certain embodiments, R⁷ is unsubstituted C₆ aryl.

In certain embodiments of the compounds of Formula I, Y is NH and/or wherein R⁶ represents one to four substituents selected from the group consisting of hydrogen, -S(O)₂OR, -C(O)OR, tetrazolyl, sulfonyl, carboxy, amino, halogen, C₁-C₄ alkoxy, C₁-C₄ alkyl, hydroxy, and nitro.

### In certain embodiments

(1) R¹² represents one to four substituents selected from the group consisting of hydrogen, halo, amino, unsubstituted C₁-C₄ alkyl or alkoxy, or hydroxy; and/or
(2) at least one of A and B is C=O or both A and B are C=O; and/or
(3) R¹ is amino; and/or
(4) R² is tetrazolyl, -S(O)₂OR or -C(O)OR

In another aspect, the present invention relates to a pharmaceutical composition comprising any of the compounds or salts of the present invention and, optionally, a pharmaceutically acceptable carrier or excipient. This composition may additionally comprise further compounds or medicaments, such as, for example, thrombolytic agents or antithrombotics besides the invented compounds.

In one aspect, the present invention relates to a compound of the invention according to alternative (A) or (B) or a pharmaceutically acceptable salt thereof for use in a method of inhibiting platelet aggregation or for use in the prevention, alleviation and/or treatment of a disease or disorder related to P2Y₁₂ receptor activity.

In still another aspect, the present invention is directed to a compound according to Formula III: 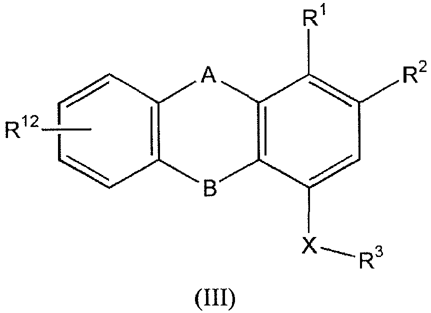 wherein:
A and B are C=O;
X is NH;
R¹ is -NH₂;
R² is selected from the group -C(O)OH and -S(O)₂OH;
R³ is selected from the group consisting of unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5-to 10-membered heteroalicyclic ring wherein 1 to 3 ring atoms are independently nitrogen, oxygen or sulfur, alkylaryl, alkylheteroaryl, an unsubstituted or substituted 7 to 12-membered bicyclic alkyl or heterocyclic ring wherein 1 to 3 ring members are independently nitrogen, oxygen or sulfur, an unsubstituted or substituted 10 to 16-membered tricyclic alkyl or heterocyclic ring wherein 1 to 3 ring members are independently nitrogen, oxygen or sulfur, 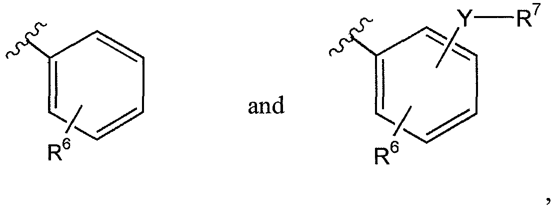
R⁶ represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR , -C(O)NRR', - S(O)₂R, -S(O)₂OR, and -S(O)₂NRR';
Y is selected from the group consisting of hydrogen, halogen, NH, O, S, CH₂, CH₂CH₂, C(O), C(O)O, S(O)₂O, or unsubstituted C₁-C₄ alkoxy, wherein when Y is hydrogen, halogen or alkoxy R⁷ is missing;
R⁷ is selected from the group consisting of hydrogen, halogen, -OR, unsubstituted or substituted C₁-C₁₀ alkyl, alkenyl or alkynyl, unsubstituted or substituted C₁-C₁₀ alkoxy, unsubstituted or substituted C₃-C₈ cycloalkoxy, unsubstituted or substituted C₃-C₈ cycloalkyl, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic wherein 1 to 3 ring atoms are independently nitrogen, oxygen or sulfur, - C(O)R, -C(O)OR , -C(O)NRR', -NRR', - S(O)₂R, -S(O)₂OR, -S(O)₂NRR', and -P(O)RR';
R¹² represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR, -C(O)NRR', - S(O)₂R, -S(O)₂OR, and -S(O)₂NRR'; and
R and R' are independently selected from the group consisting of hydrogen and unsubstituted C₁-C₄ alkyl;
or a pharmaceutically acceptable salt thereof for use in the the prevention, alleviation and/or treatment of a disease or disorder related to P2Y₁₂ receptor activity,
wherein if the C₁-C₁₀ alkyl is substituted, the substituent group(s) is one or more, for example one or two groups, individually selected from the group consisting of C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰ R¹¹ where R¹⁰ and R¹¹ are independently selected from the group consisting of hydrogen, C₁-C₄ alkyl, C₃-C₈ cycloalkyl, C₆-C₁₄ aryl, carbonyl, acetyl, sulfonyl, amino, and trifluoromethanesulfonyl, or R¹⁰ and R¹¹, together with the nitrogen atom to which they are attached, combine to form a five-or six-membered heteroalicyclic ring,
wherein if the C₃-C₈ cycloalkyl is substituted, the substituent group(s) is one or more, for example one or two groups, individually selected from C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, 0-carboxy, nitro, silyl, sulfinyl, sulfonyl, amino, and NR¹⁰ R¹¹ with R¹⁰ and R¹¹ as defined above,
wherein if the C₆-C₁₄ aryl is substituted, the substituted group(s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and NR¹⁰ R¹¹ with R¹⁰ and R¹¹ as defined above,
wherein if the 5- to 10-membered heteroaryl is substituted, the substituted group(s) is one or more, for example one or two substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰ R¹¹ with R¹⁰ and R¹¹ as defined above, and
wherein if the 5- to 10-membered heteroalicyclic ring is substituted the the substituted group (s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰ R¹¹ with R¹⁰ and R¹¹ as defined above.

In another embodiment, the compound for use in the prevention, alleviation and/or treatment of a disease or disorder related to P2Y12 receptor activity is selected from the group consisting of: 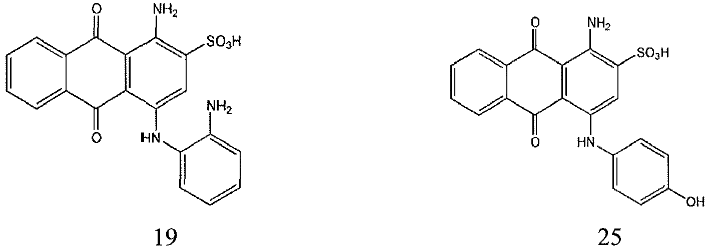 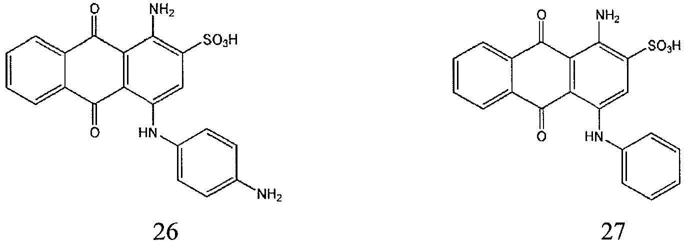 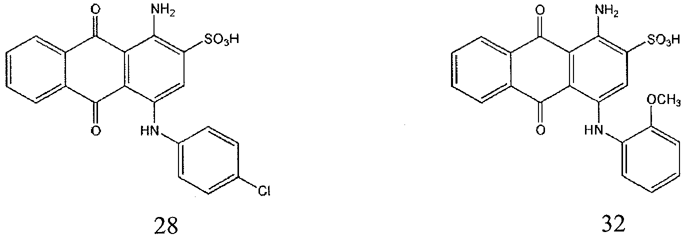 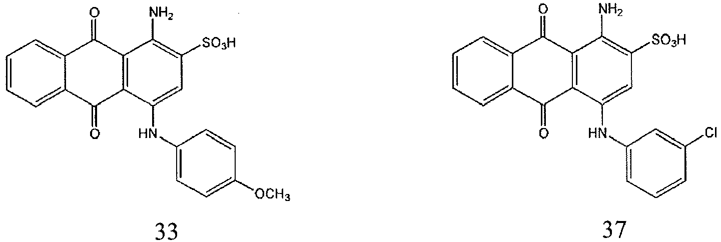 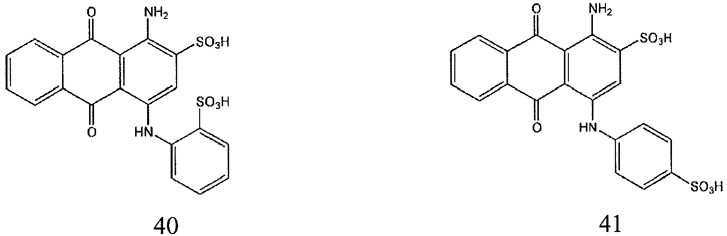 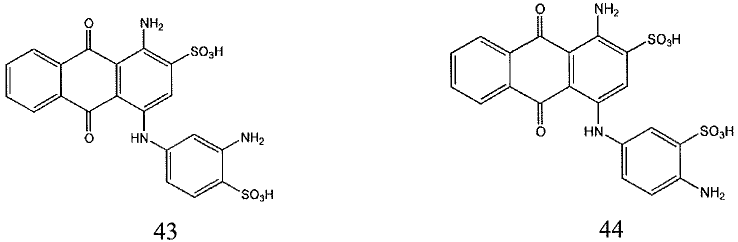 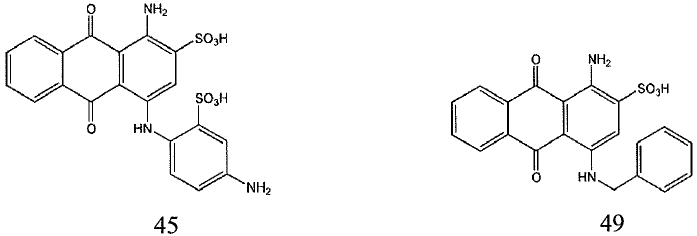 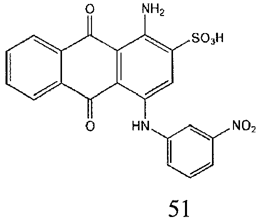 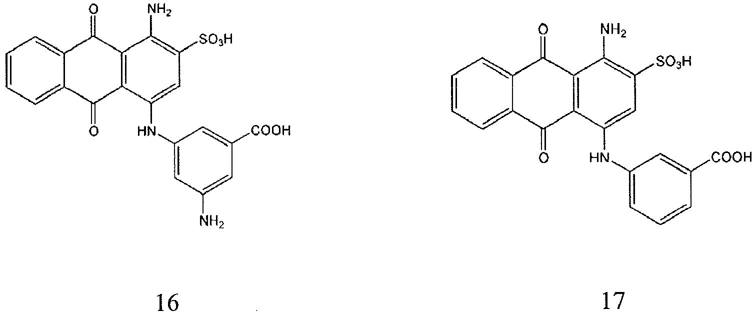 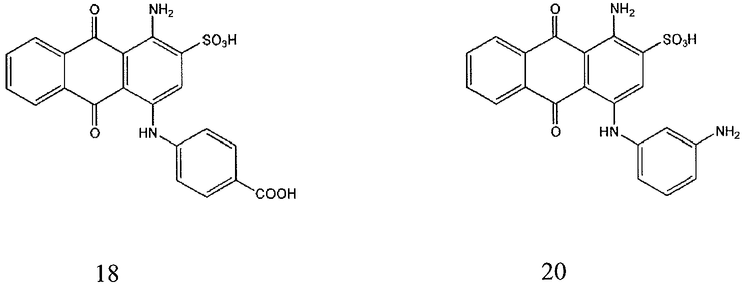 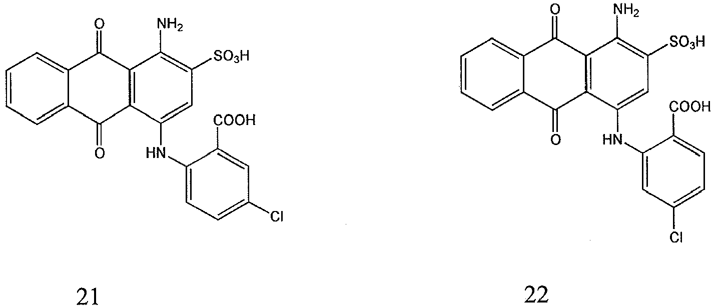 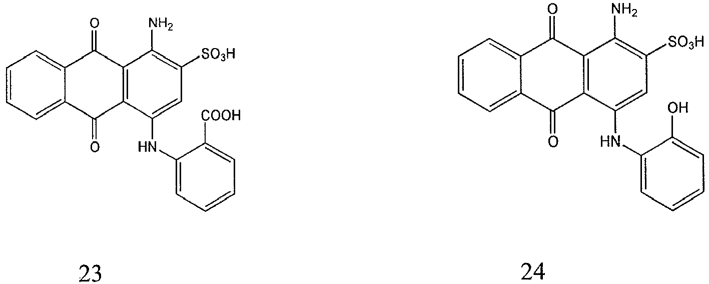 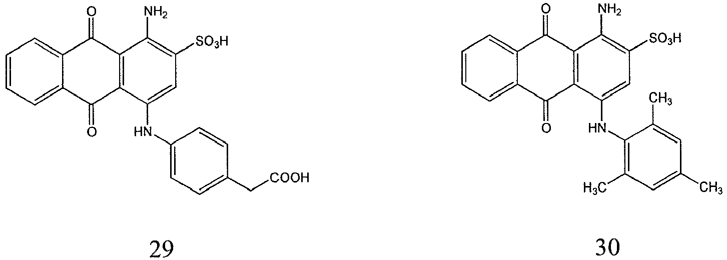 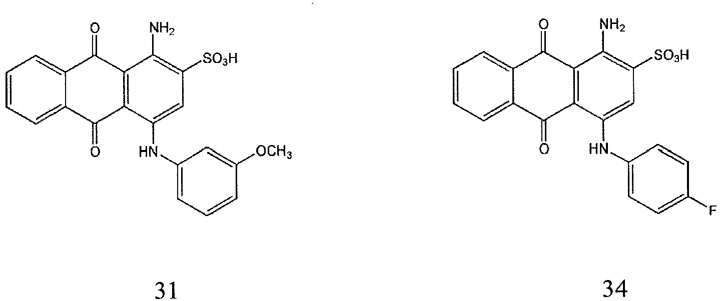 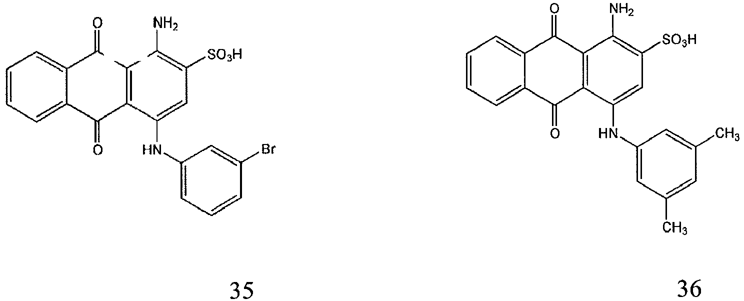 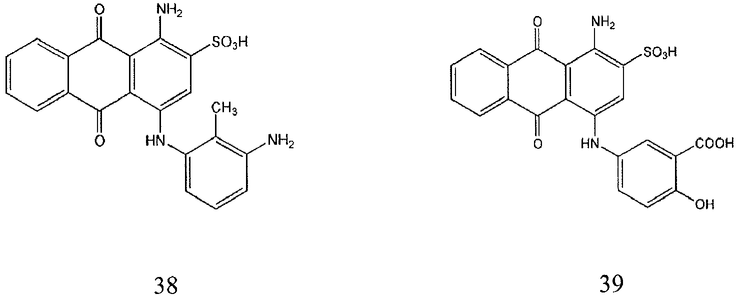 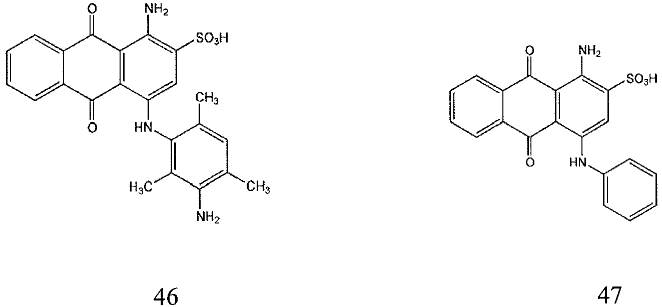 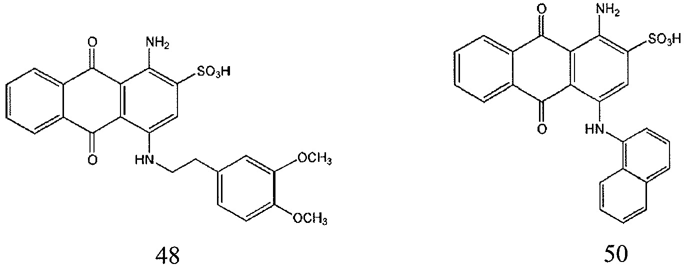 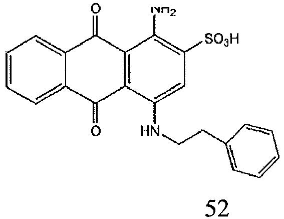 or pharmaceutically acceptable salts thereof.

In a further aspect, the disease or disorder is selected from the group of stroke, myocardial infarction, myocardial ischemia, vascular diseases, coronary artery disease, peripheral artery disease, atherosclerosis, cerebrovascular disease, embolisms and CNS disorders.

In still another aspect, the present invention is directed an in vitro method for antagonizing the P2Y₁₂ receptor function comprising the step of contacting cells expressing a P2Y₁₂ receptor with at least one compound as defined above.

### Brief description of the drawings

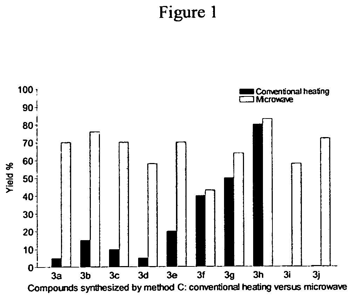

Figure 1 shows the results of the copper-catalyzed synthesis of an anilinoanthraquinone according to the present invention with microwave irradiation compared to conventional heating.

### Detailed Description of the Invention

### Definitions

Unless otherwise stated the following terms used in the specification and claims have the meanings discussed below:

"Alkyl" refers to a saturated aliphatic hydrocarbon including straight chain, or branched chain groups. Preferably, the alkyl group has 1 to 10 carbon atoms (whenever a numerical range; e.g.,"1-10", is stated herein, it means that the group, in this case the alkyl group, may contain 1 carbon atom, 2 carbon atoms, 3 carbon atoms, etc. up to and including 10 carbon atoms). More specifically, it may be a medium size alkyl having 1 to 6 carbon atoms or a lower alkyl having 1 to 4 carbon atoms e. g., methyl, ethyl, n-propyl, isopropyl, butyl, iso-butyl, tert-butyl and the like. The alkyl group may be substituted or unsubstituted. When substituted, the substituent group(s) is one or more, for example one or two groups, individually selected from the group consisting of C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰R¹¹ where R¹⁰ and R¹¹ are independently selected from the group consisting of hydrogen, C₁-C₄ alkyl, C₃-C₈ cycloalkyl, C₆-C₁₄ aryl, carbonyl, acetyl, sulfonyl, amino, and trifluoromethanesulfonyl, or R¹⁰ and R¹¹, together with the nitrogen atom to which they are attached, combine to form a five-or six-membered heteroalicyclic ring.

A "cycloalkyl" group refers to an all-carbon monocyclic ring (i.e., rings which share an adjacent pair of carbon atoms) of 3 to 8 ring atoms wherein one of more of the rings does not have a completely conjugated pi-electron system e.g., cyclopropyl, cyclobutyl, cyclopentyl, cyclohexyl, cyclobutenyl, cyclopentenyl, cyclohexenyl, and the like. Examples, without limitation, of cycloalkyl groups are cyclopropane, cyclobutane, cyclopentane, cyclopentene, cyclohexane, adamantane, cyclohexadiene, cycloheptane and, cycloheptatriene. A cycloalkyl group may be substituted or unsubstituted. When substituted, the substituent group(s) is one or more, for example one or two groups, individually selected from C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰R¹¹, with R¹⁰ and R¹¹ as defined above.

An "alkenyl" group refers to an alkyl group, as defined herein, consisting of at least two carbon atoms and at least one carbon-carbon double bond e. g., ethenyl, propenyl, butenyl or pentenyl and their structural isomeric forms such as 1-or 2-propenyl, 1-, 2-, or 3-butenyl and the like.

An "alkynyl" group refers to an alkyl group, as defined herein, consisting of at least two carbon atoms and at least one carbon-carbon triple bond e. g., acetylene, ethynyl, propynyl, butynyl, or pentynyl and their structural isomeric forms as described above.

An "aryl" group refers to an all-carbon monocyclic or fused-ring polycyclic (i.e., rings which share adjacent pairs of carbon atoms) groups of 6 to 14 ring atoms and having a completely conjugated pi-electron system. Examples, without limitation, of aryl groups are phenyl, naphthalenyl and anthracenyl. The aryl group may be substituted or unsubstituted. When substituted, the substituted group(s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰R¹¹, with R¹⁰ and R¹¹ as defined above. Preferably the substituent(s) is/are independently selected from chloro, fluoro, bromo, methyl, ethyl, hydroxy, methoxy, nitro, carboxy, methoxycarbonyl, sulfonyl, or amino.

A "heteroaryl" group refers to a monocyclic or fused aromatic ring (i.e., rings which share an adjacent pair of atoms) of 5 to 10 ring atoms in which one, two, three or four ring atoms are selected from the group consisting of nitrogen, oxygen and sulfur and the rest being carbon. Examples, without limitation, of heteroaryl groups are pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-triazinyl, 1,2,3-triazinyl, benzofuryl, isobenzofuryl, benzothienyl, benzotriazolyl, isobenzothienyl, indolyl, isoindolyl, 3H-indolyl, benzimidazolyl, benzothiazolyl, benzoxazolyl, quinolizinyl, quinazolinyl, pthalazinyl, quinoxalinyl, cinnnolinyl, napthyridinyl, quinolyl, isoquinolyl, tetrazolyl, 5,6,7,8-tetrahydroquinolyl, 5, 6, 7, 8-tetrahydroisoquinolyl, purinyl, pteridinyl, pyridinyl, pyrimidinyl, carbazolyl, xanthenyl or benzoquinolyl. The heteroaryl group may be substituted or unsubstituted. When substituted, the substituted group(s) is one or more, for example one or two substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰R¹¹, with R¹⁰ and R¹¹ as defined above. Preferably the substituent(s) is/are independently selected from chloro, fluoro, bromo, methyl, ethyl, hydroxy, methoxy, nitro, carboxy, methoxycarbonyl, sulfonyl, or amino.

A "heteroalicyclic" group refers to a monocyclic or fused ring of 5 to 10 ring atoms containing one, two, or three heteroatoms in the ring which are selected from the group consisting of nitrogen, oxygen and -S(O)ₙ where n is 0-2, the remaining ring atoms being carbon. The rings may also have one or more double bonds. However, the rings do not have a completely conjugated pi-electron system. Examples, without limitation, of heteroalicyclic groups are pyrrolidine, piperidine, piperazine, morpholine, imidazolidine,tetrahydropyridazine, tetrahydrofuran, thiomorpholine, tetrahydropyridine, and the like. The heteroalicyclic ring may be substituted or unsubstituted. When substituted, the substituted group (s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰R¹¹, with R¹⁰ and R¹¹ as defined above. The substituent(s) is/are for example independently selected from chloro, fluoro, bromo, methyl, ethyl, hydroxy, methoxy, nitro, carboxy, methoxycarbonyl, sulfonyl, or amino.

A "hydroxy" group refers to an -OH group.

An "alkoxy" group refers to an -O-unsubstituted alkyl and -O-substituted alkyl group, as defined herein. Examples include and are not limited to methoxy, ethoxy, propoxy, butoxy, and the like.

A "cycloalkoxy" group refers to an -O-cycloalkyl group, as defined herein. One example is cyclopropyloxy.

An "aryloxy" group refers to both an -O-aryl and an -O-heteroaryl group, as defined herein. Examples include and are not limited to phenoxy, napthyloxy, pyridyloxy, furanyloxy, and the like.

A "mercapto" group refers to an -SH group.

An "alkylthio" group refers to both an S-alkyl and an -S-cycloalkyl group, as defined herein. Examples include and are not limited to methylthio, ethylthio, and the like.

An "arylthio" group refers to both an -S-aryl and an -S-heteroaryl group, as defined herein. Examples include and are not limited to phenylthio, napthylthio, pyridylthio, furanylthio, and the like.

A "sulfinyl" group refers to a -S(O)-R" group, wherein, R" is selected from the group consisting of hydrogen, hydroxy, alkyl, cycloalkyl, aryl, heteroaryl (bonded through a ring carbon) and heteroalicyclic (bonded through a ring carbon), as defined herein.

A "sulfonyl" group refers to a -S(O)₂R" group wherein, R" is selected from the group consisting of hydrogen, hydroxy, alkyl, cycloalkyl, aryl, heteroaryl (bonded through a ring carbon) and heteroalicyclic (bonded through a ring carbon), as defined herein.

A "trihalomethyl" group refers to a -CX₃ group wherein X is a halo group as defined herein e. g., trifluoromethyl, trichloromethyl, tribromomethyl, dichlorofluoromethyl, and the like.

"Carbonyl" refers to a -C(=O)-R" group, where R" is selected from the group consisting of hydrogen, alkyl, cycloalkyl, aryl, heteroaryl (bonded through a ring carbon) and heteroalicyclic (bonded through a ring carbon), as defined herein. Representative examples include and the not limited to acetyl, propionyl, benzoyl, formyl, cyclopropylcarbonyl, pyridinylcarbonyl, pyrrolidin-lylcarbonyl, and the like.

A "thiocarbonyl" group refers to a -C(=S)-R" group, with R" as defined herein.

"C-carboxy" and "carboxy" which are used interchangeably herein refer to a -C(=O)O-R" group, with R" as defined herein, e. g. -COOH, methoxycarbonyl, ethoxycarbonyl, benzyloxycarbonyl, and the like.

An "O-carboxy" group refers to a -OC(=O)R" group, with R" as defined herein, e.g. methylcarbonyloxy, phenylcarbonyloxy, benzylcarbonyloxy, and the like.

An "acetyl" group refers to a -C(=O)CH₃ group.

A "carboxylic acid" group refers to a C-carboxy group in which R" is hydrogen.

A "halo" or "halogen" group refers to fluorine, chlorine, bromine or iodine.

A "cyano" group refers to a -CN group.

A "nitro" group refers to a -NO₂ group.

An "O-carbamyl" group refers to a -OC(=O)NR¹⁰R¹¹ group, with R¹⁰ and R¹¹ as defined herein.

An "N-carbamyl" group refers to a R¹¹OC (=O) NR¹⁰- group, with R¹⁰ and R¹¹ as defined herein.

An "O-thiocarbamyl" group refers to a -OC(=S)NR¹⁰R¹¹ group, with R¹⁰ and R¹¹ as defined herein.

An "N-thiocarbamyl" group refers to a R¹¹OC(=S)NR¹⁰- group, with R¹⁰ and R¹¹ as defined herein.

An "amino" group refers to an -NR¹⁰R¹¹ group, wherein R¹⁰ and R¹¹ are independently hydrogen or unsubstituted lower alkyl, e.g, -NH₂, dimethylamino, diethylamino, ethylamino, methylamino, and the like.

A "C-amido" group refers to a -C(=O)NR¹⁰R¹¹ group, with R¹⁰ and R¹¹ as defined herein. For example, R¹⁰ is hydrogen or unsubstituted C₁-C₄ alkyl and R¹¹ is hydrogen, C₁-C₄ alkyl optionally substituted with heteroalicyclic, hydroxy, or amino. For example, C(=O)NR¹⁰R¹¹ may be aminocarbonyl, dimethylaminocarbonyl, diethylaminocarbonyl, diethylaminoethylaminocarbonyl, ethylaminoethylaminocarbonyl, and the like.

An "N-amido" group refers to a R¹¹C(=O)NR¹⁰- group, with R¹⁰ and R¹¹ as defined herein, e.g. acetylamino, and the like.

A "pharmaceutical composition" refers to a mixture of one or more of the compounds described herein, or physiologically/pharmaceutically acceptable salts or prodrugs thereof, with other chemical components, such as physiologically/pharmaceutically acceptable carriers and excipients. The purpose of a pharmaceutical composition is to facilitate administration of a compound to an organism.

As used herein, a "physiologically/pharmaceutically acceptable carrier" refers to a carrier or diluent that does not cause significant irritation to an organism and does not abrogate the biological activity and properties of the administered compound.

A "pharmaceutically acceptable excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

As used herein, the term "pharmaceutically acceptable salt" refers to those salts which retain the biological effectiveness and properties of the parent compound. Such salts include, but are not restricted to: (1) an acid addition salt which is obtained by reaction of the free base of the parent compound with inorganic acids such as hydrochloric acid, hydrobromic acid, nitric acid, phosphoric acid, sulfuric acid, and perchloric acid and the like, or with organic acids such as acetic acid, oxalic acid, (D) or (L) malic acid, maleic acid, methanesulfonic acid, ethanesulfonic acid, p-toluenesulfonic acid, salicylic acid, tartaric acid, citric acid, succinic acid or malonic acid and the like, preferably hydrochloric acid or (L)-malic acid; or (2) salts formed when an acidic proton present in the parent compound either is replaced by a metal ion, e. g., an alkali metal ion, such as sodium or potassium, an alkaline earth ion, such as magnesium or calcium, or an aluminum ion; or coordinates with an organic base such as ethanolamine, diethanolamine, triethanolamine, tromethamine, N-methylglucamine, and the like.

The terms "P2Y₁₂ related disease or disorder", "P2Y₁₂ associated disease or disorder" and "P2Y₁₂ connected disease or disorder" are used interchangeably herein to refer to a condition involving P2Y₁₂ activity. Examples for such diseases and disorders are stroke, myocardial infarction, such as non-ST elevation myocardial infarction (NSTEMI), myocardial ischemia, for example manifested in stable or unstable angina, various vascular diseases, such as coronary artery disease, peripheral artery disease, atherosclerosis, and cerebrovascular disease, and embolisms, such as thromboembolisms. Since the P2Y₁₂ receptor is also expressed in brain, e.g. in microglial cells, also included are CNS disorders, such as neuroinflammatory conditions and neurodegenerative disorders (e.g. Alzheimer's and Parkinson's disease).

"Treat", "treating" and "treatment" refer to a method of alleviating or abrogating a P2Y₁₂ related disease or disorder and/or its attendant symptoms.

"Prevent", "preventing" and "prevention" refer to a method of hindering a P2Y₁₂ related disease or disorder from occuring, i.e. a prophylactic method.

"Organism" refers to any living entity comprised of at least one cell. A living organism can be as simple as, for example, a single eukariotic cell or as complex as a mammal, including a human being.

"Therapeutically effective amount" refers to that amount of the compound being administered which will relieve to some extent one or more of the symptoms of the disorder being treated.

### Preferred embodiments

### Compounds

With respect to the compounds defined in the Summary of the invention, certain compounds of Formulae I or III are exemplified below.

One specific group of compounds of Formula I is that wherein:
wherein R⁷ is unsubstituted C₆ aryl.

One specific group of compounds of Formula I is that, wherein Y is NH and/or wherein R⁶ represents one to four substituents selected from the group consisting of hydrogen, -S(O)₂OR, -C(O)OR, tetrazolyl, sulfonyl, carboxy, amino, halogen, C₁-C₄ alkoxy, C₁-C₄ alkyl, hydroxy, and nitro.

One specific group of compounds of Formula I is that, wherein
(1) R¹² represents one to four substituents selected from the group consisting of hydrogen, halo, amino, unsubstituted C₁-C₄ alkyl or alkoxy, or hydroxy; and/or
(2) at least one of A and B is C=O or both A and B are C=O; and/or
(3) R¹ is amino; and/or
(4) R² is tetrazolyl, -S(O)₂OR or -C(O)OR.

The compounds of the invention can be administered in pharmaceutical formulations, either alone, or in combination with other pharmacologically active compounds. Suitable other pharmacologically active compounds may, for example, be selected from other P2Y₁₂ inhibitors, such as clopidogrel, or acetylsalicylic acid (Aspirin).

### Utility

The compounds of the present invention bind with high specificity and selectivity to human platelet P2Y₁₂ receptors. Preferably, they bind to their target receptor P2Y₁₂ with at least 10-fold, more preferably at least 100-fold, most preferably 10³-10⁸-fold higher affinity compared to related P2Y receptors, such as, for example, human P2Y₂, human P2Y₄, mouse P2Y₂ and rat P2Y₆.

Upon binding to P2Y₁₂, the compounds of the present invention reduce or abrogate P2Y₁₂ signaling. A precise understanding of the mechanism by which the compounds of the invention inhibit P2Y₁₂ signalling is not required in order to practice the present invention. However, while not hereby bound to any particular mechanism or theory, it is believed that the compounds interact with amino acids of P2Y₁₂ in the ADP binding region or in close proximity thereto, blocking the binding of ADP and thus the activation of the receptor. Accordingly, the compounds of the present invention are useful as inhibitors of P2Y₁₂ signalling. Because P2Y₁₂ plays a critical role in platelet aggregation, the cross-linking of platelets by fibrin, via the glycoprotein IIb/IIIa pathway, the compounds of the invention have anti-thrombotic activity and thus can be utilized in the treatment of diseases and disorders that are connected to an inappropriate or abnormal P2Y₁₂ function, in particular increased P2Y₁₂ activity, or that involve P2Y₁₂ function. Moreover, the compounds can also be used for the prevention of diseases and disorders that involve platelet aggregation, such as stroke, myocardial infarction, such as non-ST elevation myocardial infarction (NSTEMI), myocardial ischemia, for example manifested in stable or unstable angina, various vascular diseases, such as coronary artery disease, peripheral artery disease, atherosclerosis, and cerebrovascular disease, and embolisms, such as thromboembolisms. The present invention further encompasses an in vitro method for antagonizing the P2Y12 receptor function comprising the step of contacting cells expressing a P2Y12 receptor with at least one compound as defined above. In this context, it should be noted that for the efficacy of a compound of the invention as a pharmaceutical a number of variables besides binding affinity may play a crucial role. Accordingly, compounds of the invention can be specifically selected for use as a pharmaceutical not only based on the determined binding specificity and affinity, but also based on other factors, such as bioavailability, severity of side effects caused, metabolic conversion of the compound, half-life of the compound in the organism and the like.

### Administration and Pharmaceutical Composition

A compound of the present invention or a pharmaceutically acceptable salt thereof, can be administered as such to a human patient or can be administered in pharmaceutical compositions in which the foregoing materials are mixed with suitable carriers or excipient(s). Techniques for formulation and administration of drugs may be found in "Remington's Pharmacological Sciences," Mack Publishing Co., Easton, PA., latest edition.

As used herein, "administer" or "administration" refers to the delivery of a compound of Formula (I) or a pharmaceutically acceptable salt thereof or of a pharmaceutical composition containing a compound of Formula (I) or a pharmaceutically acceptable salt thereof of this invention to an organism for the purpose of prevention or treatment of a P2Y₁₂-related disease or disorder.

Suitable routes of administration may include, without limitation, oral, rectal, transmucosal or intestinal administration or intramuscular, subcutaneous, intramedullary, intrathecal, direct intraventricular, intravenous, intravitreal, intraperitoneal, intranasal, or intraocular injections. The preferred routes of administration are oral and parenteral.

Alternatively, one may administer the compound in a local rather than systemic manner, for example, via injection of the compound directly into a vessel, optionally in a depot or sustained release formulation.

Pharmaceutical compositions of the present invention may be manufactured by processes well known in the art, e. g., by means of conventional mixing, dissolving, granulating, drageemaking, levigating, emulsifying, encapsulating, entrapping or lyophilizing processes.

Pharmaceutical compositions for use in accordance with the present invention may be formulated in conventional manner using one or more physiologically acceptable carriers comprising excipients and auxiliaries which facilitate processing of the active compounds into preparations which can be used pharmaceutically. Proper formulation is dependent upon the route of administration chosen.

For injection, the compounds of the invention may be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art.

For oral administration, the compounds can be formulated by combining the active compounds with pharmaceutically acceptable carriers well known in the art. Such carriers enable the compounds of the invention to be formulated as tablets, pills, lozenges, dragees, capsules, liquids, gels, syrups, slurries, suspensions and the like, for oral ingestion by a patient. Pharmaceutical preparations for oral use can be made using a solid excipient, optionally grinding the resulting mixture, and processing the mixture of granules, after adding other suitable auxiliaries if desired, to obtain tablets or dragee cores. Useful excipients are, in particular, fillers such as sugars, including lactose, sucrose, mannitol, or sorbitol, cellulose preparations such as, for example, maize starch, wheat starch, rice starch and potato starch and other materials such as gelatin, gum tragacanth, methyl cellulose, hydroxypropylmethyl-cellulose, sodium carboxymethylcellulose, and/or polyvinylpyrrolidone (PVP). If desired, disintegrating agents may be added, such as cross-linked polyvinyl pyrrolidone, agar, or alginic acid. A salt such as sodium alginate may also be used.

Dragee cores are provided with suitable coatings. For this purpose, concentrated sugar solutions may be used which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical compositions which can be used orally include push-fit capsules made of gelatin, as well as soft, sealed capsules made of gelatin and a plasticizer, such as glycerol or sorbitol. The push-fit capsules can contain the active ingredients in admixture with a filler such as lactose, a binder such as starch, and/or a lubricant such as talc or magnesium stearate and, optionally, stabilizers. In soft capsules, the active compounds may be dissolved or suspended in suitable liquids, such as fatty oils, liquid paraffin, or liquid polyethylene glycols. Stabilizers may be added in these formulations, also.

The compounds may also be formulated for parenteral administration, e. g., by bolus injection or continuous infusion. Formulations for injection may be presented in unit dosage form, e. g., in ampoules or in multi-dose containers, with an added preservative. The compositions may take such forms as suspensions, solutions or emulsions in oily or aqueous vehicles, and may contain formulating materials such as suspending, stabilizing and/or dispersing agents.

Pharmaceutical compositions for parenteral administration include aqueous solutions of a water soluble form, such as, without limitation, a salt, of the active compound.

Additionally, suspensions of the active compounds may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances which increase the viscosity of the suspension, such as sodium carboxymethyl cellulose, sorbitol, or dextran. Optionally, the suspension may also contain suitable stabilizers and/or agents that increase the solubility of the compounds to allow for the preparation of highly concentrated solutions.

Alternatively, the active ingredient may be in powder form for constitution with a suitable vehicle, e. g., sterile, pyrogen-free water, before use.

The compounds may also be formulated in rectal compositions such as suppositories or retention enemas, using, e. g., conventional suppository bases such as cocoa butter or other glycerides.

In addition to the formulations described previously, the compounds may also be formulated as depot preparations. Such long acting formulations may be administered by implantation (for example, subcutaneously or intramuscularly) or by intramuscular injection. A compound of this invention may be formulated for this route of administration with suitable polymeric or hydrophobic materials (for instance, in an emulsion with a pharmacologically acceptable oil), with ion exchange resins, or as a sparingly soluble derivative such as, without limitation, a sparingly soluble salt.

A non-limiting example of a pharmaceutical carrier for the hydrophobic compounds of the invention is a cosolvent system comprising benzyl alcohol, a nonpolar surfactant, a water-miscible organic polymer and an aqueous phase such as the VPD co-solvent system. VPD is a solution of 3% w/v benzyl alcohol,8% w/v of the nonpolar surfactant Polysorbate 80, and 65% w/v polyethylene glycol 300, made up to volume in absolute ethanol. The VPD co-solvent system (VPD: D5W) consists of VPD diluted 1: 1 with a 5% dextrose in water solution. This cosolvent system dissolves hydrophobic compounds well, and itself produces low toxicity upon systemic administration.

Naturally, the proportions of such a co-solvent system may be varied considerably without destroying its solubility and toxicity characteristics. Furthermore, the identity of the co-solvent components may be varied: for example, other lowtoxicity nonpolar surfactants may be used instead of Polysorbate 80, the fraction size of polyethylene glycol may be varied, other biocompatible polymers may replace polyethylene glycol, e.g., polyvinyl pyrrolidone, and other sugars or polysaccharides may substitute for dextrose.

Alternatively, other delivery systems for hydrophobic pharmaceutical compounds may be employed. Liposomes and emulsions are well known examples of delivery vehicles or carriers for hydrophobic drugs. In addition, certain organic solvents such as dimethylsulfoxide also may be employed, although often at the cost of greater toxicity.

Additionally, the compounds may be delivered using a sustained-release system, such as semipermeable matrices of solid hydrophobic polymers containing the therapeutic agent.

Various sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for stabilization may be employed.

The pharmaceutical compositions herein also may comprise suitable solid or gel phase carriers or excipients. Examples of such carriers or excipients include, but are not limited to, calcium carbonate, calcium phosphate, various sugars, starch, cellulose derivatives, gelatin, and polymers such as polyethylene glycols.

Many of the compounds of the invention may be provided as physiologically acceptable salts wherein the claimed compound may form the negatively or the positively charged species. Examples of salts in which the compound forms the positively charged moiety include, without limitation, the sodium, potassium, calcium and magnesium salts formed by the reaction of a carboxylic acid or sulfonic acid group in the compound with an appropriate base (e.g. sodium hydroxide (NaOH), potassium hydroxide (KOH), Calcium hydroxide (Ca(OH)₂), etc.).

Pharmaceutical compositions suitable for use in the present invention include compositions wherein the active ingredients are contained in an amount sufficient to achieve the intended purpose, e. g., the inhibition of P2Y₁₂ function or the treatment or prevention of a P2Y₁₂-related disease or disorder.

More specifically, a therapeutically effective amount means an amount of compound effective to prevent, alleviate or ameliorate symptoms of disease or prolong the survival of the subject being treated.

Determination of a therapeutically effective amount is well within the capability of those skilled in the art, especially in light of the detailed disclosure provided herein.

For any compound used in the methods of the invention, the therapeutically effective amount or dose can be estimated initially from cell culture assays. Then, the dosage can be formulated for use in animal models so as to achieve a circulating concentration range that includes the IC₅₀ as determined in cell culture (i. e., the concentration of the test compound which achieves a half-maximal inhibition of the P2Y₁₂ activity). Such information can then be used to more accurately determine useful doses in humans.

Toxicity and therapeutic efficacy of the compounds described herein can be determined by standard pharmaceutical procedures in cell cultures or experimental animals, e.g., by determining the IC₅₀ and the LD₅₀ for a subject compound. The data obtained from these cell culture assays and animal studies can be used in formulating a range of dosage for use in humans. The dosage may vary depending upon the dosage form employed and the route of administration utilized. The exact formulation, route of administration and dosage can be chosen by the individual physician in view of the patient's condition.

Dosage amount and interval may be adjusted individually to provide plasma levels of the active species which are sufficient to maintain the P2Y₁₂ inhibiting effects. These plasma levels are referred to as minimal effective concentrations (MECs). The MEC will vary for each compound but can be estimated from in vitro data, e. g., the concentration necessary to achieve 50-90% inhibition of P2Y₁₂.

Dosages necessary to achieve the MEC will depend on individual characteristics and route of administration. HPLC assays or bioassays can be used to determine plasma concentrations.

Dosage intervals can also be determined using MEC value.

Compounds should be administered using a regimen that maintains plasma levels above the MEC for 10-90% of the time, preferably between 30-90% and most preferably between50-90%.

In cases of local administration or selective uptake, the effective local concentration of the drug may not be related to plasma concentration and other procedures known in the art may be employed to determine the correct dosage amount and interval.

The amount of a composition administered will, of course, be dependent on the subject being treated, the severity of the affliction, the manner of administration, the judgment of the prescribing physician, etc.

The compositions may, if desired, be presented in a pack or dispenser device, such as a kit approved by a regulatory authority, such as EMEA or FDA, which may contain one or more unit dosage forms containing the active ingredient. The pack may for example comprise metal or plastic foil, such as a blister pack. The pack or dispenser device may be accompanied by instructions for administration.

The pack or dispenser may also be accompanied by a notice associated with the container in a form prescribed by a governmental agency regulating the manufacture, use or sale of pharmaceuticals, which notice is reflective of approval by the agency of the form of the compositions or of human or veterinary administration. Compositions comprising a compound of the invention formulated in a compatible pharmaceutical carrier may also be prepared, placed in an appropriate container, and labeled for treatment of an indicated condition.

It is also an aspect of this invention that a compound described herein, or its salt or prodrug, might be combined with other agents for the treatment of the diseases and disorders discussed above.

### Synthesis

The most widely employed strategy for the synthesis of anilinoanthracene derivatives utilizes the Ullmann coupling reaction, which involves the treatment of a anthracene derivative substituted with a leaving group, such as halogen, with an arylamine in the presence of a copper catalyst (cf. Scheme 2, wherein R¹, R², R⁴, R⁵, A, B are defined as above, Z is a leaving group, and R^{x} can be any one to five substituents). As noted above, the reaction typically requires harsh conditions e.g., high temperatures and long reaction times, and suffers from poor yields.

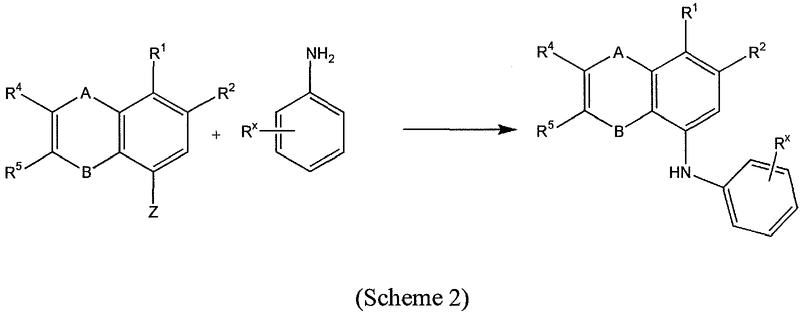

Typical reaction conditions include:
(a) reaction in the presence of CuCl, Na₂CO₃, and Na₂SO₃ in H₂O at room temperature for 8-24 h, or under reflux at 120 °C for 8-10 h (method A);
(b) reaction in the presence of CuSO₄ and Na₂CO₃ in H₂O at 120 °C for 12-48 h (method B); and
(c) reaction in the presence of Cu⁰ in sodium phosphate buffer pH 6-7 at 120 °C for 2-15 h (method C).

However, all three methods suffer from unsatisfactory product yield.

It has been found by the inventors of the present invention that microwave irradiation could improve the Ullmann coupling reactions by increasing product yield and accelerating the reaction. The present invention thus discloses an improved method for the synthesis of compounds of Formulae I or III, wherein the method includes a microwave-assisted copper-catalyzed Ullmann coupling reaction according to Scheme 1 wherein R¹, R², R³, R⁴, R⁵, Z, R⁶, Y and R⁷ are defined as above.

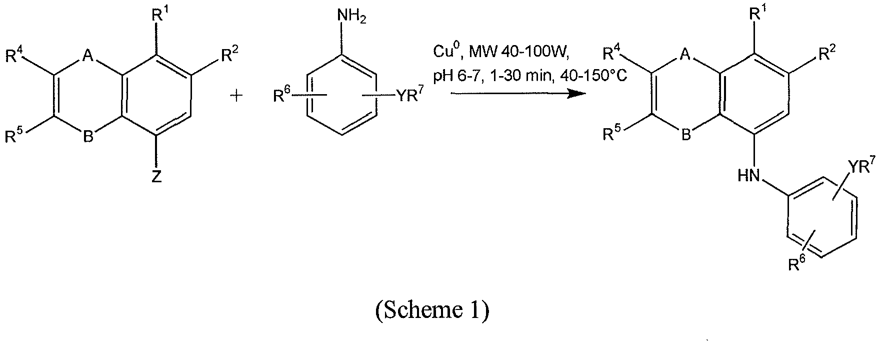

According to the invented method, the reaction mixtures were subjected to microwave irradiation, typically for only 1-30 min at 40-150 °C applying 40-100 W. All other conditions (catalyst, salts, solvent) were the same as in the classical method C. Instead of Cu(0), copper salts (Cu(I) or Cu(II)) can also be used.

The invented method is characterized by dramatically increased yields (30-90 % isolated) especially of compounds poorly accessible without microwaves and a reaction duration of only about 1-30 minutes.

### Examples

The present inventions will be explained in more detail in the following examples. However, the examples are only used for illustration and do not limit the scope of the present invention.

### Synthetic procedures

**General:** All materials were used as purchased (Sigma-Aldrich or Acros, Germany). Thin-layer chromatography was performed using TLC aluminum sheets silica gel 60 F₂₅₄, or TLC aluminum sheets RP silica gel 18 F₂₅₄ (Merck, Darmstadt, Germany). Colored compounds were visible at daylight; other compounds were visualized under UV light (254 nm). Flash chromatography was performed on a Büchi system using silica gel RP-18 (Merck, Darmstadt, Germany). ¹H- and ¹³C-NMR data were collected on a Bruker Avance 500 MHz NMR spectrometer at 500 MHz (¹H), or 126 MHz (¹³C), respectively. DMSO-d₆ was used as a solvent. Chemical shifts are reported in parts per million (ppm) relative to the deuterated solvent, i. e. DMSO, δ ¹H: 2.49 ppm, ¹³C: 39.7 ppm, coupling constants *J* are given in Hertz and spin multiplicities are given as s (singlet), d (doublet), t (triplet), q (quartet), m (multiplet), br (broad). The purities of isolated products were determined by ESI-mass spectra obtained on an LCMS instrument (Applied Biosystems API 2000 LCMS/MS, HPLC Agilent 1100) using the following procedure: the compounds were dissolved at a concentration of 0.5 mg/ml in H₂O : MeOH = 1 : 1, containing 2 mM NH₄CH₃COO. Then, 10 µl of the sample was injected into an HPLC column (Phenomenix Luna 3µ C18, 50 x 2.00 mm). Elution was performed with a gradient of water : methanol (containing 2 mM NH₄CH₃COO) from 90 : 10 to 0 : 100 for 30 min at a flow rate of 250 µl/min, starting the gradient after 10 min. UV absorption was detected from 200 to 950 nm using a diode array detector. Purity of the compounds was determined at 254 nm. For microwave reactions a CEM Focused™ Microwave Synthesis type Discover apparatus was used. A freeze dryer (CHRIST ALPHA 1-4 LSC) was used for lyophilization, reduction was achieved by using a Hogen^{®} GC hydrogen generator from the Proton Energy System (USA) company.

### Example 1: General synthesis procedure

### Procedure A: Ullmann coupling reactions of bromaminic acid or related derivatives with an aryl amine or an aliphatic amine or another amino-substituted compound

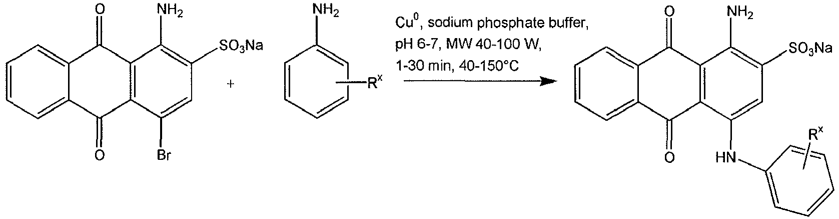

To a 5 mL microwave reaction vial equipped with a magnetic stirring bar were added a bromo-substituted phenyl derivative or another mono-, bi- or tricyclic bromo-substituted compound (e.g. bromaminic acid sodium salt) (0.20 mmol) and the appropriate aniline derivative or other amino-substituted compound (0.40 mmol), followed by a buffer solution of Na₂HPO₄ (pH 9.6) (4 mL) and NaH₂PO₄ (pH 4.2) (1 mL). A catalytic amount (e.g. 0.002-0.003 g) of finely powdered elemental copper (or copper(I)-salt, or copper(II)-salt, respectively) was added. The mixture was capped and irradiated in the microwave oven (e.g. 40-100 W) for 1-30 min at 40-150 °C. Then the reaction mixture was cooled to rt, and the product was purified using the following procedure. The contents of the vial were filtered to remove the elemental copper. Then ca. 200 mL of water was added to the filtrate and the aqueous solution was extracted with chloroform (200 mL). The extraction procedure was repeated until the chloroform layer became colorless (2-3 times). Then the aqueous layer was reduced by rotary evaporation to a volume of 10-20 mL, which was subsequently submitted to flash column chromatography using RP-18 silica gel and water as an eluent. The polarity of the eluent was then gradually decreased by the addition of methanol in the following steps: 20, 40, 60, 80, and 100 %. Fractions containing blue product were collected. For some compounds the last step of purification (RP-18 flash chromatography) had to be repeated two to three times to obtain pure product (≥ 95% purity as determined by LC-MS). The pooled product-containing fractions were evaporated under vacuum to remove the methanol, and the remaining water was subsequently removed by lyophilization to yield the products (30-90%) as blue powders.

According to the following reaction scheme the results shown in Table 2 were obtained.

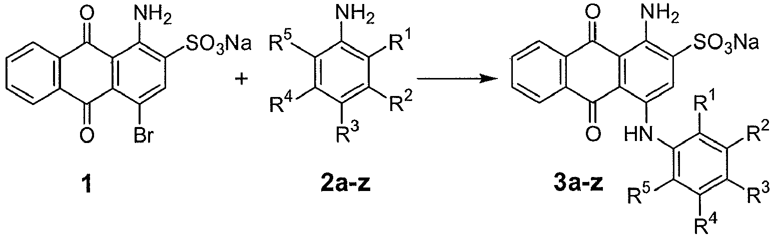

**Table 2**

| entry | R¹ | R² | R³ | R⁴ | R⁵ | products 3 (yield [%])*^{b}* |
|---|---|---|---|---|---|---|
| 1 | H | COOH | H | NH₂ | H | **3a** (70) |
| 2 | H | COOH | H | H | H | **3b** (76) |
| 3 | H | H | COOH | H | H | **3c** (70) |
| 4 | NH₂ | H | H | H | H | **3d** (58) |
| 5 | H | NH₂ | H | H | H | **3e** (70) |
| 6 | COOH | H | Cl | H | H | **3f** (43) |
| 7 | COOH | H | H | Cl | H | **3g** (64) |
| 8 | COOH | H | H | H | H | **3h** (83) |
| 9 | OH | H | H | H | H | **3i** (58) |
| 10 | H | H | OH | H | H | **3j** (72) |
| 11 | H | H | NH₂ | H | H | **3k** (90) |
| 12 | H | H | H | H | H | **3l** (55) |
| 13 | H | H | Cl | H | H | **3m** (87) |
| 14 | H | H | 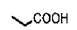 | H | H | **3n** (44) |
| 15 | CH₃ | H | CH₃ | H | CH₃ | **3o** (77) |
| 16 | OMe | H | H | H | H | **3p** (79) |
| 17 | H | OMe | H | H | H | **3q** (67) |
| 18 | H | H | OMe | H | H | **3r** (85) |
| 19 | H | H | F | H | H | **3s** (76) |
| 20 | H | Br | H | H | H | **3t** (41) |
| 21 | H | CH₃ | H | CH₃ | H | **3u** (32) |
| 22 | H | Cl | H | H | H | **3v** (56) |
| 23 | CH₃ | NH₂ | H | H | H | **3w** (34) |
| 24 | H | COOH | OH | H | H | **3x** (30) |
| 25 | SO₃H | H | H | H | H | **3y** (30) |
| 26 | H | H | SO₃H | H | H | **3z** (40) |

| | | | | | | |
|---|---|---|---|---|---|---|
| "Reaction mixtures were irradiated for 2 to 20 min in sodium phosphate buffer, pH 6-7, in the presence of Cu⁰ at 80-120 °C. *^{b}*Isolated yields (purity of products ≥ 95% as determined by HPLC-UV (254 nm)-ESI-MS) calculated based on educt **1**; true yields were higher since commercial **1** was only 90% pure (main contaminant: desbromo derivative, 8%). | | | | | | |

For some selected compounds the obtained product yields were compared with those obtained by the conventional method C, which was proven superior to methods A and B (See above). The results of this comparison, shown in Figure 1, clearly demonstrate that the method of the present invention leads to improved product yields compared to the conventional method C.

### Specific Example for procedure A

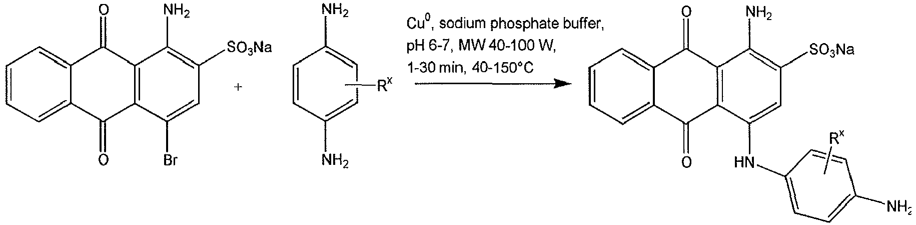

### Procedure B: Preparation of compound 4

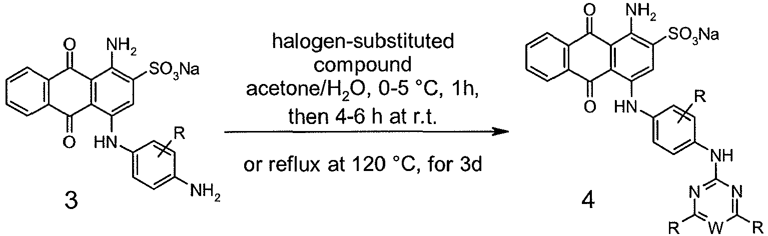

An ice-cooled solution of an halogen-substituted derivative (0.5-1 mmol) in water (25 ml) and acetone (25 ml) was added to a stirred solution of compound **3** (0.5 mmol) in water (25 ml) at 0-5 °C. The resulting mixture was stirred for 1 h at 0-5 °C, then at room temperature for 4-6 h. The formation of product was monitored by RP-TLC using a mobile phase of acetone/water (2:3). After completion of the reaction the solvents were evaporated and the residue was purified by FCC RP-18 silica gel using an acetone/water eluent to obtain compound **4** (or analogs).

### Procedure C: Alternative preparation of compound 4

An ice-cooled solution of 2-bromopyrimidine or another halogen compound (0.5-1 mmol) in water (25 ml) and acetone (25 ml) was added to a stirred solution of the respective compound 3 (0.5 mmol) in water (25 ml) at 0-5 °C. Then the temperature was graduelly increased for refluxing at 120 °C for 1-3d. The formation of product was monitored by RP-TLC using a mobile phase of methanol/water (2:3). After completion of the reaction the solvents were evaporated and the residue was purified by FCC RP-18 silica gel using an methanol/water eluent to obtain compound **4.**

### General procedure D: Preparation of compound 5

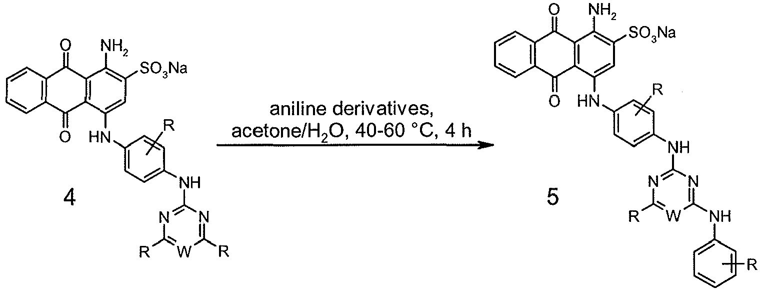

### Example for the preparation of products 5

An ice-cooled solution of an aniline derivative or another amino-substituted compound (1-2 mmol) in water (25 ml) and acetone (25 ml) was added to a stirred solution of the compound **4** (1 mmol) in water (25 ml) at 0-5 °C. Then the temperature was graduelly increased to reach 40-60 °C for 4 h. The formation of product was monitored by RP-TLC using a mobile phase of acetone/water (3:2). After completion of the reaction the solvents were evaporated and the residue was purified by FCC RP-18 silica gel using an acetone/water eluent to obtain compound **5.**

### General procedure E: Synthesis of intermediate compounds having Formula VIII

To an 80 mL microwave reaction vial equipped with a magnetic stirring bar were added 5-nitro-2-chlorobenzoic acid (2.520 g, 12.5 mmol) and an excess (e.g. 6 eq.) of the appropriate aliphatic or aromatic amine derivative (ca. 75 mmol) to obtain a homogenous mixture. The mixture was irradiated in a microwave oven (80-100 W) for 5-30 min at 80-120 °C. Then the reaction mixture was cooled to rt, and the product was purified using the following procedure. The contents of the vial were dissolved in ca. 500 mL of dichloromethane and the organic solution was extracted with diluted aq. sodium hydroxide solution (ca. 500 mL). The extraction procedure was repeated until the aqueous layer became light yellow (3-4 times). Then the aqueous layer was collected and acidified using concentrated aq. hydrochloric acid (37 %) until pH ≤ 3 and the desired product precipitated in the acidic medium. The precipitated solid was filtered off and washed several times (2-3 times) with 100 mL water each to remove the remaining sodium chloride as well as hydrochloric acid. The product was dried in an oven at 100 °C. The purity of the product was ≥ 95% as determined by LC-MS.

### General procedure F: Reduction of the intermediate nitro derivative of Formula VIII

To a 10 mL vial equipped with a magnetic stirring bar were added the nitro-substituted compound (VIII, 1 mmol) followed by a buffer solution of Na₂HPO₄ (pH 9.6) (4 mL) and NaH₂PO₄ (pH 4.2) (1 mL). A catalytic amount (ca. 0.005-0.010 g) of 10% palladium on activated charcoal, finely powdered, was added. The mixture was hydrogenated at 275,790 kPA - 413,685 kPa ("40-60 psi") for 3-6 h. The resulting mixture was used directly for the next step without prior purification.

### General procedure G: Coupling reaction of the reduction products of general procedure F with halogen-substituted compounds

To a 5 mL microwave reaction vial equipped with a magnetic stirring bar were added bromaminic acid sodium salt, or another halogen-substituted compound, (0.20 mmol) and the mixture obtained by general procedure F without further purification, followed by a buffer solution of Na₂HPO₄ (pH 9.6) (4 mL) and NaH₂PO₄ (pH 4.2) (1 mL). A catalytic amount (ca. 0.004-0.006 g) of finely powdered elemental copper was added. The mixture was irradiated in a microwave oven (100 W) for 5 min at 120 °C, or according to the general procedure **A.** The purification of the compounds was the same as described in general procedure **A.**

### Example 2: Sodium 1-amino-4-(3-amino-5-carboxyphenylamino)-9,10-dioxo-9,10-dihydroanthracene 2-sulfonate.

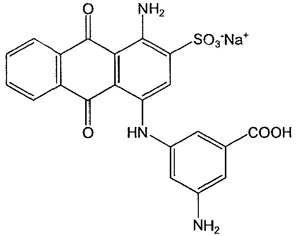

**Reaction conditions:** according to the general procedure A: 20 min, 120 °C, 100 W; pressure up to 10 bar.

**Analytical data:** mp > 300 °C, blue powder. **¹H-NMR:** δ 5.29 (bs, 2H, 3'-NH₂), 6.53 (dd, 1H, 6'-H), 7.03 (d, 1H, 2'-H), 7.08 (d, 1H, 4'-H), 7.83 (m, 2H, 6-H, 7-H), 7.96 (s, 1H, 3-H), 8.26 (m, 2H, 5-H, 8-H), 10.13 (br, 2H, 1-NH2), 12.04 (s, 1H, 4-NH). **¹³C**-**NMR:** δ 109.05 (C-9a), 110.75 (C-4a), 110.93, 112.33, 112.51 (C-2', C-4', C-6'), 123.40 (C-3), 126.07 (C-5), 126.15 (C-8), 132.86 (C-6), 133.17 (C-7), 133.83 (C-10a), 134.29 (C-8a), 139.11 (C-1'), 142.12 (C-4), 142.94 (C-2, C-5'), 144.35 (C-1), 149.77 (C-3'), 172.50 (COOH), 181.78 (C-9), 182.14 (C-10). **LC-MS (m/z):** 471 [M-Na+NH₄⁺]⁺, 454 [M-Na]⁺, 452 [M-Na]⁻. Purity by **HPLC-UV (254 nm)-ESI-MS:** 95%.

### Example 3: Sodium 1-amino-4-(3-carboxyphenylamino)-9,10-dioxo-9,10-dihydroanthracene 2-sulfonate.

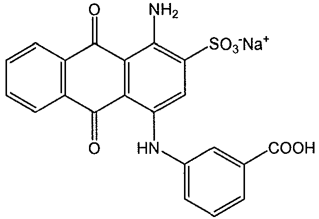

**Reaction conditions:** according to the general procedure A: 20 min, 120 °C, 80 W; pressure up to 10 bar.

**Analytical data:** mp > 300 °C, blue powder. **¹H**-**NMR:** δ 7.38 (dd, 1H, 6'-H), 7.47 (dd, 1H, 5'-H), 7.76 (dd, 1H, 4'-H), 7.84 (m, 3H, 2'-H, 6-H, 7-H), 7.97 (s, 1H, 3-H), 8.26 (m, 2H, 5-H, 8-H), 10.00 (br, 2H, 1-NH₂), 12.69 (s, 1H, 4-NH). **¹³C-NMR:** δ 109.35 (C-9a), 111.72 (C-4a), 122.81 (C-3), 124.05 (C-2'), 125.45 (C-5), 125.62 (C-8), 126.11, 126.19 (C-4', C-6'), 129.38 (C-5'), 132.94 (C-6), 133.35 (C-7), 133.72 (C-10a), 134.30 (C-8a), 139.18 (C-1'), 140.95 (C-4), 142.91 (C-2), 144.51 (C-1), 145.21 (C-3'), 169.63 (COOH), 182.00 (C-9), 182.71 (C-10). **LC-MS (m/z):** 439 [M-Na]⁺, 437 [M-Na]⁻. Purity by **HPLC-UV (254 nm)-ESI-MS:** 96%.

### Example 4: Sodium 1-amino-4-(4-carboxyphenylamino)-9,10-dioxo-9,10-dihydroanthracene 2-sulfonate.

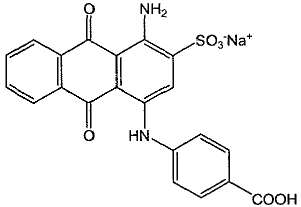

**Reaction conditions:** according to the general procedure A: 20 min, 120 °C, 80 W; pressure up to 10 bar.

**Analytical data:** mp > 300 °C, blue powder. **¹H-NMR:** δ 7.17 (d, 2H, 2'-H, 6'-H), 7.84 (m, 2H, 6-H, 7-H), 7.93 (d, 2H, 3'-H, 5'-H), 8.07 (s, 1H, 3-H), 8.26 (m, 2H, 5-H, 8-H), 10.22 (br, 2H, 1-NH₂), 12.07 (s, 1H, 4-NH). **¹³C-NMR:** 109.28 (C-9a), 111.62 (C-4a), 121.43 (C-2', C-6'), 123.11 (C-3), 126.11 (C-5), 126.17 (C-8), 130.64 (C-3', C-5'), 132.91 (C-6), 133.29 (C-7), 133.74 (C-10a), 134.29 (C-8a), 136.59 (C-4'), 139.77 (C-1'), 140.78 (C-4), 142.85 (C-2), 144.53 (C-1),169.78 (COOH), 181.93 (C-9), 182.56 (C-10). **LC-MS (m/z):** 439 [M-Na]⁺, 437 [M-Na]⁻. Purity by **HPLC-UV (254 nm)-ESI-MS:** 98%.

### Example 5: Sodium 1-amino-4-(2-aminophenylamino)-9,10-dioxo-9,10-dihydroanthracene 2-sulfonate.

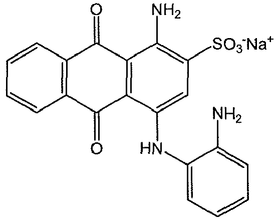

**Reaction conditions:** according to the general procedure A: 20 min, 120 °C, 80 W; pressure up to 10 bar.

**Analytical data:** mp > 300 °C, blue powder. **¹H**-**NMR:** δ 5.02 (bs, 2H, 2'-NH₂), 6.62 (ddd, 1H, 4'-H), 6.84 (dd, 1H, 3'-H), 7.01 (m, 2H, 5'-H, 6'-H), 7.61 (s, 1H, 3-H), 7.83 (m, 2H, 6-H, 7-H), 8.27 (m, 2H, 5-H, 8-H), 10.15 (br, 2H, 1-NH₂), 11.67 (s, 1H, 4-NH). **¹³C-NMR:** 109.10 (C-9a), 110.58 (C-4a), 115.77, 116.72 (C-4', C-5'), 123.22 (C-3), 123.70, 126.01, 126.12, 126.64 (C-5, C-8, C-6', C-3'), 127.06 (C-1'), 132.71 (C-6), 132.93 (C-7), 133.96 (C-10a), 134.33 (C-8a), 143.08 (C-4), 143.66 (C-2), 144.26(C-1), 144.28 C-2'), 181.67 (C-9), 181.92 (C-10). **LC-MS (m/z):** 410 [M-Na]⁺, 408 [M-Na]⁻. Purity by **HPLC-UV (254 nm)-ESI-MS:** 95%.

### Example 6: Sodium 1-amino-4-(3-aminophenylamino)-9,10-dioxo-9,10-dihydroanthracene 2-sulfonate.

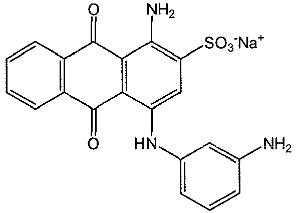

**Reaction conditions:** according to the general procedure A: 2 min, 120 °C, 80 W; pressure up to 10 bar.

**Analytical data:** mp > 300 °C, blue powder. **¹H-NMR:** δ 5.28 (bs, 2H, 3'-NH₂), 6.42 (m, 3H, 2'-H, 4'-H, 6'-H), 7.06 (dd, 1H, 5'-H), 7.83 (m, 2H, 6-H, 7-H), 8.02 (s, 1H, 3-H), 8.26 (m, 2H, 5-H, 8-H), 10.12 (br, 2H, 1-NH₂), 12.03 (s, 1H, 4-NH). **¹³C**-**NMR:** 108.69, 109.02, 110.76, 110.83, 110.95 (C-9a, C-4a, C-2', C-4', C-6'), 123.39 (C-3), 126.05 (C-5), 126.13 (C-8), 130.06 (C-5'), 132.79 (C-6), 133.10 (C-7), 133.82 (C-10a), 134.29 (C-8a), 139.76 (C-1'), 141.86 (C-4), 143.03 (C-2), 144.39 (C-1), 150.27 (C-3'), 181.74 (C-9), 182.11 (C-10). **LC-MS (m/z):** 410 [M-Na]⁺, 408 [M-Na]⁻. Purity by **HPLC-UV (254 nm)-ESI-MS:** 95%.

### Example 7: Sodium 1-amino-4-(2-carboxy-4-chlorophenylamino)-9,10-dioxo-9,10-dihydro-anthracene 2-sulfonate.

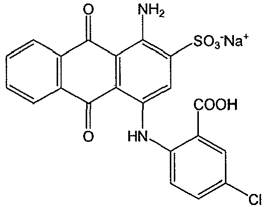

**Reaction conditions:** according to the general procedure **A:** 5 min, 120 °C, 80 W; pressure up to 10 bar.

**Analytical data:** mp > 300 °C, blue powder. **¹H-NMR:** δ 7.13 (d, 1H, 6'-H), 7.29 (dd, 1H, 5'-H), 7.81 (m, 2H, 6-H, 7-H), 7.90 (d, 1H, 3'-H), 8.11 (s, 1H, 3-H), 8.22 (m, 2H, 5-H, 8-H), 9.97 (br, 2H, 1-NH₂), 12.72 (s, 1H, 4-NH). **¹³C-NMR:** δ 110.28 (C-9a), 115.81 (C-4a), 121.00 (C-3), 124.35, 126.08, 126.14 (C-2', C-4', C-6'), 125.43 (C-5, C-8), 129.50, 131.15 (C-5', C-3'), 32.92 (C-6), 133.23 (C-7), 133.93 (C-10a), 134.20 (C-8a), 136.71 (C-1'), 140.04 (C-4), 141.22 (C-2) 144.91 (C-1), signal for COOH not detectable (too broad and flat), 181.80 (C-9), 182.54 (C-10). **LC-MS (m/z):** 439 [M-Na]⁺, 437 [M-Na]⁻. Purity by **HPLC-UV (254 nm)-ESI-MS:** 95%.

### Example 8: Sodium 1-amino-4-(2-carboxy-5-chlorophenylamino)-9,10-dioxo-9,10-dihydro-anthracene 2-sulfonate.

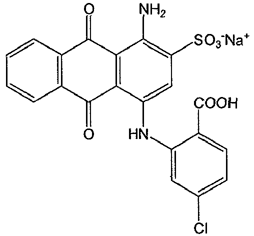

**Reaction conditions:** according to the general procedure **A:** 5 min, 120 °C, 80 W; pressure up to 10 bar.

**Analytical data:** mp > 300 °C, blue powder. **¹H**-**NMR:** δ 6.92 (d, 1H, 6'-H), 7.05 (d, 1H, 4'-H), 7.70 (d, 1H, 3'-H), 7.80 (m , 2H, 6-H, 7-H), 8.12 (s, 1H, 3-H), 8.21 (m, 2H, 5-H, 8-H), 9.93 (br, 2H, 1-NH₂), 12.65 (s, 1H, 4-NH). **¹³C-NMR:** δ 110.58 (C-9a), 117.22, 117.50 (C-4a, C-2', C-4'), 120.02 (C-3), 125.90 (C-5'), 126.13, 126.19 (C-6', C-5, C-8) 132.91 (C-6), 133.48 (C-7), 133.78 (C-10a), 134.18 (C-8a), 135.05, 135.39 (C-3', C-1'), 141.01 (C-4), 143.26 (C-2), 145.21 (C-1), signal for COOH not detectable (too broad and flat), 182.36 (C-9), 182.69 (C-10). **LC-MS (m/z):** 473 [M-Na]⁺, 471 [M-Na]⁻. Purity by **HPLC-UV (254 nm)-ESI-MS:** 95%.

### Example 9: Sodium 1-amino-4-(2-carboxyphenylamino)-9,10-dioxo-9,10-dihydroanthracene 2-sulfonate.

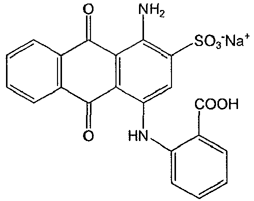

**Reaction conditions:** according to the general procedure A: 5 min, 120 °C, 80 W; pressure up to 10 bar.

**Analytical data:** mp > 300 °C, blue powder. **¹H-NMR:** 6.67 (dd, 1H, 4'-H), 7.13 (d, 1H, 6'-H), 7.30 (d, 1H, 5'-H), 7.80 (m, 2H, 6-H, 7-H), 8.15 (s, 1H, 3-H), 8.23 (m, 2H, 5-H, 8-H), 10.00 (br, 2H, 1-NH₂), 12.69 (s, 1H, 4-NH). **¹³C**-**NMR:** 110.12 (C-9a), 115.18 (C-4a), 119.75 (C-2'), 121.24 (C-3), 125.31, 126.06, 126.21 (C-6', C-4', C-5, C-8), 130.73, 131.88 (C-5', C-3'), 132.76 (C-6), 133.20 (C-7), 133.91 (C-10a), 134.23 (C-8a), 137.34 (C-1'), 140.98 (C-4), 141.40 (C-2), 144.89 (C-1), signal for COOH not detectable (too broad and flat), 181.88 (C-9), 182.37 (C-10). **LC-MS (m/z):** 439 [M-Na]⁺, 437 [M-Na]⁻. Purity by **HPLC-UV (254 nm)-ESI-MS:** 95%.

### Example 10: Sodium 1-amino-4-(2-hydroxyphenylamino)-9,10-dioxo-9,10-dihydroanthracene 2-sulfonate.

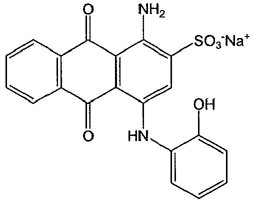

**Reaction conditions:** according to the general procedure A: 10 min, 120 °C, 80 W; pressure up to 10 bar.

**Analytical data:** mp > 300 °C, blue powder. **¹H-NMR:** δ 6.86 (ddd, 1H, 4'-H), 6.99 (dd, 1H, 6'-H), 7.04 (ddd, 1H, 5'-H), 7.21 (dd, H, 3'-H), 7.83 (m, 2H, 6-H, 7-H), 7.93 (s, 1H, 3-H), 8.27 (m, 2H, 5-H, 8-H), 9.87 (br, 2H, 1-NH₂), 10.15 (br, 1H, 2'-OH), 11.92 (s, 1H, 4-NH). **¹³C-NMR:** 109.19 (C-9a), 110.97 (C-4a), 116.30, 119.55, 123.24, 123.91, 125.60, 126.03, 126.12, 126.67 (C-1', C-3', C-4', C-5', C-6', C-3, C-5, C-8), 132.79 (C-6) 133.23 (C-7), 133.88 (C-10a), 134.28 (C-8a), 141.66 (C-4), 142.78 (C-2), 144.29 (C-1), 150.65 (C-2'), 181.81 (C-9), 181.99 (C-10). **LC-MS (m/z):** 411 [M-Na]⁺, 409 [M-Na]⁻. Purity by **HPLC-UV (254 nm)-ESI-MS:** 96%.

### Example 11: Sodium 1-amino-4-(4-hydroxyphenylamino)-9,10-dioxo-9,10-dihydroanthracene 2-sulfonate.

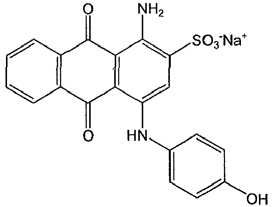

**Reaction conditions:** according to the general procedure **A:** 10 min, 120 °C, 80 W; pressure up to 10 bar.

**Analytical data:** mp > 300 °C, blue powder. **¹H-NMR:** δ 6.82 (d, 2H, 2'-H, 6'-H), 7.08 (d, 2H, 3'-H, 5'-H), 7.81 (s, 1H, 3-H), 7.83 (m, 2H, 6-H, 7-H), 8.27 (m, 2H, 5-H, 8-H), 10.15 (br, 2H, 1-NH₂), 12.05 (s, 1H, 4-NH). **¹³C**-**NMR:** δ 108.97 (C-9a), 110.03 (C-4a), 116.39 (C-2', C6'), 122.93 (C-3), 125.98 (C-5), 126.13 (C-8), 126.36 (C-3', C5'), 129.98 (C-1'), 132.75 (C-6), 132.96 (C-7), 133.90 (C-10a), 134.29 (C-8a), 143.22 (C-4), 143.25 (C-2), 144.19 (C-1), 155.52 (C-4'), 181.69 (C-9), 181.70 (C-10). **LC-MS** (**m**/**z**)**:** 411 [M-Na]⁺, 409 [M-Na]⁻. Purity by **HPLC-UV (254 nm)-ESI-MS:** 95%.

### Example 12: Sodium 1-amino-4-(4-aminophenylamino)-9,10-dioxo-9,10-dihydroanthracene 2-sulfonate.

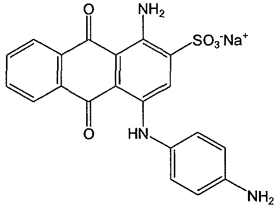

**Reaction conditions:** according to the general procedure **A:** 5 min, 100 °C, 60 W; pressure up to 10 bar.

**Analytical data:** mp > 300 °C, blue powder. **¹H-NMR:** δ 5.17 (bs, 2H, 4'-NH₂), 6.64 (d, 2H, 2'-H, 6'-H), 6.94 (d, 2H, 3'-H, 5'-H), 7.81 (s, 1H, 3-H), 7.82 (m, 2H, 6-H, 7-H), 8.27 (m, 2H, 5-H, 8-H), 10.17 (br, 2H, 1-NH₂), 12.09 (s, 1H, 4-NH). **¹³C-NMR:** δ 108.84 (C-9a), 109.52 (C-4a), 114.78 (C-2', C6'), 122.85 (C-3), 125.93 (C-5), 126.09 (C-8), 126.14, 126.82 (C-3', C5', C-1'), 132.65 (C-6), 132.75 (C-7), 133.99 (C-10a), 134.26 (C-8a), 143.24 (C-4), 143.88 (C-2), 144.15 (C-1), 147.08 (C-4'), 181.17 (C-9), 181.52 (C-10). LC-**MS (m/z):** 432 [M]⁺, 427 [M-Na+NH₄⁺]⁺, 410 [M-Na]⁺, 408 [M-Na]⁻. Purity by **HPLC-UV (254 nm)-ESI-MS:** 95%.

### Example 13: Sodium 1-amino-9,10-dioxo-4-phenylamino-9,10-dihydroanthracene 2-sulfonate.

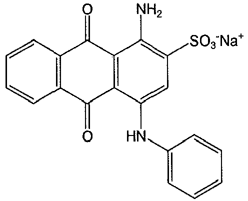

**Reaction conditions:** according to the general procedure **A:** 5 min, 80 °C, 40 W; pressure up to 10 bar.

**Analytical data:** mp > 300 °C, blue powder. **¹H**-**NMR:** δ 7.20 (ddd, 1H, 4'-H), 7.28 (dd, 2H, 2'-H, 6'-H), 7.44 (ddd, 2H, 3'-H, 5'-H), 7.84 (m, 2H, 6-H, 7-H), 8.02 (s, 1H, 3-H), 8.27 (m, 2H, 5-H, 8-H), 10.10 (br, 2H, 1-NH₂), 12.04 (s, 1H, 4-NH). **¹³C**-**NMR:** δ 109.30 (C-9a), 111.56 (C-4a), 122.82 (C-3), 123.20 (C-2', C6'), 124.58 (C-4'), 126.09 (C-5), 126.17 (C-8), 129.84 (C-3', C5'), 132.90 (C-6), 133.30 (C-7), 133.72 (C-10a), 134.29 (C-8a), 139.41 (C-1'), 140.93 (C-4), 142.95 (C-2), 144.50 (C-1), 181.94 (C-9), 182.62 (C-10). **LC-MS (m/z):** 395 [M-Na]⁺, 393 [M-Na]⁻. Purity by **HPLC-UV (254 nm)-ESI-MS:** 98%.

### Example 14: Sodium 1-amino-4-(4-chlorophenylamino)-9,10-dioxo-9,10-dihydroanthracene 2-sulfonate.

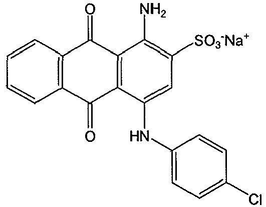

**Reaction conditions:** according to the general procedure **A:** 5 min, 100 °C, 80 W; pressure up to 10 bar.

**Analytical data:** mp > 300 °C, blue powder. **¹H-NMR:** δ 7.30 (d, 2H, 2'-H, 6'-H), 7.47 (d, 2H, 3'-H, 5'-H), 7.85 (m, 2H, 6-H, 7-H), 7.96 (s, 1H, 3-H), 8.26 (m, 2H, 5-H, 8-H), 10.05 (br, 2H, 1-NH₂), 11.87 (s, 1H, 4-NH). **¹³C-NMR:** δ 109.46 (C-9a), 112.24 (C-4a), 122.79 (C-3), 124.61 (C-2', C6'), 126.08 (C-2'), 126.20 (C-5), 126.20 (C-8), 128.18 (C-4'), 129.70 (C-3' and C-5'), 133.00 (C-6), 133.47 (C-7), 133.63 (C-10a), 134.28 (C-8a), 138.66 (C-1'), 140.15 (C-4), 142.80 (C-2), 144.59 (C-1), 182.08 (C-9), 182.94 (C-10). **LC-MS** (**m/z**): 446 [M-Na+NH₄⁺]⁺, 429 [M-Na]⁺, 427 [M-Na]⁻. Purity by **HPLC-UV (254 nm)-ESI-MS:** 96%.

### Example 15: Sodium 1-amino-4-(4-carboxymethylphenylamino)-9,10-dioxo-9,10-dihydro-anthracene 2-sulfonate.

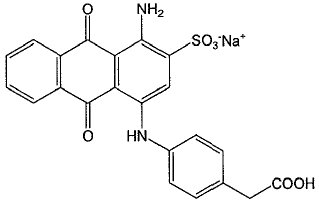

**Reaction conditions:** according to the general procedure A: 10 min, 100 °C, 80 W; pressure up to 10 bar.

**Analytical data:** mp > 300 °C, blue powder. **¹H-NMR:** δ 3.24 (s, 2H, CH₂), 7.12 (d, 2H, 2'-H, 6'-H), 7.30 (d, 2H, 3'-H, 5'-H), 7.84 (m, 2H, 6-H, 7-H), 7.99 (s, 1H, 3-H), 8.27 (m, 2H, 5-H, 8-H), 10.12 (br, 2H, 1-NH₂), 12.12 (s, 1H, 4-NH). **¹³C**-**NMR:** δ 45.83 (CH₂), 109.11 (C-9a), 110.75 (C-4a), 122.78 (C-3), 122.99 (C-2', C6'), 126.05 (C-5), 126.15 (C-8), 130.49 (C-3', C-5'), 132.82 (C-6), 133.11 (C-7), 133.83 (C-10a) 134.30 (C-8a), 136.00, 137.11 (C-4', C-1'), 141.93 (C-4), 143.10 (C-2), 144.38 (C-1), 174.50 (COOH), 181.78 (C-9), 182.10 (C-10). **LC-MS (m/z):** 470 [M-Na+NH₄⁺]⁺, 453 [M-Na]⁺, 451 [M-Na]⁻. Purity by **HPLC-UV (254 nm)-ESI-MS:** 95%.

### Example 16: Sodium 1-amino-9,10-dioxo-4-(2,4,6-trimethylphenylamino)-9,10-dihydroanthracene 2-sulfonate.

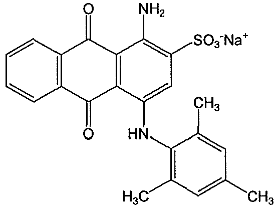

**Reaction conditions:** according to the general procedure **A:** 5 min, 120 °C, 100 W; pressure up to 10 bar.

**Analytical data:** mp > 300 °C, blue powder. **¹H-NMR:** δ 2.10 (s, 6H, 2'-CH₃, 6'CH₃), 2.30 (s, 3H, 4'-CH₃), 7.03 (s, 2H, 3'-H, 5'-H), 7.13 (s, 1H, 3-H), 7.84 (m, 2H, 6-H, 7-H), 8.29 (m, 2H, 5-H, 8-H), 10.10 (br, 2H, 1-NH₂), 11.73 (s, 1H, 4-NH). **¹³C-NMR:** δ 20.71 (C4'-CH₃), 18.10 (C2'-CH₃, C3'-CH₃), 109.20 (C-9a), 109.49 (C-4a), 121.92 (C-3), 126.04 (C-5), 126.16 (C-8), 129.38 (C-3' and C-5'), 132.77 (C-6), 133.02 (C-7), 133.43 (C-4'), 133.82 (C-10a), 134.32 (C-8a), 135.30 (C-2', C6'), 136.20 (C-1'), 143.59 (C-4), 143.86 (C-2), 144.08 (C-1), 181.77 (C-9), 182.18 (C-10). **LC-MS (m/z):** 454 [M-Na+NH₄⁺]⁺, 437 [M-Na]⁺, 435 [M-Na]⁻. Purity by **HPLC-UV (254 nm)-ESI-MS:** 95%.

### Example 17: Sodium 1-amino-4-(2-methoxyphenylamino)-9,10-dioxo-9,10-dihydroanthracene 2-sulfonate.

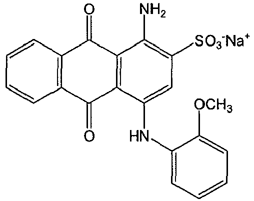

**Reaction conditions:** according to the general procedure **A:** 5 min, 100 °C, 80 W; pressure up to 10 bar.

**Analytical data:** mp > 300 °C, blue powder. **¹H**-**NMR:** δ 3.86 (s, 3H, 3'-OCH₃), 7.01 (ddd, 1H, 4'-H), 7.17 (m, 2H, 5'-H, 6'-H), 7.29 (dd, 1H, 3'-H), 7.84 (m, 2H, 6-H, 7-H), 7.96 (s, 1H, 3-H), 8.27 (m, 2H, 5-H, 8-H), 10.10 (br, 2H, 1-NH₂), 11.92 (s, 1H, 4-NH). **¹³C**-**NMR:** δ 55.93 (C3'-OCH₃), 109.31 (C-9a), 111.56 (C-4a), 112.38, 120.93, 122.88, 123.06 (C-3', C6', C-3 C-4'), 125.32 (C-5), 126.11 (C-8), 126.15 (C-5'), 128.07 (C-6), 132.86 (C-7), 133.20 (C-10a), 133.80 (C-8a), 134.29 (C-1'), 140.81 (C-4), 142.72 (C-2), 144.38 (C-1), 151.99 (C-2'), 181.92 (C-9), 182.37 (C-10). **LC-MS (m/z):** 442 [M-Na+NH₄⁺]⁺, 425 [M-Na]⁺, 423 [M-Na]⁻. Purity by **HPLC-UV (254 nm)-ESI-MS:** 98%.

### Example 18: Sodium 1-amino-4-(3-methoxyphenylamino)-9,10-dioxo-9,10-dihydroanthracene 2-sulfonate.

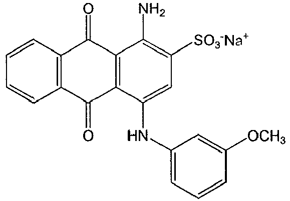

**Reaction conditions:** according to the general procedure A: 5 min, 100 °C, 80 W; pressure up to 10 bar.

**Analytical data:** mp > 300 °C, blue powder. **¹H-NMR:** δ 3.78 (s, 3H, 3'-OCH₃), 6.75 (dd, 1H, 4'-H), 6.85 (m, 2H, 2'-H, 6'-H), 7.33 (dd, 1H, 5'-H), 7.85 (m, 2H, 6-H, 7-H), 8.09 (s, 1H, 3-H), 8.27 (m, 2H, 5-H, 8-H), 10.10 (br, 2H, 1-NH₂), 11.99 (s, 1H, 4-NH). **¹³C-NMR:** δ 55.33 (C3'-OCH₃), 108.41, 110.34, (C-2', C4'), 109.32 (C-9a), 111.77 (C-4a), 115.08 (C-6'), 123.23 (C-3), 126.10 (C-5), 126.18 (C-8), 130.54 (C-5'), 132.93 (C-6), 133.35 (C-7), 133.69 (C-10a), 134.29 (C-8a), 140.61 (C-1'), 140.71 (C-4), 142.81 (C-2), 144.52 (C-1), 160.51 (C-3'), 181.97 (C-9), 182.69 (C-10). **LC-MS (m/z):** 442 [M-Na+NH₄⁺]⁺, 425 [M-Na]⁺, 423 [M-Na]⁻. Purity by **HPLC-UV (254 nm)-ESI-MS:** 100%.

### Example 19: Sodium 1-amino-4-(4-methoxyphenylamino)-9,10-dioxo-9,10-dihydroanthracene 2-sulfonate.

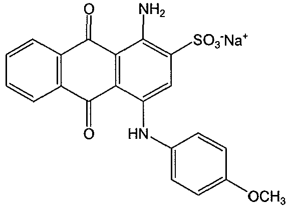

**Reaction conditions:** according to the general procedure A: 5 min, 100 °C, 80 W; pressure up to 10 bar.

**Analytical data:** mp > 300 °C, blue powder. **¹H-NMR:** δ 3.80 (s, 3H, 4'-OCH₃), 7.03 (d, 2H, 2'-H, 6'-H), 7.22 (d, 2H, 3'-H, 5'-H), 7.83 (m, 2H, 6-H, 7-H), 7.84 (s, 1H, 3-H), 8.27 (m, 2H, 5-H, 8-H), 10.10 (br, 2H, 1-NH₂), 12.04 (s, 1H, 4-NH). **¹³C-NMR:** δ 55.49 (C4'-OCH₃), 109.07 (C-9a), 110.43 (C-4a), 115.13 (C-2', C6'), 122.57(C-3), 126.03, 126.15, (C-5, C-8, C-3', C-5'), 131.77 (C-6), 132.81 (C-7), 133.08 (C-10a), 133.84 (C-8a), 134.30 (C-1'), 142.71 (C-4), 143.19 (C-2), 144.26 (C-1), 157.05 (C-4') 181.77 (C-9), 181.99 (C-10). **LC-MS (m/z):** 442 [M-Na+NH₄⁺]⁺, 425 [M-Na]⁺, 423 [M-Na]⁻. Purity by **HPLC-UV (254 nm)-ESI-MS:** 100%.

### Example 20: Sodium 1-amino-4-(4-fluorophenylamino)-9,10-dioxo-9,10-dihydroanthracene 2-sulfonate.

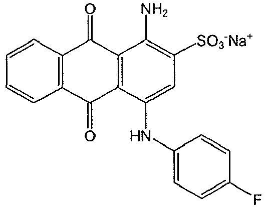

**Reaction conditions:** according to the general procedure **A:** 5 min, 120 °C, 80 W; pressure up to 10 bar.

**Analytical data:** mp > 300 °C, blue powder. **¹H-NMR:** δ 7.31 (m, 4H, 2'-H, 3'-H, 5'-H, 6'-H), 7.84 (m, 2H, 6-H, 7-H), 7.88 (s, 1H, 3-H), 8.26 (m, 2H, 5-H, 8-H), 10.07 (br, 2H, 1-NH₂), 11.94 (s, 1H, 4-NH). **¹³C-NMR:** δ 109.26 (C-9a), 111.32 (C-4a), 116.48, 116.65 (d, *J* =22.5 Hz, C-2', C6'), 122.47 (C-3), 125.85 (C-5), 125.92 (C-8), 126.07, 126.18 (d, *J* =14.0 Hz, C-3', C-5'), 132.92 (C-6), 133.31 (C-7), 133.71 (C-10a), 134.30 (C-8a), 135.73 (C-1'), 141.50 (C-4), 143.06 (C-2), 144.40 (C-1), 158.46, 160.39 (d, *J* = 242.3 Hz, C-4') 181.96 (C-9), 182.58 (C-10). **LC-MS (m/z):** 430 [M-Na+NH₄⁺]⁺, 413 [M-Na]⁺, 411[M-Na]⁻. Purity by **HPLC-UV (254 nm)-ESI-MS:** 99%.

### Example 21: Sodium 1-amino-4-(3-bromophenylamino)-9,10-dioxo-9,10-dihydroanthracene 2-sulfonate.

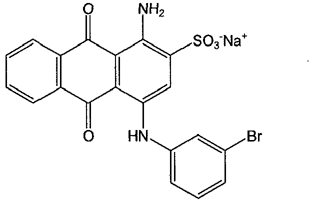

**Reaction conditions:** according to the general procedure **A:** 20 min, 120 °C, 100 W; pressure up to 10 bar.

**Analytical data:** mp > 300 °C, blue powder. **¹H-NMR:** δ 7.27 (dd, 1H, 4'-H), 7.33 (dd, 1H, 6'-H), 7.35 (dd, 1H, 5'-H), 7.49 (dd, 1H, 2'-H), 7.85 (m, 2H, 6-H, 7-H), 8.00 (s, 1H, 3-H), 8.26 (m, 2H, 5-H, 8-H), 10.03 (br, 2H, 1-NH₂), 11.78 (s, 1H, 4-NH). **¹³C-NMR:** δ 109.58 (C-9a), 112.89 (C-4a), 121.36, 122.52 (C-2', C6'), 123.07 (C-3), 124.97 (C-4'), 126.15, 126.22, 126.67 (C-5, C-8, C-5'), 131.53 (C-6), 133.04 (C-7), 133.57 (C3', C-10a), 134.27 (C-8a), 139.37 (C-1'), 141.96 (C-4), 142.62 (C-2), 144.72 (C-1), 182.18 (C-9), 183.19 (C-10). **LC-MS (m/z):** 490 [M-Na+NH₄⁺]⁺, 473 [M-Na]⁺, 471 [M-Na]⁻. Purity by **HPLC-UV (254 nm)-ESI-MS:** 99%.

### Example 22: Sodium 1-amino-4-(3,5-dimethylphenylamino)-9,10-dioxo-9,10-dihydroanthracene 2-sulfonate.

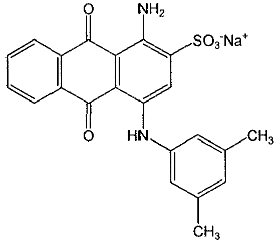

**Reaction conditions:** according to the general procedure **A:** 5 min, 100 °C, 80 W; pressure up to 10 bar.

**Analytical data:** mp > 300 °C, blue powder. **¹H-NMR:** δ 2.29 (s, 6H, 3'-CH₃, 5'-CH₃), 6.85 (s, 1H, 4'-H), 6.89 (s, 2H, 2'-H, 6'-H), 7.84 (m, 2H, 6-H, 7-H), 7.99 (s, 1H, 3-H), 8.27 (m, 2H, 5-H, 8-H), 10.10 (br, 2H, 1-NH₂), 12.01 (s, 1H, 4-NH). **¹³C-NMR:** δ 21.07 (C3'-CH₃, C5'-CH₃), 109.18 (C-9a), 111.21 (C-4a), 121.18 (C-2', C6'), 123.11 (C-3), 126.07 (C-4'), 126.16, 126.42 (C-5, C-8), 132.87 (C-6), 133.23 (C-7), 133.76 (C-10a), 134.29 (C-8a), 139.05 (C-3' and C-5'), 139.16 (C-1'), 141.40 (C-4), 142.95 (C-2), 144.42 (C-1), 181.87 (C-9), 182.40 (C-10). **LC-MS (m/z):** 440 [M-Na+NH₄⁺]⁺, 422 [M-Na]⁺, 420 [M-Na]⁻. Purity by **HPLC-UV (254 nm)-ESI-MS:** 99%.

### Example 23: Sodium 1-amino-4-(3-chlorophenylamino)-9,10-dioxo-9,10-dihydroanthracene 2-sulfonate.

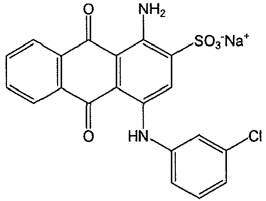

**Reaction conditions:** according to the general procedure **A:** 5 min, 100 °C, 80 W; pressure up to 10 bar.

**Analytical data:** mp > 300 °C, blue powder. **¹H-NMR:** δ 7.20 (dd, 1H, 6'-H), 7.23 (dd, 1H, 4'-H), 7.35 (dd, 1H, 2'-H), 7.43 (dd, 1H, 5'-H), 7.85 (m, 2H, 6-H, 7-H), 8.00 (s, 1H, 3-H), 8.24 (m, 2H, 5-H, 8-H), 10.02 (br, 2H, 1-NH₂), 11.78 (s, 1H, 4-NH). **¹³C-NMR:** δ 109.59 (C-9a), 112.90 (C-4a), 120.96 (C-6'), 122.09 (C-3), 123.10, 123.77 (C-2', C-4'), 126.16 (C-5), 126.22 (C-8), 131.28 (C-6), 133.05 (C-7), 133.58 (C-3', C-5') 134.13 (C-10a), 134.28, (C-8a), 139.37 (C-1'), 141.56 (C-4), 142.62 (C-2), 144.72 (C-1), 182.19 (C-9), 183.20 (C-10). **LC-MS (m/z):** 439 [M-Na]⁺, 437 [M-Na]⁻. **LC-MS (m/z):** 446 [M-Na+NH₄⁺]⁺, 429 [M-Na]⁺, 427 [M-Na]⁻. Purity by **HPLC-UV (254 nm)-ESI-MS:** 95%.

### Example 24: Sodium 1-amino-4-(3-amino-2-methylphenylamino)-9,10-dioxo-9,10-dihydroanthracene 2-sulfonate.

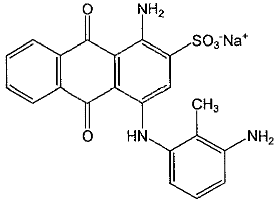

**Reaction conditions:** according to the general procedure **A:** 5 min, 120 °C, 80 W; pressure up to 10 bar.

**Analytical data:** mp > 300 °C, blue powder. **¹H-NMR:** δ 1.99 (s, 3H, C2'-CH₃), 5.05 (bs, 2H, 3'-NH₂), 6.44 (d, 1H, 6'-H), 6.59 (d, 1H, 4'-H), 6.96 (dd, 1H, 5'-H), 7.66 (s, 1H, 3-H), 7.83 (m, 2H, 6-H, 7-H), 8.28 (m, 2H, 5-H, 8-H), 10.17 (br, 2H, 1-NH₂), 12.04 (s, 1H, 4-NH). **¹³C-NMR:** δ 11.84 (C2'-CH₃), 108.90 (C-9a), 109.96 (C-4a), 112.21 (C-6'), 113.66 (C-4'), 116.45 (C-2'), 123.14 (C-3), 126.03 (C-5), 126.13 (C-8), 126.65 (C-5'), 132.72 (C-6), 132.98 (C-7), 133.89 (C-10a), 134.33 (C-8a), 137.58 (C-1'), 143.22 (C-4), 143.44 (C-2), 144.16 (C-1), 148.27 (C-3'), 181.63 (C-9), 181.92 (C-10). **LC-MS (m/z):** 441 [M-Na+NH₄⁺]⁺, 424 [M-Na]⁺, 422 [M-Na]⁻. Purity by **HPLC-UV (254 nm)-ESI-MS:** 96%.

### Example 25: Sodium 1-amino-4-(3-carboxy-4-hydroxyphenylamino)-9,10-dioxo-9,10-dihydroanthracene 2-sulfonate.

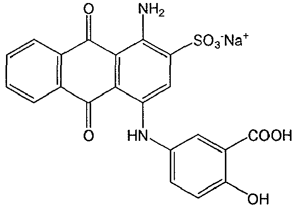

**Reaction conditions:** according to the general procedure A: 5 min, 120 °C, 80 W; pressure up to 10 bar.

**Analytical data:** mp > 300 °C, blue powder. **¹H-NMR:** δ 6.70 (d, 1H, 5'-H), 7.07 (dd, 1H, 6'-H), 7.53 (d, 1H, 2'-H), 7.75 (s, 1H, 3-H), 7.82 (m, 2H, 6-H, 7-H), 8.27 (m, 2H, 5-H, 8-H), 10.18 (br, 2H, 1-NH₂), 12.07 (s, 1H, 4-NH). **¹³C-NMR:** δ 108.86 (C-9a), 114.37 (C-4a), 116.96 (C-2'), 122.74 (C-3), 125.98 (C-5), 126.09 (C-8), 126.67 (C-6'), 128.87 (C-5'), 132.66 (C-6), 132.80 (C-7), 132.93 (C-3'), 133.59 (C-10a), 134.29 (C-8a), 134.62 (C-1'), 143.30 (C-4), 143.96 (C-2), 144.15 (C-1), 161.45 (C-4') 170.76 (COOH), 181.40 (C-9), 181.59 (C-10). **LC-MS (m/z):** 472 [M-Na+NH₄⁺]⁺, 455 [M-Na]⁺, 453 [M-Na]⁻. Purity by **HPLC-UV (254 nm)-ESI-MS:** 95%.

### Example 26: Disodium 1-amino-4-(2-sulfophenylamino)-9,10-dioxo-9,10-dihydroanthracene 2-sulfonate.

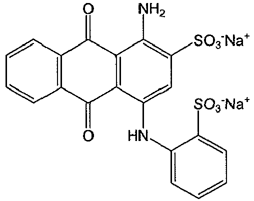

**Reaction conditions:** according to the general procedure **A:** 5 min, 120 °C, 80 W; pressure up to 10 bar.

**Analytical data:** mp > 300 °C, blue powder. **¹H-NMR:** 7.02 (ddd, 1H, 4'-H), 7.13 (dd, 1H, 6'-H), 7.28 (ddd, 1H, 5'-H), 7.75 (dd, 1H, 3'-H), 7.80 (m, 2H, 6-H, 7-H), 7.99 (s, 1H, 3-H), 8.23 (m, 2H, 5-H, 8-H), 10.03 (br, 2H, 1-NH₂), 11.78 (s, 1H, 4-NH). **¹³C-NMR:** 109.72 (C-9a), 113.42 (C-4a), 122.32 (C-4'), 122.69 (C-6'), 124.86 (C-3), 126.05 (C-5), 126.09 (C-8), 128.01 (C-3'), 129.45 (C-5'), 132.84 (C-6), 132.98 (C-7), 133.99 (C-10a), 134.24 (C-8a), 137.43 (C-1'), 139.14 (C-4), 139.59 (C-2'), 141.67 (C-2), 144.62 (C-1), 181.51 (C-9), 182.25 (C-10). **LC-MS (m/z):** 497 [M-Na]⁺, 495 [M-Na]⁻, 475 [M-2Na]⁺, 473 [M-2Na]⁻, 236 [M-2Na]²⁻. Purity by **HPLC-UV (254 nm)-ESI-MS:** 98%.

### Example 27: Disodium 1-amino-4-(4-sulfophenylamino)-9,10-dioxo-9,10-dihydroanthracene 2-sulfonate.

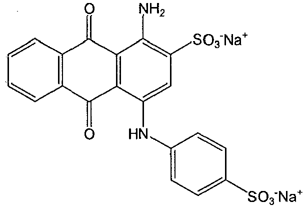

**Reaction conditions:** according to the general procedure **A:** 5 min, 120 °C, 80 W; pressure up to 10 bar.

**Analytical data:** mp > 300 °C, blue powder. **¹H-NMR:** δ 7.19 (d, 2H, 2'-H, 6'-H), 7.62 (d, 2H, 3'-H, 5'-H), 7.84 (m, 2H, 6-H, 7-H), 8.04 (s, 1H, 3-H), 8.27 (m, 2H, 5-H, 8-H), 10.10 (br, 2H, 1-NH₂), 12.01 (s, 1H, 4-NH). **¹³C-NMR:** 109.33 (C-9a), 111.87 (C-4a), 121.76 (C-2', C-6'), 122.98 (C-3), 126.12 (C-5), 126.17 (C-8), 127.14 (C-3', C-5'), 132.92 (C-6), 133.34 (C-7), 133.71 (C-10a), 134.29 (C-8a), 139.40 (C-1'), 140.45 (C-4), 142.86 (C-2), 144.56 (C-4'), 144.58 (C-1), 181.98 (C-9), 182.72 (C-10). **LC-MS (m/z):** 492 [M-Na+NH₄⁺]⁺, 473 [M-2Na]⁻, 236 [M-2Na]²⁻. Purity by **HPLC-UV (254 nm)-ESI-MS:** 96%.

### Example 28: 1-Amino-4-[4-([1,3]diazine-2-ylamino)-3-sulfonatophenylamino]-9,10-dioxo-9,10 dihydroanthracene-2-sulfonic acid disodium salt.

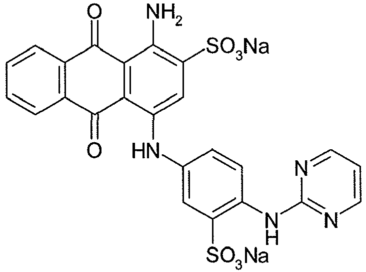

According to the general procedure **B,** the solution of 160 mg (1 mmol) of 2-bromopyrimidine and 268 mg (0.5 mmol) of 1-amino-4-[4-amino-3-sulfophenylamino]-9,10-dioxo-9,10 dihydroanthracene-2-sulfonic acid disodium salt was stirred at 0-5 °C and then the temperature was increased for refluxing at 120 °C for three days. RP-FCC was performed with a methanol/water (2:3) eluent.

**Analytical data:** yield (295 mg) 97 %, a blue powder, mp > 300 °C.
**¹H-NMR:** δ 6.88 (dd, 1H, 4"-H), 7.26 (dd, 1H, 6'-H), 7.55 (d, 1H, 2'-H), 7.84 (m, 2H, 6-H, 7-H), 7.92 (s, 1H, 3-H), 8.28 (m, 2H, 5-H, 8-H), 8.52 (d, 2H, 3"-H, 5"-H), 8.58 (d, 1H, 5'-H), 10.15 (br, 2H, 1-NH2), 10.03 (br, 1H, 4'-NH), 12.12 (br, 1H, 4-NH). **¹³C-NMR:** δ 108.89 (C-9a), 110,56 (C-4a), 112.61 (C-5"), 119.67 (C-5'), 122.41 (C-3, C-2'), 124.33 (C-6'), 125.76 (C-5, C-8), 131.23 (C-1'), 132.37 (C-6), 132.67 (C-7), 133.54 (C-4', C-10a), 134.01 (C-8a), 136.06 (C-3'), 141.78 (C-4), 142.98 (C-2), 144.12 (C-1), 157.80 (C-4", C-6"), 159.24 (C-2"), 181.51 (C-9), 181.84 (C-10). **LC-MS (m/z):** 585 [M-2Na+NH₄⁺]⁺. Purity by **HPLC-UV (254 nm)-ESI-MS:** 95%.

### Example 29: 1-Amino-4-[4-phenylamino-3-sulfophenylamino]-9,10-dioxo-9,10 dihydroanthracene-2-sulfonic acid disodium salt.

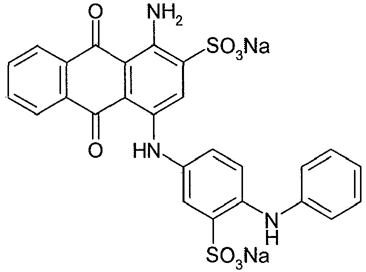

**Reaction conditions:** according to the general procedure **A:** 5 min, 120 °C, 100 W; pressure up to 10 bar.

**Analytical data:** mp > 300 °C, blue powder.
**¹H-NMR:** δ 6.91 (dd, 1H, 4"-H), 7.12 (m, 3H), 7.30 (m, 3H), 7.50 (d, 1H), 7.83 (s, 1H, 3-H), 7.83 (m, 2H, 5-H, 8-H), 8.28 (m, 2H, 6-H, 7-H), 12.12 (br, 1H, 4-NH).
**¹³C-NMR:** δ 109.11, 110.42, 115.79, 118.28, 120.98, 122.68, 124.10, 126.07, 126.13, 129.51, 132.78, 133.01, 133.89, 134.28, 135.31, 137.88, 142.38, 142.76, 143.25, 144.25, 181.75, 181.88.
**LC-MS (m/z):** 564 [M-2Na]⁻, 281[M-2Na]²⁻, 583 [M-2Na+NH₄⁺]⁺.
Purity by **HPLC-UV (254 nm)-ESI-MS:** 100%.

### Example 30: 1-Amino-4-[4-phenoxy-3-sulfophenylamino]-9,10-dioxo-9,10 dihydroanthracene-2-sulfonic acid disodium salt.

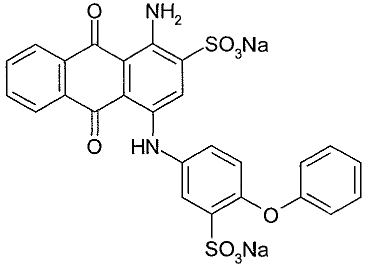

**Reaction conditions:** according to the general procedure **A:** 5 min, 120 °C, 100 W; pressure up to 10 bar.

**Analytical data:** mp > 300 °C, blue powder.
**¹H-NMR:** δ 6.84 (d, 1H), 6.98 (dd, 2H), 7.06 (dd, 1H), 7.21 (dd, 1H), 7.34 (dd, 2H), 7.63 (d, 1H), 7.84 (m, 2H, 5-H, 8-H), 7.91 (s, 1H, 3-H), 8.28 (m, 2H, 6-H, 7-H), 10.15 (br, 2H, 1-NH2), 12.07 (br, 1H, 4-NH).
**¹³C-NMR:** δ 109.22, 111.20, 119.01, 121.31, 122.61, 122.72, 124.26, 125.18, 126.14, 129.63, 132.87, 133.22, 133.80, 134.28, 140.86, 141.60, 143.14, 144.44, 150.71, 158.12, 181.91, 182.47.
**LC-MS (m/z):** 565 [M-2Na]⁻, 282 [M-2Na]²⁻, 584 [M-2Na+NH₄⁺]⁺.
Purity by **HPLC-UV (254 nm)-ESI-MS:** 99%.

### Example 31: 1-Amino-4-[4-phenylamino-3-carboxyphenylamino]-9,10-dioxo-9,10 dihydroanthracene-2- sulfonic acid sodium salt.

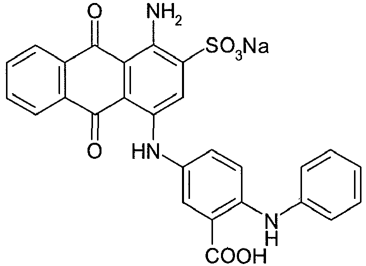

**Reaction conditions:** according to the general procedures **E, F,** and **G:**

**Analytical data:** mp > 300 °C, blue powder.
**¹H-NMR:** δ 7.09 (dd, 1H, 4"-H), 7.29 (m, 3H), 7.37 (m, 3H), 7.78 (d, 1H), 7.81 (s, 1H, 3-H), 7.83 (m, 2H, 5-H, 8-H), 8.27 (m, 2H, 6-H, 7-H), 9.60 (br, 2H, 1-NH2), 10.03 (br, 1H, 4'-NH), 12.00 (br, 1H, 4-NH).
**¹³C-NMR:** δ 109.1, 110.6, 113.5, 115.3, 121.6, 122.5, 123.3, 126.0, 126.1, 127.8, 128.8, 129.7, 131.4, 132.8, 133.1, 133.8, 134.3, 140.6, 142.6, 143.19, 144.3, 144.8, 169.5, 181.8, 182.1.
**LC-MS (m/z):** 528 [M-Na]⁻, 529 [M-Na]⁺, 528 [M-Na]⁻, 547 [M-Na+NH₄⁺]⁺.
Purity by **HPLC-UV (254 nm)-ESI-MS:** 98%.

### Example 32: 1-Amino-4-[4-phenylamino-3-sulfophenylamino]-9,10-dioxo-9,10 dihydroanthracene-2-carboxylic acid sodium salt.

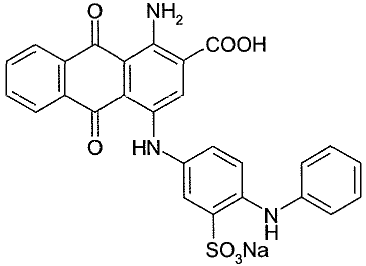

**Reaction conditions:** according to the general procedure A: 5 min, 120 °C, 100 W; pressure up to 10 bar.

**Analytical data:** mp > 300 °C, blue powder.
**¹H-NMR:** δ 6.93 (dd, 1H, 4"-H), 7.13 (d, 2H), 7.21 (d, 1H), 7.31 (dd, 2H), 7.35 (d, 1H), 7.56 (d, 1H), 7.88 (m, 2H, 6-H, 7-H), 8.19 (s, 1H, 3-H), 8.29 (m, 2H, 5-H, 8-H), 11.71 (br, 1H, 4-NH).
**¹³C-NMR:** δ 110.44, 113.86, 115.91, 118.25, 121.00, 122.97, 123.69, 125.92, 126.18, 126.29, 129.17, 129.7, 133.19, 133.47, 133.163, 134.13, 135.25, 137.72, 140.82, 142.37, 147.24, 167.68, 182.29, 183.13.
**LC-MS (m/z):** 528 [M-Na]⁻, 529 [M-Na]⁺, 528 [M-Na]⁻, 547 [M-Na+NH₄⁺]⁺.
Purity by **HPLC-UV (254 nm)-ESI-MS:** 98%.

### Example 33: 1-Amino-2-methyl-4-[4-phenylamino-3-sulfophenylamino]-9,10-dioxo-9,10 dihydroanthracene sodium salt.

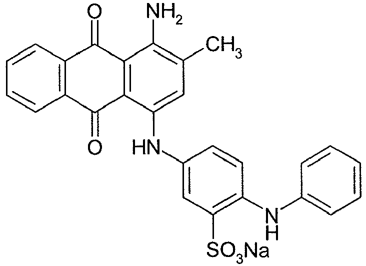

**Reaction conditions:** according to the general procedure **A:** 5 min, 120 °C, 100 W; pressure up to 10 bar.

**Analytical data:** mp > 300 °C, blue powder.
**¹H-NMR:** δ 2.25 (s, 3H, CH₃), 6.91 (dd, 1H, 4"-H), 7.11 (d, 2H), 7.18 (dd, 1H), 7.30 (m, 4H), 7.51 (d, 1H), 7.81 (m, 2H, 5-H, 8-H), 8.27 (m, 2H, 6-H, 7-H), 8.55 (s, 1H, 3-H), 12.27 (br, 1H, 4-NH).
**¹³C-NMR:** δ 18.77, 107.9, 108.6, 115.9, 118.2, 120.9, 123.9, 124.3, 125.9, 126.1, 126.2, 129.4, 129.5, 132.7, 134.1, 135.2, 137.6, 137.7, 142.4, 143.7, 147.0, 181.2, 181.8. **LC-MS (m/z):** 498 [M-Na]⁻, 500 [M-Na]⁺.
Purity by **HPLC-UV (254 nm)-ESI-MS:** 96%.

### Biological Assays

### Example 56: Human platelet membrane preparation

Membranes were prepared from human outdated platelets from the blood bank. Outdated human platelets were obtained from the University of Bonn blood bank 4-5 days after donation. Platelet rich plasma was washed by centrifugation (10 min at 1000 g) in a buffer consisting of 50 mM Tris-HCl, pH 7.4, 5 mM EDTA and 150 mM NaCl. The supernatant was centrifuged twice (48400 g for 60 min) and the resultant platelet pellet were resuspended in 5 mM Tris-HCl (pH 7.4) containing 5 mM EDTA. The platelets were homogenized by a homogenizer. The final suspension was stored frozen as multiple aliquots at -80°C until needed. Protein concentrations were determined by the method of Lowry using a Sigma Chemie protein assay kit.

### Example 57: Radioligand binding assay

Radioligand binding assays were performed using 5 nM [³H]PSB-0413 and 100 µg of protein in Tris-HCl buffer 50 mM, pH 7.4, as previously described. (El-Tayeb, A., Griessmeier, K.J, Muller, C.E. Bioorg. Med. Chem. Lett. 2005, 15, 5450-52; Atzler, K. Doctoral Thesis, University of Bonn, 2006). Competition by 10 µM of test compound was initially determined. The mixture was incubated for 1 h at rt followed by filtration through GF/B filters. Nonspecific binding was determined with 1 mM ADP or 250 µM RB 2. For potent compounds concentration-inhibition curves were determined using at least 6-7 different concentrations spanning 3 orders of magnitude. At least three independent experiments were performed each in triplicate.

In Table 3 Kᵢ values of selected compounds as antagonist of human platelets are given. In order to investigate selectivity of selected compounds, potencies at other P2Y receptor subtypes were determined for comparison.

Selected compounds were investigated for their functionality in GTP shift experiments. Radioligand binding studies as described above were performed in the presence and absence of GTP (100 µM). Agonists, such as ADP, showed a significant right-shift of the concentration-inhibition curve, while the investigated compounds (e.g. Reactive Blue 2, and entry 33 and entry 37 in Table 3) did not. These results show that the compounds behave as antagonists rather than agonists at P2Y₁₂ receptors.

### Example 58: Determination of potency as inhibitors of P2Y₂, P2Y₄, and P2Y₆ receptors

Inhibition of agonist-induced calcium mobilization was determined as previously described (Kaulich M. et al, Drug Dev. Res. 2003, 59, 72-81) using NG108-15 cells for the mouse P2Y₂ receptor, or human recombinant P2Y receptors stably expressed in human astrocytoma cells (El-Tayeb, A. et al., J. Med. Chem. 2006, 49, 7076-87).

### Data analysis

The data were analyzed using GraphPad Prism 4.0 (GraphPad Software, Inc., San Diego, CA). The values are presented as means of the percent inhibition at 10 µM ± SEM (n=3) or Kᵢ value in µM ± SEM (n=3).

**Table 3. Affinities of selected compounds for human platelet P2Y₁₂ receptors and antagonistic potency of selected compounds at P2Y receptor subtypes The data represent means ± SEM of usually three separate experiments each run in triplicate. (Abbreviations: h = human, m = mouse; n.d. = not determined).**

| **Entry** | ***Structure*** | **hP2Y₂** Receptor IC₅₀ (µM ± SEM) | **mP2Y₂** Receptor IC₅₀ (µM ± SEM) | **hP2Y₄** Receptor IC₅₀ (µM ± SEM) | **rP2Y₆** Receptor IC₅₀ (µM ± SEM) | **hP2Y₁₂** Receptor Kᵢ [µM± SEM] vs. [³H]PSB -0413 |
|---|---|---|---|---|---|---|
| **1** | 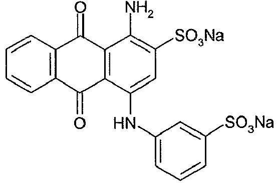 | n.d. | n.d. | **18.5** ± 2.5 | ca. 100 | **3.81** ± 1.08 |
| **2** | 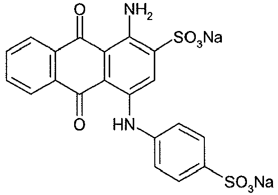 | n.d. | n.d. | n.d. | n.d. | **3.13** ± 0.39 |
| **3** | 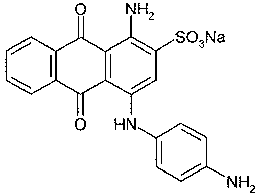 | n.d. | n.d. | n.d. | n.d. | **>10** |
| **4** | 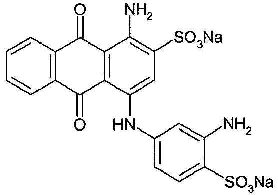 | n.d. | n.d. | n.d. | n.d. | **2.10** ± 0.47 |
| **5** | 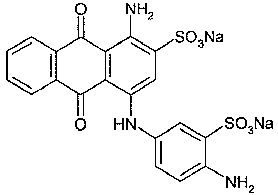 | n.d. | n.d. | 11.0 ± 3.0 | 24.3 ± 3.4 | **2.39** ± 0.65 |
| **6** | 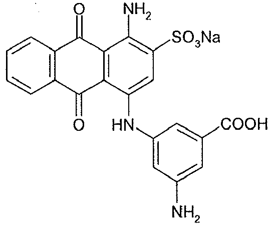 | n.d. | n.d. | n.d. | n.d. | **7.07** ± 1.70 |
| **7** | 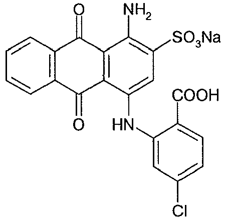 | n.d. | n.d. | n.d. | n.d. | **12.2** ± 0.3 |
| **8** | 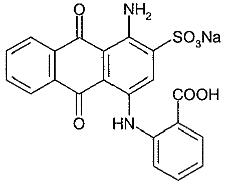 | n.d. | n.d. | n.d. | n.d. | **25.1** ± 7.0 |
| **9** | 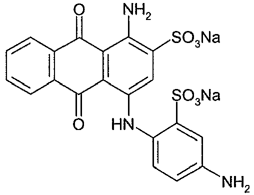 | n.d. | n.d. | n.d. | n.d. | **19.74** ± 8.27 |
| **10** | 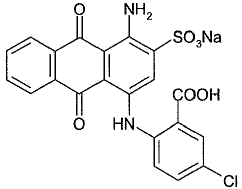 | n.d. | n.d. | n.d. | n.d. | **12.3** ± 1.7 |
| **11** | 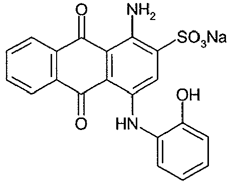 | n.d. | n.d. | n.d. | n.d. | **ca. 10** |
| **12** | 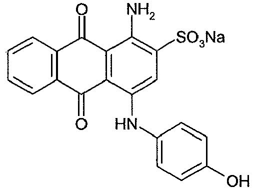 | n.d. | n.d. | n.d. | n.d. | **ca. 10** |
| **13** | 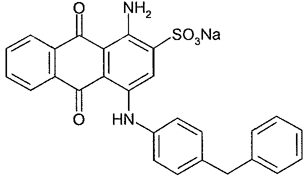 | n.d. | n.d. | n.d. | n.d. | **0.614** |
| **14** | 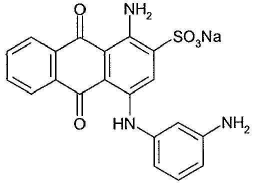 | n.d. | n.d. | n.d. | n.d. | **>10** |
| **15** | 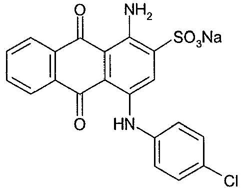 | 4.46 ± 0.75 | 20.0 ± 7.0 | >>3 | >>10 | **7,35** ± 1,72 |
| **16** | 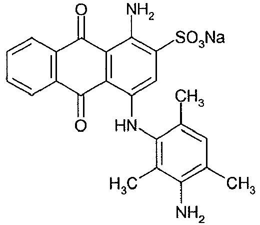 | n.d. | n.d. | n.d. | n.d. | **>10** |
| **17** | 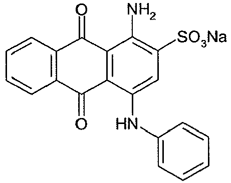 | 5.61 ± 0.47 | 11.1 ± 3.0 | n.d. | n.d. | **9.83** ± 2.43 |
| **18** | 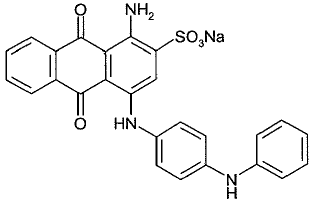 | n.d. | 30.0 ± 7.0 | >>3 | >>1 | **1.85** ± 0.57 |
| **19** | 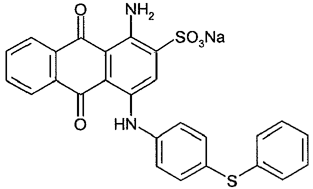 | n.d. | n.d. | n.d. | n.d. | **0.884** ± 0.525 |
| **20** | 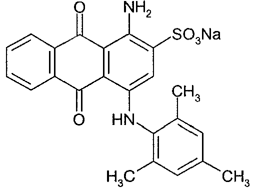 | n.d. | n.d. | n.d. | n.d. | **>10** |
| **21** | 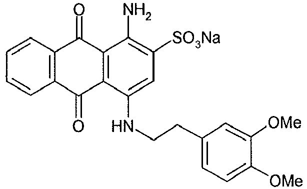 | n.d. | n.d. | >30 | >100 | **>10** |
| **22** | 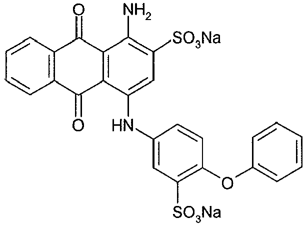 | n.d. | n.d. | n.d. | n.d. | **0.063** ± 0.009 |
| **23** | 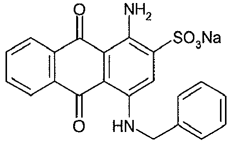 | n.d. | n.d. | n.d. | n.d. | **>>10** |
| **24** | 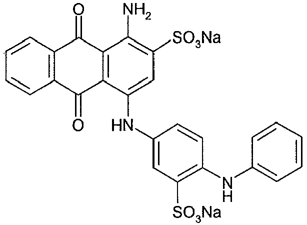 | n.d. | n.d. | n.d. | n.d. | **0.0249** ± 0.0032 |
| **25** | 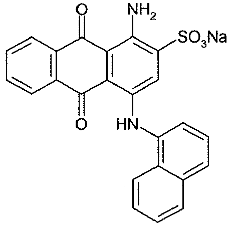 | 6.67 ± 0.32 | 10.7 ± 1.0 | 10.1 ± 1.4 | 26.7 | **>10** |
| **26** | 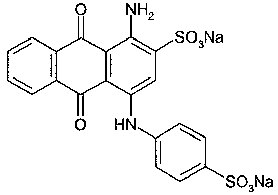 | n.d. | n.d. | n.d. | n.d. | **3.13** ± 0.39 |
| **27** | 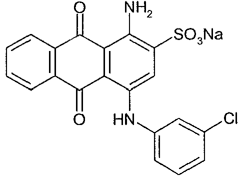 | 24.5 ± 10.4 | > 100 | >> 3 | >> 100 | **6.76** ± 2.07 |
| **28** | 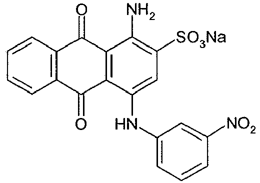 | 7.52 ± 0.82 | >100 | n.d. | n.d. | **>>10** |
| **29** | 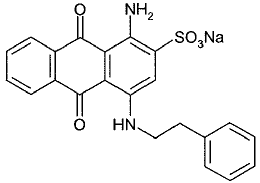 | n.d. | n.d. | n.d. | n.d. | **>>10** |
| **30** | 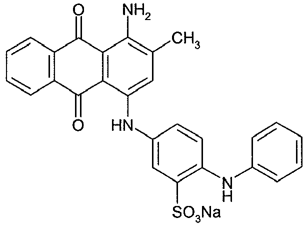 | n.d. | n.d. | n.d. | n.d. | **0.541** |
| **31** | 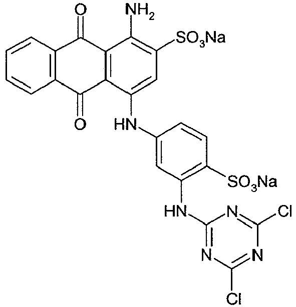 | n.d. | n.d. | n.d. | n.d. | **17.1** ± 7.8 |
| **32** | 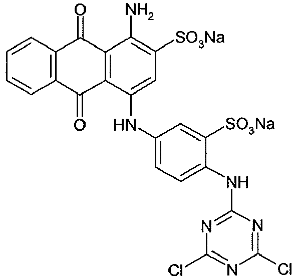 | n.d. | n.d. | n.d. | n.d. | **2.45** ± 1.00 |
| **33** | 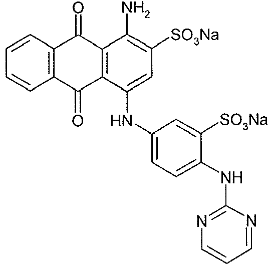 | n.d. | n.d. | 9.04 ± 1.20 | 28.6 ± 2.7 | **0.0507** ± 0.0160 |
| **34** | 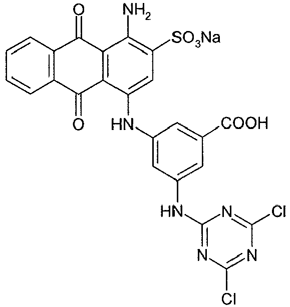 | n.d. | n.d. | n.d. | n.d. | **3.76** ± 1.03 |
| **35** | 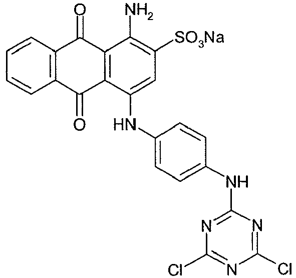 | n.d. | n.d. | n.d. | n.d. | **1.90** ± 0.24 |
| **36** | 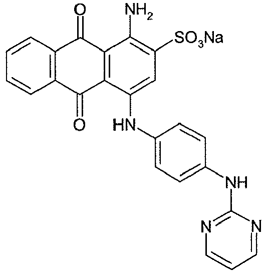 | n.d. | n.d. | n.d. | n.d. | ca. 10 |
| **37** | 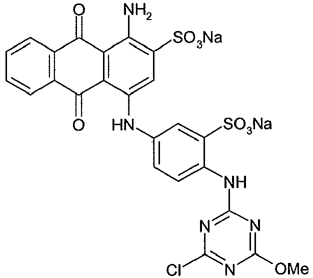 | n.d. | n.d. | n.d. | n.d. | **0.66** ± 0.12 |
| **38** | 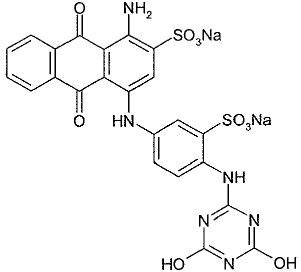 | n.d. | n.d. | n.d. | n.d. | **ca. 10** |
| **39** | 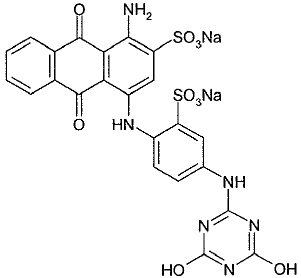 | n.d. | n.d. | n.d. | n.d. | **>>10** |
| **40** | 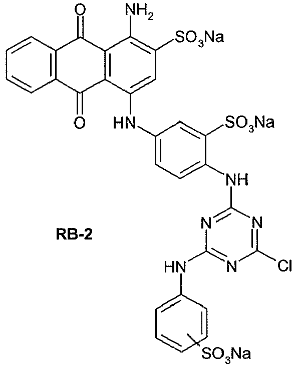 | 12.0 ± 5.7 | 2.90 | 9.79 ± 3.91 | 4.34 ± 0.89 | **0.68** ± 0.26 |

## Claims

1. A compound
(A) of Formula I: 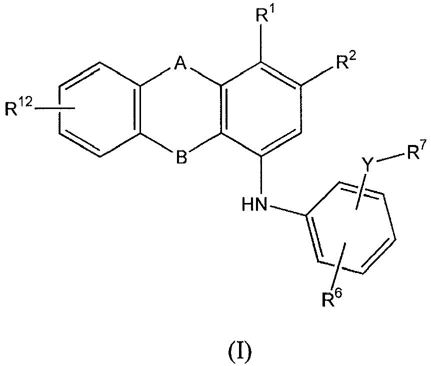 wherein:
A and B are independently selected from the group consisting of CH₂, O, S, NH, C=O, C=NH, C=S, or C=N-OH;
R¹ is independently selected from the group consisting of hydrogen, unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -C(O)R, -C(O)OR, -C(O)NRR', -NRR', - S(O)₂R, -S(O)₂OR, and -S(O)₂NRR';
R² is independently selected from the group consisting of unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5-to 10-membered heteroalicyclic ring wherein 1 to 3 ring atoms are independently nitrogen, oxygen or sulfur, -OR, -C(O)R, -C(O)OR, -C(O)NRR', -NRR', - S(O)₂R, -S(O)₂OR, and - S(O)₂NRR';
R⁶ represents substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR, -C(O)NRR', - S(O)₂R, -S(O)₂OR, and -S(O)₂NRR'; wherein in the meta position of the aryl ring R⁶ is a tetrazolyl, sulfonic acid or carboxylic acid group or the respective sodium or potassium salt;
Y is selected from the group consisting of NH, O, S, CH₂, CH₂CH₂, C(O), C(O)O, or S(O)₂O;
R⁷ is unsubstituted C₆-C₁₄ aryl;
R¹² represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR, -C(O)NRR', - S(O)₂R, -S(O)₂OR, and -S(O)₂NRR'; and
R and R' are independently selected from the group consisting of hydrogen and unsubstituted C₁-C₄ alkyl;
or a pharmaceutically acceptable salt or thereof
wherein if the C₁-C₁₀ alkyl is substituted, the substituent group(s) is one or more, for example one or two groups, individually selected from the group consisting of C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰ R¹¹ where R¹⁰ and R¹¹ are independently selected from the group consisting of hydrogen, C₁-C₄ alkyl, C₃-C₈ cycloalkyl, C₆-C₁₄ aryl, carbonyl, acetyl, sulfonyl, amino, and trifluoromethanesulfonyl, or R¹⁰ and R¹¹, together with the nitrogen atom to which they are attached, combine to form a five-or six-membered heteroalicyclic ring,
wherein if the C₃-C₈ cycloalkyl is substituted, the substituent group(s) is one or more, for example one or two groups, individually selected from C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, 0-carboxy, nitro, silyl, sulfinyl, sulfonyl, amino, and NR¹⁰ R¹¹ with R¹⁰ and R¹¹ as defined above,
wherein if the C₆-C₁₄ aryl is substituted, the substituted group(s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and NR¹⁰ R¹¹ with R¹⁰ and R¹¹ as defined above,
wherein if the 5- to 10-membered heteroaryl is substituted, the substituted group(s) is one or more, for example one or two substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰R¹¹ with R¹⁰ and R¹¹ as defined above, and
wherein if the 5- to 10-membered heteroalicyclic ring is substituted the the substituted group (s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰ R¹¹ with R¹⁰ and R¹¹ as defined above;
(B) selected from the group consisting of: 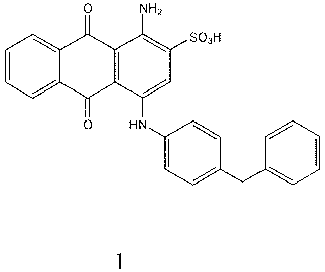 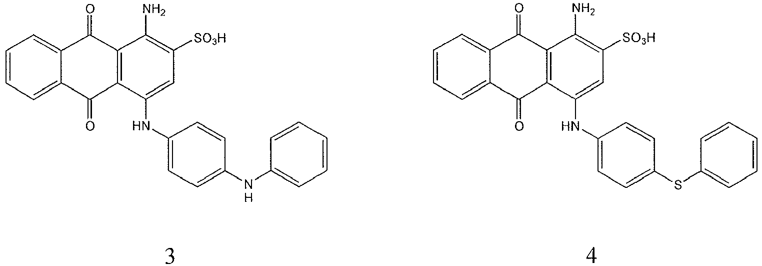 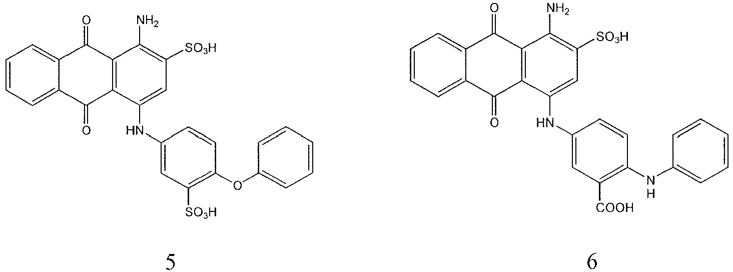 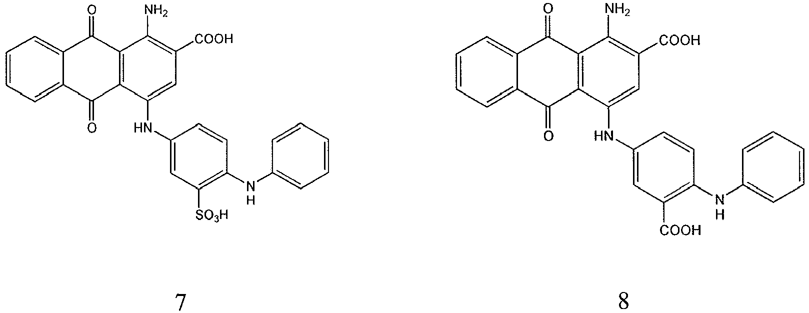 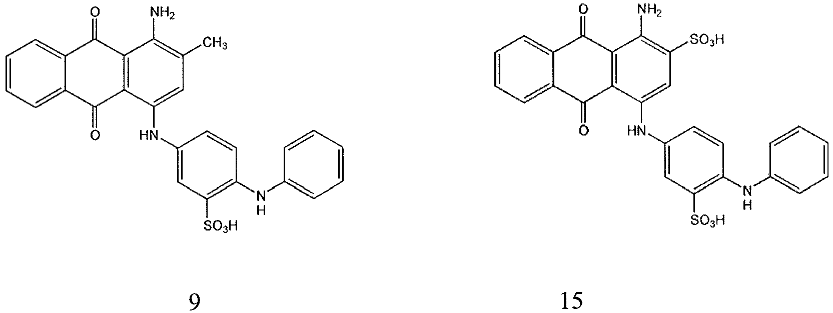 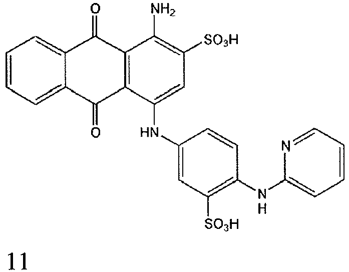 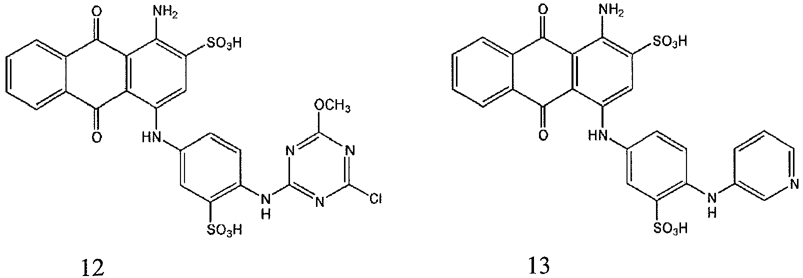 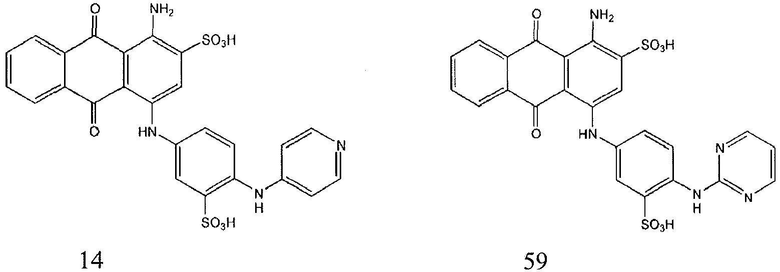 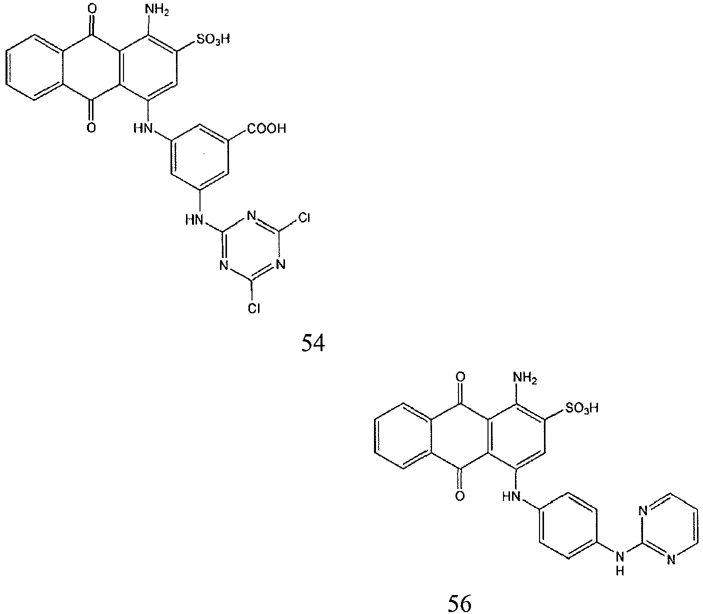 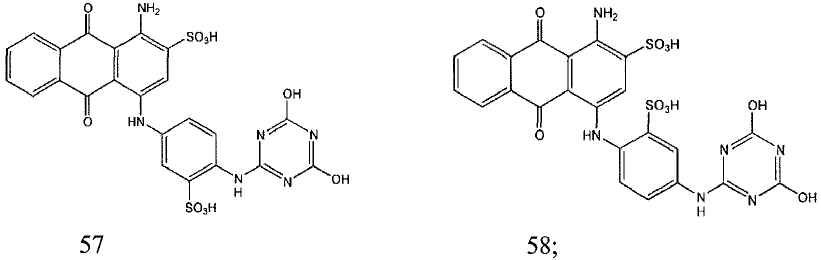 or pharmaceutically acceptable salts thereof; or
(C) selected from the group consisting of: 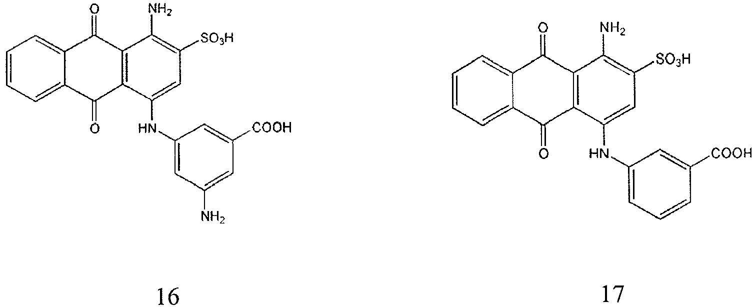 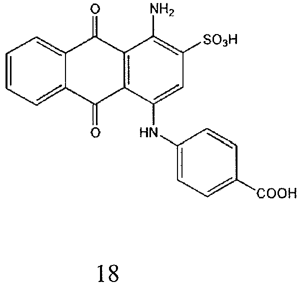 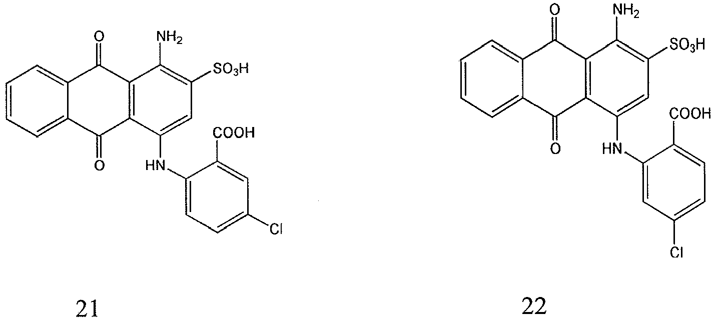 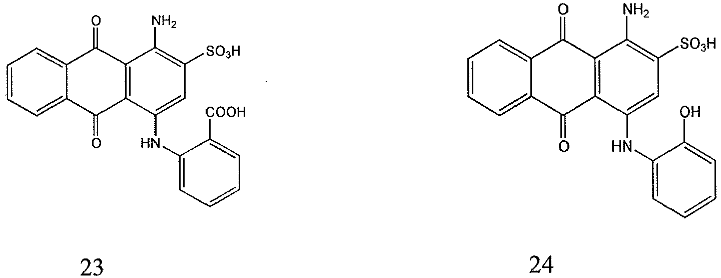 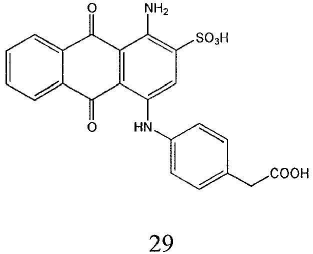 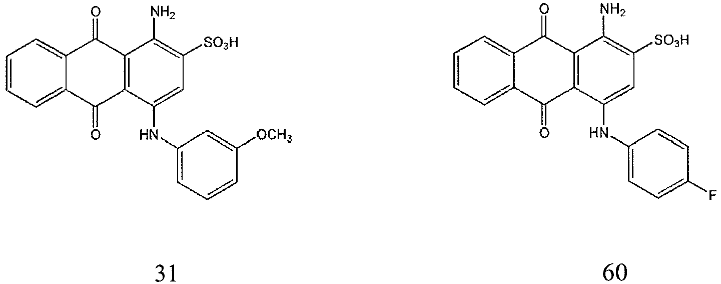 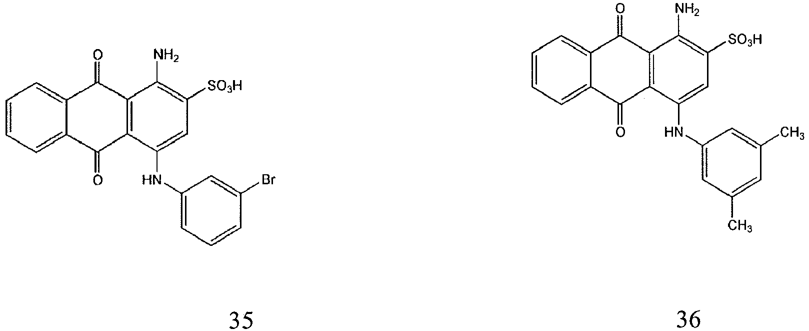 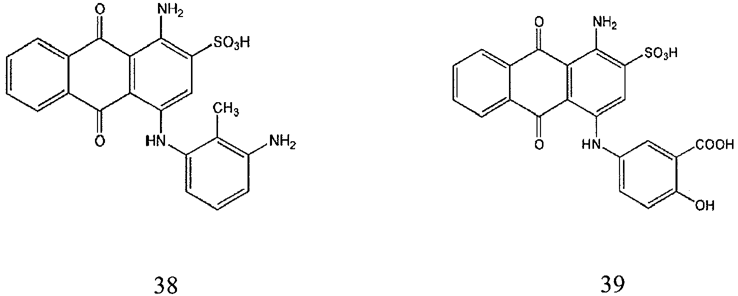 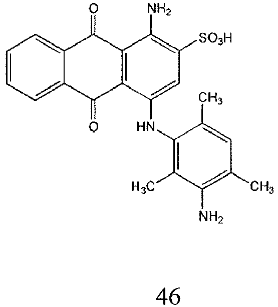 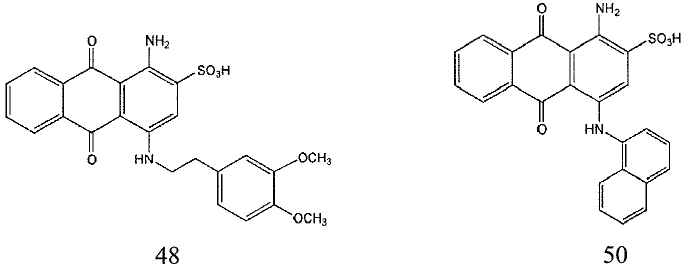 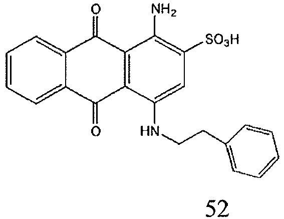 or pharmaceutically acceptable salts thereof.

2. The compound of claim 1, wherein R⁷ is unsubstituted C₆ aryl.

3. The compound of claim 1 or 2, wherein Y is NH and/or wherein R⁶ represents substituents selected from the group consisting of hydrogen, -S(O)₂OR, -C(O)OR, tetrazolyl, sulfonyl, carboxy, amino, halogen, C₁-C₄ alkoxy, C₁-C₄ alkyl, hydroxy, and nitro.

4. The compound of any one of claims 1-3, wherein
(1) R¹² represents one to four substituents selected from the group consisting of hydrogen, halo, amino, unsubstituted C₁-C₄ alkyl or alkoxy, or hydroxy; and/or
(2) at least one of A and B is C=O or both A and B are C=O; and/or
(3) R¹ is amino; and/or
(4) R² is tetrazolyl, -S(O)₂OR or -C(O)OR.

5. A pharmaceutical composition comprising a compound or salt of any one of claims 1 to 4 and a pharmaceutically acceptable carrier or excipient and optionally further comprising an additional medicament or drug.

6. A compound according to any one of claims 1 (A) or (B) to 4 or a pharmaceutically acceptable salt thereof for use in a method of inhibiting platelet aggregation or for use in the prevention, alleviation and/or treatment of a disease or disorder related to P2Y₁₂ receptor activity.

7. A compound according to Formula III: 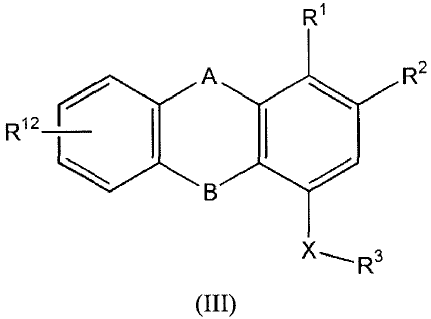 wherein:
A and B are C=O;
X is NH;
R¹ is -NH₂;
R² is selected from the group -C(O)OH and -S(O)₂OH;
R³ is selected from the group consisting of unsubstituted or substituted C₁-C₁₀ alkyl, unsubstituted or substituted C₁-C₁₀ alkenyl, unsubstituted or substituted C₁-C₁₀ alkynyl, unsubstituted or substituted C₃-C₈ cycloalkyl, unsubstituted or substituted C₁-C₁₀ alkoxy, unsubstituted or substituted C₃-C₈ cycloalkoxy, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, alkylaryl, alkylheteroaryl, an unsubstituted or substituted 7 to 12-membered bicyclic alkyl or heterocyclic ring wherein 1 to 3 ring members are independently nitrogen, oxygen or sulfur, an unsubstituted or substituted 10 to 16-membered tricyclic alkyl or heterocyclic ring wherein 1 to 3 ring members are independently nitrogen, oxygen or sulfur, 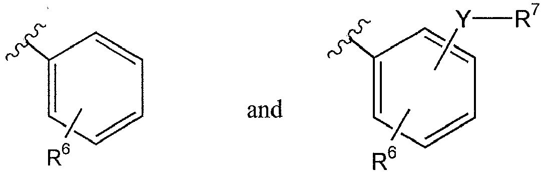
,
R⁶ represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR, -C(O)NRR', - S(O)₂R, -S(O)₂OR, and -S(O)₂NRR';
Y is selected from the group consisting of hydrogen, halogen, NH, O, S, CH₂, CH₂CH₂, C(O), C(O)O, S(O)₂O, or unsubstituted C₁-C₄ alkoxy, wherein when Y is hydrogen, halogen or alkoxy R⁷ is missing;
R⁷ is selected from the group consisting of hydrogen, halogen, -OR, unsubstituted or substituted C₁-C₁₀ alkyl, alkenyl or alkynyl, unsubstituted or substituted C₁-C₁₀ alkoxy, unsubstituted or substituted C₃-C₈ cycloalkoxy, unsubstituted or substituted C₃-C₈ cycloalkyl, unsubstituted or substituted C₆-C₁₄ aryl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic wherein 1 to 3 ring atoms are independently nitrogen, oxygen or sulfur, -C(O)R, -C(O)OR, -C(O)NRR', -NRR', - S(O)₂R, -S(O)₂OR, -S(O)₂NRR', and -P(O)RR';
R¹² represents one to four substituents independently selected from the group consisting of hydrogen, halogen, unsubstituted or substituted C₁-C₄ alkyl, alkenyl or alkynyl, an unsubstituted or substituted 5- to 10-membered heteroaryl wherein 1 to 4 ring atoms are independently selected from nitrogen, oxygen or sulfur, an unsubstituted or substituted 5- to 10-membered heteroalicyclic ring wherein 1 to 3 ring atoms are independently nitrogen, oxygen or sulfur, -OR, -NRR', -NO₂, unsubstituted or substituted C₁-C₄ alkoxy, -C(O)R, -C(O)OR, -C(O)NRR', - S(O)₂R, -S(O)₂OR, and -S(O)₂NRR'; and
R and R' are independently selected from the group consisting of hydrogen and unsubstituted C₁-C₄ alkyl;
or a pharmaceutically acceptable salt thereof for use in the prevention, alleviation and/or treatment of a disease or disorder related to P2Y₁₂ receptor activity,
wherein if the C₁-C₁₀ alkyl is substituted, the substituent group(s) is one or more, for example one or two groups, individually selected from the group consisting of C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰ R¹¹ where R¹⁰ and R¹¹ are independently selected from the group consisting of hydrogen, C₁-C₄ alkyl, C₃-C₈ cycloalkyl, C₆-C₁₄ aryl, carbonyl, acetyl, sulfonyl, amino, and trifluoromethanesulfonyl, or R¹⁰ and R¹¹, together with the nitrogen atom to which they are attached, combine to form a five-or six-membered heteroalicyclic ring,
wherein if the C₃-C₈ cycloalkyl is substituted, the substituent group(s) is one or more, for example one or two groups, individually selected from C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, 0-carboxy, nitro, silyl, sulfinyl, sulfonyl, amino, and NR¹⁰ R¹¹ with R¹⁰ and R¹¹ as defined above,
wherein if the C₆-C₁₄ aryl is substituted, the substituted group(s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and NR¹⁰ R¹¹ with R¹⁰ and R¹¹ as defined above,
wherein if the 5- to 10-membered heteroaryl is substituted, the substituted group(s) is one or more, for example one or two substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰ R¹¹ with R¹⁰ and R¹¹ as defined above, and
wherein if the 5- to 10-membered heteroalicyclic ring is substituted the the substituted group (s) is one or more, for example one, two, or three substituents, independently selected from the group consisting of C₁-C₁₀ alkyl, C₃-C₈ cycloalkyl, C₆-C₁₄ aryl, 5-10 membered heteroaryl wherein 1 to 4 ring atoms are independently selected from nitrogen, oxygen or sulfur, 5-10 membered heteroalicyclic wherein 1 to 3 ring atoms are independently nitrogen, oxygen or sulfur, hydroxy, C₁-C₁₀ alkoxy, C₃-C₈ cycloalkoxy, aryloxy, mercapto, alkylthio, arylthio, cyano, halo, trihalomethyl, carbonyl, thiocarbonyl, O-carbamyl, N-carbamyl, O-thiocarbamyl, N-thiocarbamyl, C-amido, N-amido, C-carboxy, O-carboxy, nitro, silyl, sulfinyl, sulfonyl, amino, and -NR¹⁰ R¹¹ with R¹⁰ and R¹¹ as defined above.

8. The compound for use according to claim 7, wherein is selected from the group consisting of: 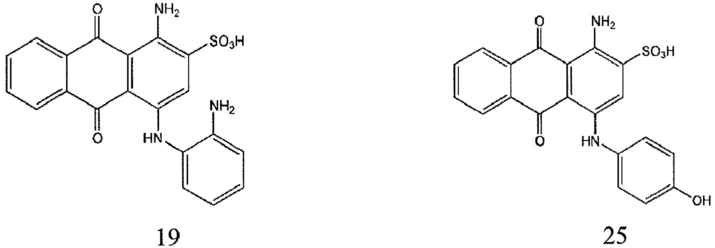 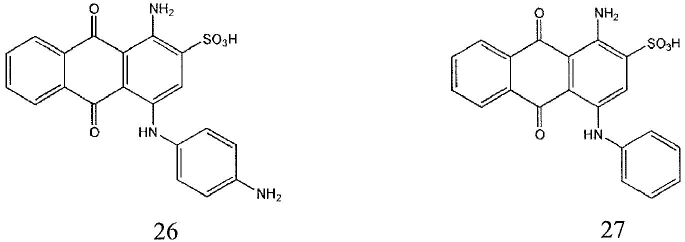 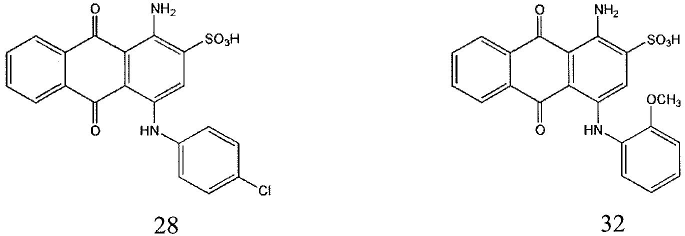 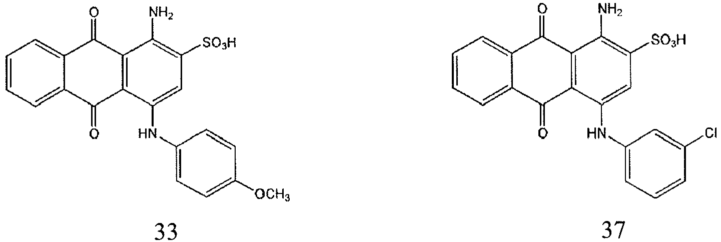 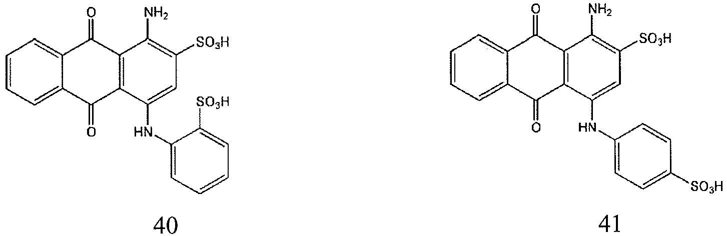 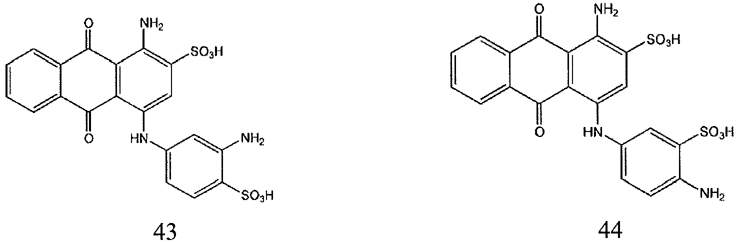 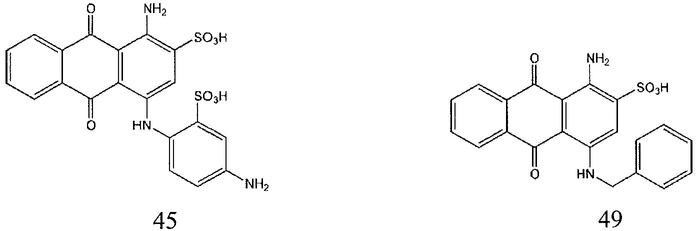 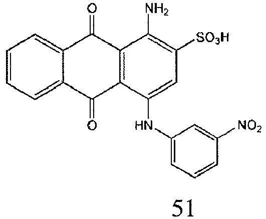 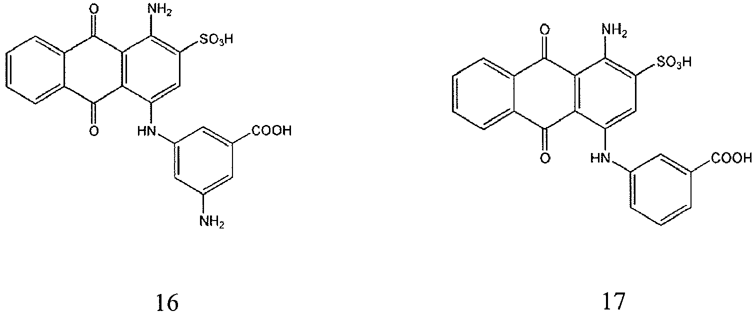 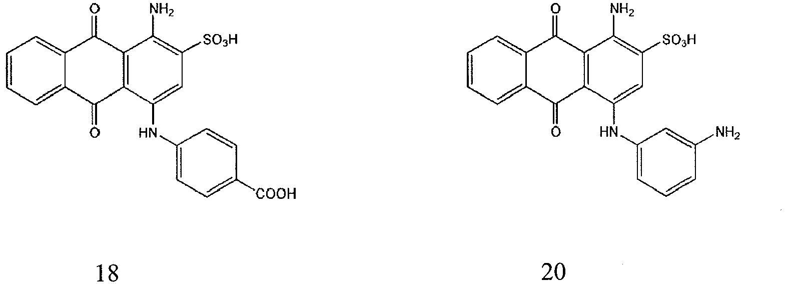 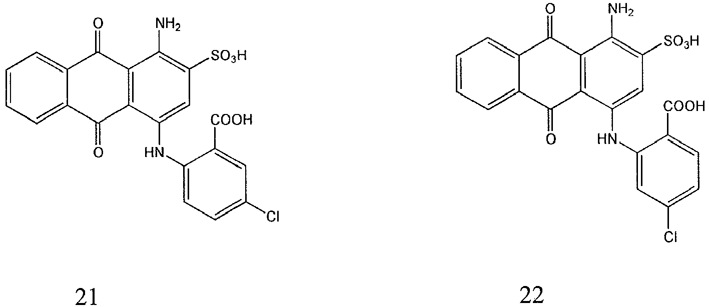 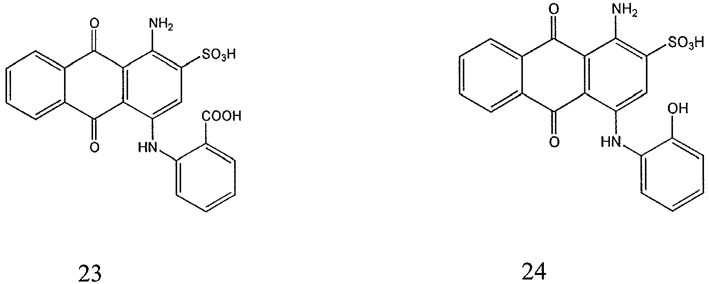 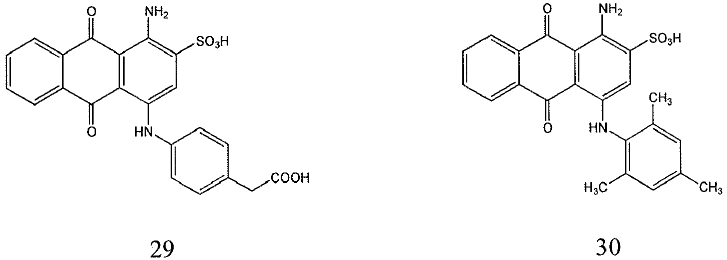 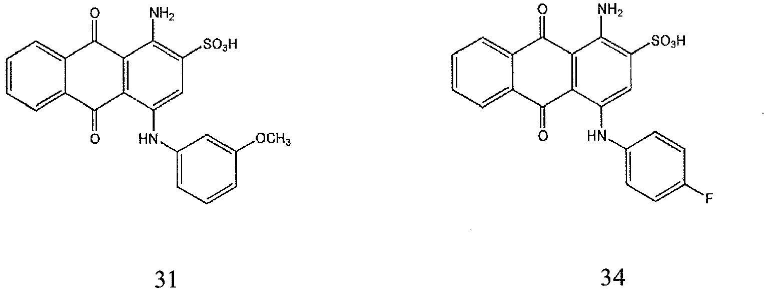 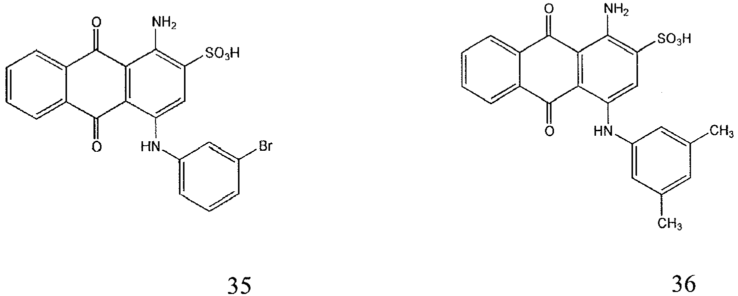 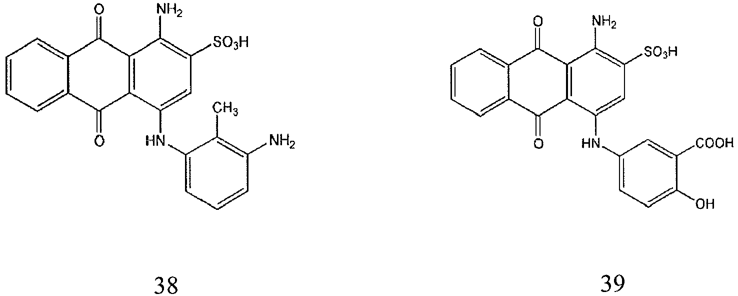 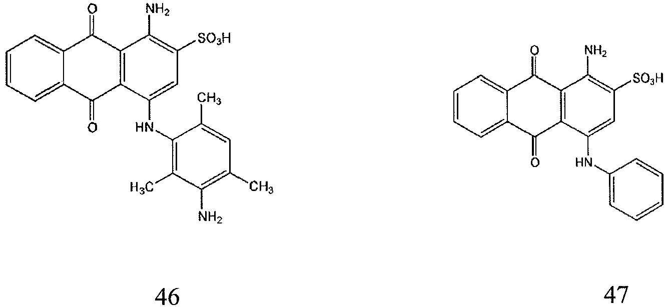 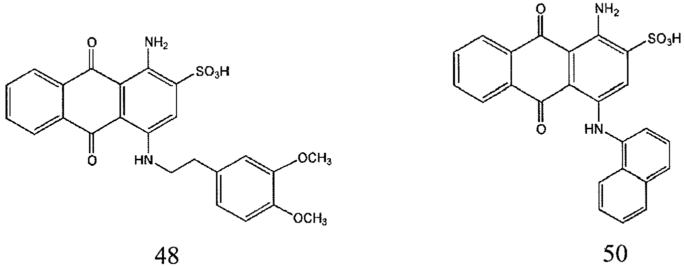 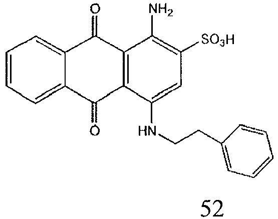 or pharmaceutically acceptable salts thereof.

9. The compound for use according to any one of claims 6 to 8, wherein the disease or disorder is selected from the group of stroke, myocardial infarction, myocardial ischemia, vascular diseases, coronary artery disease, peripheral artery disease, atherosclerosis, cerebrovascular disease, embolisms and CNS disorders.

10. An *in vitro* method for antagonizing the P2Y₁₂ receptor function comprising the step of contacting cells expressing a P2Y₁₂ receptor with at least one compound as defined in any one of claims 1 to 4 and 6 to 8.

## Patentansprüche

1. Eine Verbindung
(A) der Formel I: 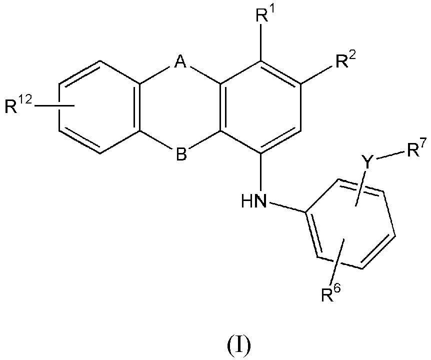 wobei:
A und B unabhängig voneinander ausgewählt sind aus der Gruppe bestehend aus CH₂, O, S, NH, C=O, C=NH, C=S, oder C=N-OH;
R¹ unabhängig ausgewählt ist aus der Gruppe bestehend aus Wasserstoff, unsubstituiertes oder substituiertes C₁-C₁₀ Alkyl, unsubstituiertes oder substituiertes C₁-C₁₀ Alkenyl, unsubstituiertes oder substituiertes C₁-C₁₀ Alkynyl, unsubstituiertes oder substituiertes C₃-C₈ Cycloalkyl, unsubstituiertes oder substituiertes C₁-C₁₀ Alkoxy, unsubstituiertes oder substituiertes C₃-C₈ Cycloalkoxy, unsubstituiertes oder substituiertes C₆-C₁₄ Aryl, ein unsubstituiertes oder substituiertes 5- bis 10-gliedriges Heteroaryl, wobei 1 bis 4 Ringatome unabhähngig ausgewählt sind aus Stickstoff, Sauerstoff oder Schwefel, ein unsubstituierter oder substituierter 5- bis 10-gliedriger heteroalicyclischer Ring, wobei 1 bis 3 Ringatome unabhängig Stickstoff, Sauerstoff oder Schwefel sind, -OR, -C(O)R, -C(O)OR,-C(O)NRR', -NRR', - S(O)₂R, -S(O)₂OR, und -S(O)₂NRR';
R² unabhängig ausgewählt ist aus der Gruppe bestehend aus unsubstituiertes oder substituiertes C₁-C₁₀ Alkyl, unsubstituiertes oder substituiertes C₁-C₁₀ Alkenyl, unsubstituiertes oder substituiertes C₁-C₁₀ Alkynyl, unsubstituiertes oder substituiertes C₃-C₈ Cycloalkyl, unsubstituiertes oder substituiertes C₁-C₁₀ Alkoxy, unsubstituiertes oder substituiertes C₃-C₈ Cycloalkoxy, unsubstituiertes oder substituiertes C₆-C₁₄ Aryl, ein unsubstituiertes oder substituiertes 5- bis 10-gliedriges Heteroaryl, wobei 1 bis 4 Ringatome unabhängig ausgewählt sind aus Stickstoff, Sauerstoff oder Schwefel, ein unsubstituierter oder substituierter 5- bis 10-gliedriger heteroalicyclischer Ring, wobei 1 bis 3 Ringatome unabhängig Stickstoff, Sauerstoff oder Schwefel sind, -OR, -C(O)R, -C(O)OR, -C(O)NRR',-NRR', - S(O)₂R, -S(O)₂OR, und -S(O)₂NRR';
R⁶ für Substituenten steht, die unabhängig ausgewählt sind aus der Gruppe bestehend aus Wasserstoff, Halogen, unsubstituiertes oder substituiertes C₁-C₄ Alkyl, Alkenyl oder Alkynyl, ein unsubstituiertes oder substituiertes 5- bis 10-gliedriges Heteroaryl, wobei 1 bis 4 Ringatome unabhängig ausgewählt sind aus Stickstoff, Sauerstoff oder Schwefel, ein unsubstituierter oder substituierter 5- bis 10-gliedriger heteroalicyclischer Ring, wobei 1 bis 3 Ringatome unabhängig Stickstoff, Sauerstoff oder Schwefel sind, -OR, -NRR', -NO₂, unsubstituiertes oder substituiertes C₁-C₄ Alkoxy, -C(O)R, -C(O)OR, -C(O)NRR', - S(O)₂R, -S(O)₂OR, und -S(O)₂NRR'; wobei in der Meta-Position des Arylrings R⁶ ein Tetrazolyl, eine Sulfonsäure- oder Carbonsäure-Gruppe oder das jeweilige Natrium- oder Kalium-Salz ist;
Y ausgewählt ist aus der Gruppe bestehend aus NH, O, S, CH₂, CH₂CH₂, C(O), C(O)O, oder S(O)₂O;
R⁷ unsubstituiertes C₆-C₁₄ Aryl ist;
R¹² für ein bis vier Substituenten steht, die unabhängig ausgewählt sind aus der Gruppe bestehend aus Wasserstoff, Halogen, unsubstituiertes oder substituiertes C₁-C₄ Alkyl, Alkenyl oder Alkynyl, ein unsubstituiertes oder substituiertes 5- bis 10-gliedriges Heteroaryl, wobei 1 bis 4 Ringatome unabhängig ausgewählt sind aus Stickstoff, Sauerstoff oder Schwefel, ein unsubstituierter oder substituierter 5- bis 10-gliedriger heteroalicyclischer Ring, wobei 1 bis 3 Ringatome unabhängig Stickstoff, Sauerstoff oder Schwefel sind, -OR, -NRR', -NO₂, unsubstituiertes oder substituiertes C₁-C₄ Alkoxy, -C(O)R, -C(O)OR, -C(O)NRR', - S(O)₂R, -S(O)₂OR, und -S(O)₂NRR'; und
R und R' unabhängig ausgewählt sind aus der Gruppe bestehend aus Wasserstoff und unsubstituiertes C₁-C₄ Alkyl;
oder ein pharmazeutisch annehmbares Salz davon, oder
wenn das C₁-C₁₀ Alkyl substituiert ist, die Substituentengruppe(n) eins oder mehr ist, zum Beispiel eine oder zwei Gruppen, individuell ausgewählt aus der Gruppe bestehend aus C₃-C₈ Cycloalkyl, C₆-C₁₄ Aryl, 5-10 gliedriges Heteroaryl, wobei 1 bis 4 Ringatome unabhängig ausgewählt sind aus Stickstoff, Sauerstoff oder Schwefel, 5-10 gliedriger heteroalicyclischer Ring, wobei 1 bis 3 Ringatome unabhängig Stickstoff, Sauerstoff oder Schwefel sind, Hydroxy, C₁-C₁₀ Alkoxy, C₃-C₈ Cycloalkoxy, Aryloxy, Mercapto, Alkylthio, Arylthio, Cyano, Halo, Carbonyl, Thiocarbonyl, O-carbamyl, N-carbamyl, O-thiocarbamyl, N-thiocarbamyl, C-amido, N-amido, C-carboxy, O-carboxy, Nitro, Silyl, Sulfinyl, Sulfonyl, Amino, und -NR¹⁰ R¹¹ wobei R¹⁰ und R¹¹ unabhängig ausgewählt sind aus der Gruppe bestehend aus Wasserstoff, C₁-C₄ Alkyl, C₃-C₈ Cycloalkyl, C₆-C₁₄ Aryl, Carbonyl, Acetyl, Sulfonyl, Amino, und Trifluoromethanesulfonyl, oder R¹⁰ und R¹¹, zusammen mit dem Stickstoffatom, an das sie gebunden sind, sich vereinen, um einen fünf- oder sechs-gliedrigen heteroalicyclischen Ring zu formen,
wobei wenn das C₃-C₈ Cycloalkyl substituiert ist, die Substituentengruppe(n) eins oder mehr ist, zum Beispiel eine oder zwei Gruppen, individuell ausgewählt aus der Gruppe bestehend aus C₁-C₁₀ Alkyl, C₃-C₈ Cycloalkyl, C₆-C₁₄ Aryl, 5-10 gliedrigen Heteroaryl, wobei 1 bis 4 Ringatome unabhängig ausgewählt sind aus Stickstoff, Sauerstoff oder Schwefel, 5-10 gliedrige heteroalicyclische Ringe, wobei 1 bis 3 Ringatome unabhängig Stickstoff, Sauerstoff oder Schwefel sind, Hydroxy, C₁-C₁₀ Alkoxy, C₃-C₈ Cycloalkoxy, Aryloxy, Mercapto, Alkylthio, Arylthio, Cyano, Halo, Carbonyl, Thiocarbonyl, O-carbamyl, N-carbamyl, O-thiocarbamyl, N-thiocarbamyl, C-amido, N-amido, C-carboxy, O-carboxy, Nitro, Silyl, Sulfinyl, Sulfonyl, Amino, und NR¹⁰ R¹¹ mit R¹⁰ und R¹¹ wie oben definiert,
wobei wenn das C₆-C₁₄ Aryl substituiert ist, die Substituentengruppe(n) eins oder mehr ist, zum Beispiel eine, zwei oder drei Gruppen, individuell ausgewählt aus der Gruppe bestehend aus C₁-C₁₀ Alkyl, C₃-C₈ Cycloalkyl, C₆-C₁₄ Aryl, 5-10 gliedriges Heteroaryl, wobei 1 bis 4 Ringatome unabhängig ausgewählt sind aus Stickstoff, Sauerstoff oder Schwefel, 5-10 gliedrige heteroalicyclische Ringe, wobei 1 bis 3 Ringatome unabhängig Stickstoff, Sauerstoff oder Schwefel sind, Hydroxy, C₁-C₁₀ Alkoxy, C₃-C₈ Cycloalkoxy, Aryloxy, Mercapto, Alkylthio, Arylthio, Cyano, Halo, Trihalomethyl, Carbonyl, Thiocarbonyl, O-carbamyl, N-carbamyl, O-thiocarbamyl, N-thiocarbamyl, C-amido, N-amido, C-carboxy, O-carboxy, Nitro, Silyl, Sulfinyl, Sulfonyl, Amino, und NR¹⁰ R¹¹ mit R¹⁰ und R¹¹ wie oben definiert,
wobei wenn das 5- bis 10-gliedrige Heteroaryl substituiert ist, die Substituentengruppe(n) eins oder mehr ist, zum Beispiel eine oder zwei Gruppen, individuell ausgewählt aus der Gruppe bestehend aus C₁-C₁₀ Alkyl, C₃-C₈ Cycloalkyl, C₆-C₁₄ Aryl, 5-10 gliedriges Heteroaryl, wobei 1 bis 4 Ringatome unabhängig ausgewählt sind aus Stickstoff, Sauerstoff oder Schwefel, 5-10 gliedriger heteroalicyclischer Ring, wobei 1 bis 3 Ringatome unabhängig Stickstoff, Sauerstoff oder Schwefel sind, Hydroxy, C₁-C₁₀ Alkoxy, C₃-C₈ Cycloalkoxy, Aryloxy, Mercapto, Alkylthio, Arylthio, Cyano, Halo, Trihalomethyl, Carbonyl, Thiocarbonyl, O-carbamyl, N-carbamyl, O-thiocarbamyl, N-thiocarbamyl, C-amido, N-amido, C-carboxy, O-carboxy, Nitro, Silyl, Sulfinyl, Sulfonyl, Amino, und -NR¹⁰ R¹¹ mit R¹⁰ und R¹¹ wie oben definiert, und
wobei wenn der 5- bis 10-gliedrige heteroalicyclische Ring substituiert ist, die Substituentengruppe(n) eins oder mehr ist, zum Beispiel eine, zwei oder drei Gruppen, individuell ausgewählt aus der Gruppe bestehend aus C₁-C₁₀ Alkyl, C₃-C₈ Cycloalkyl, C₆-C₁₄ Aryl, 5-10 gliedriges Heteroaryl, wobei 1 bis 4 Ringatome unabhängig ausgewählt sind aus Stickstoff, Sauerstoff oder Schwefel, 5-10 gliedriger heteroalicyclischer Ring, wobei 1 bis 3 Ringatome unabhängig Stickstoff, Sauerstoff oder Schwefel sind, Hydroxy, C₁-C₁₀ Alkoxy, C₃-C₈ Cycloalkoxy, Aryloxy, Mercapto, Alkylthio, Arylthio, Cyano, Halo, Trihalomethyl, Carbonyl, Thiocarbonyl, O-carbamyl, N-carbamyl, O-thiocarbamyl, N-thiocarbamyl, C-amido, N-amido, C-carboxy, O-carboxy, Nitro, Silyl, Sulfinyl, Sulfonyl, Amino, und -NR¹⁰ R¹¹ mit R¹⁰ und R¹¹ wie oben definiert;
(B) ausgewählt aus der Gruppe bestehend aus: 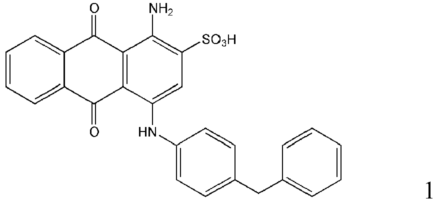 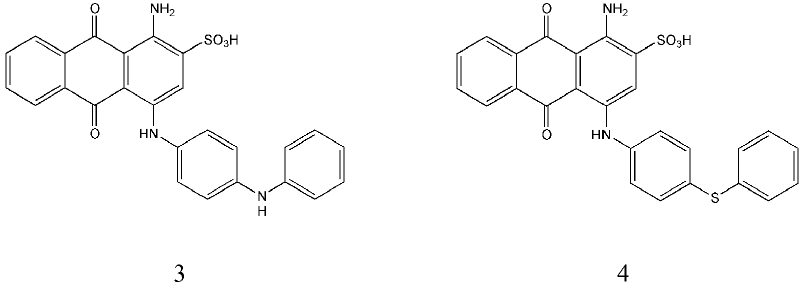 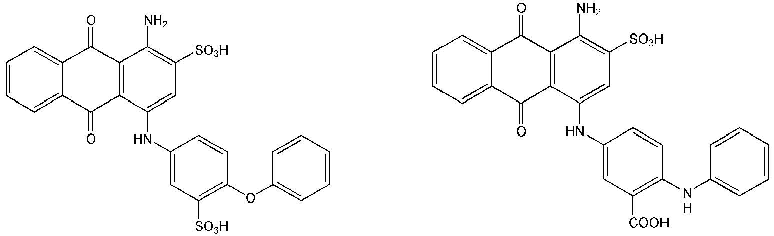 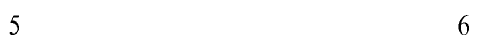 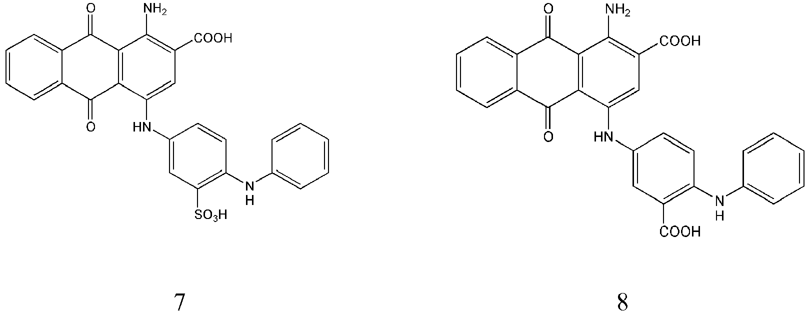 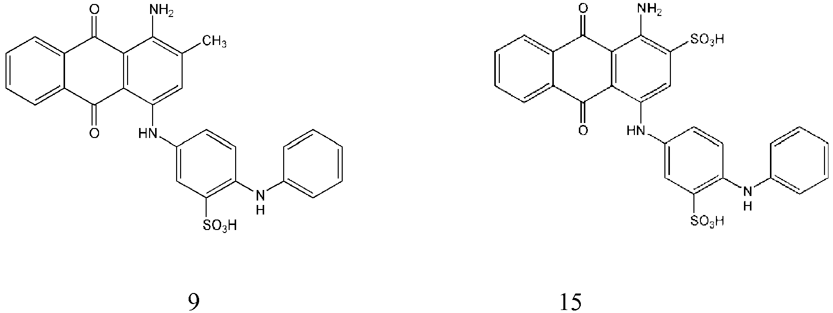 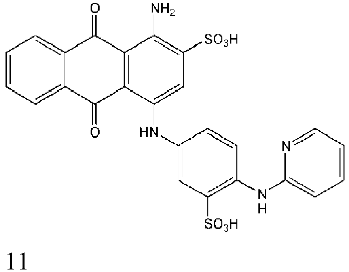 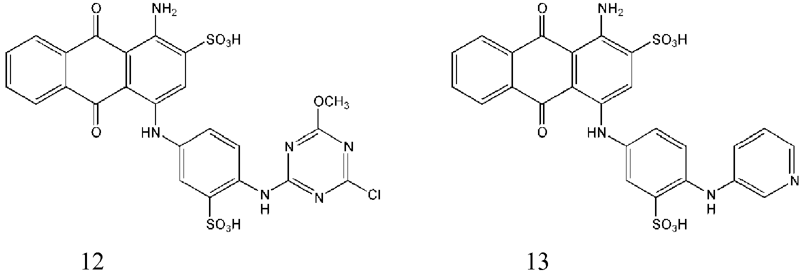 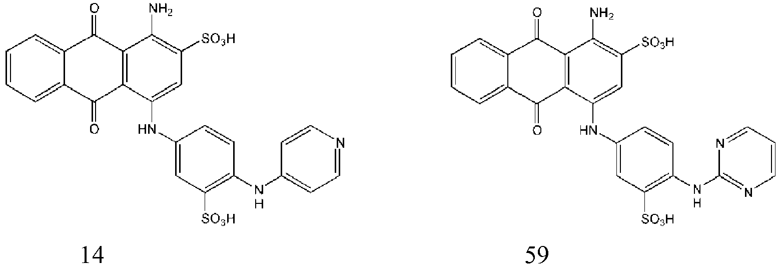 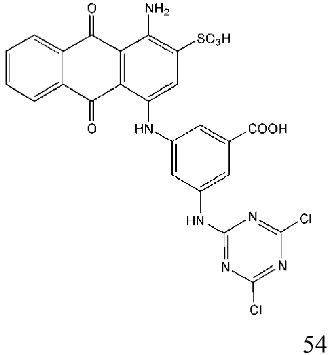 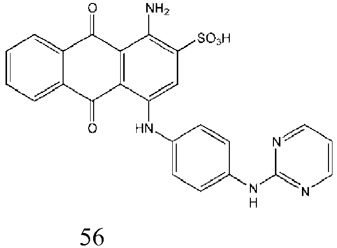 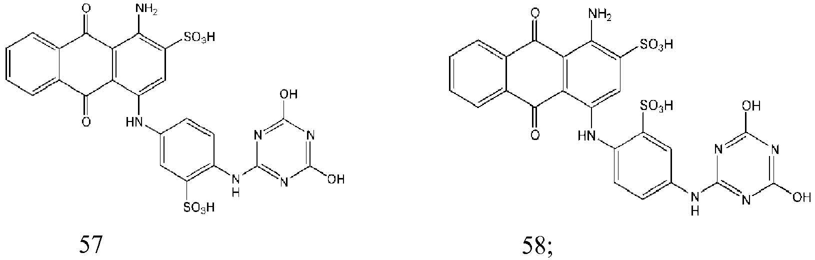 oder ein pharmazeutsich annehmbaren Salzen davon; oder
(C) ausgewählt ist aus der Gruppe bestehend aus: 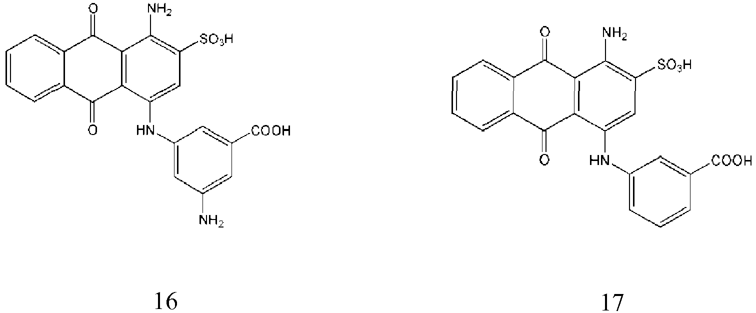 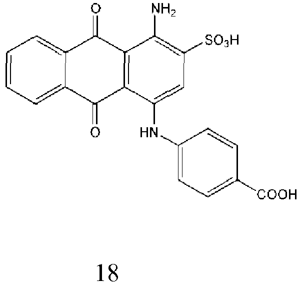 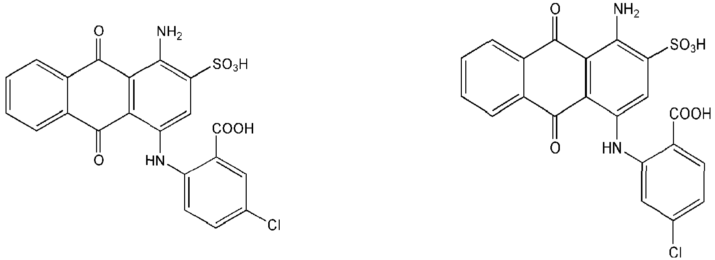 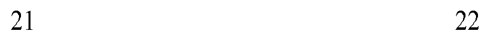 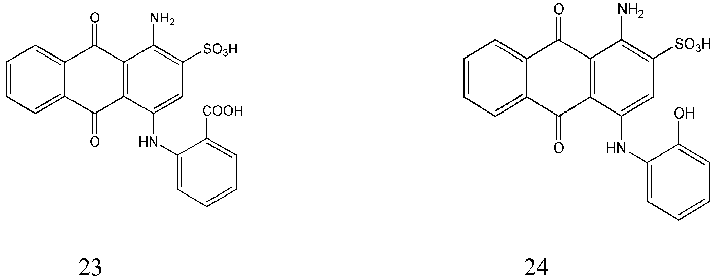 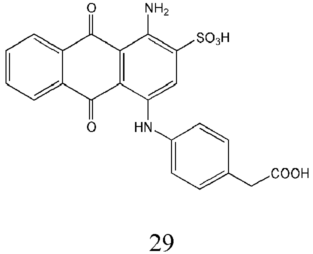 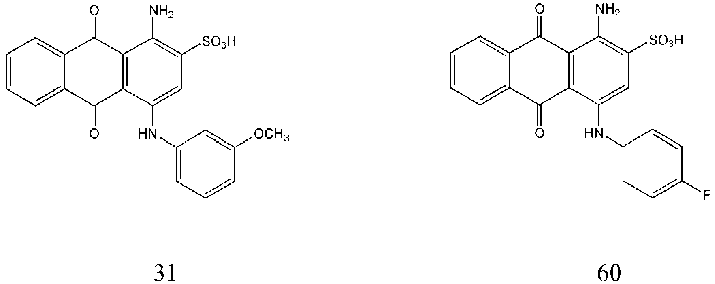 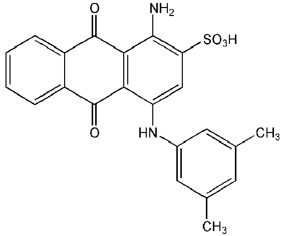 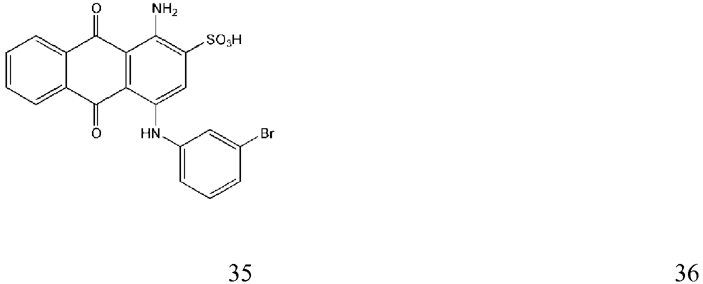 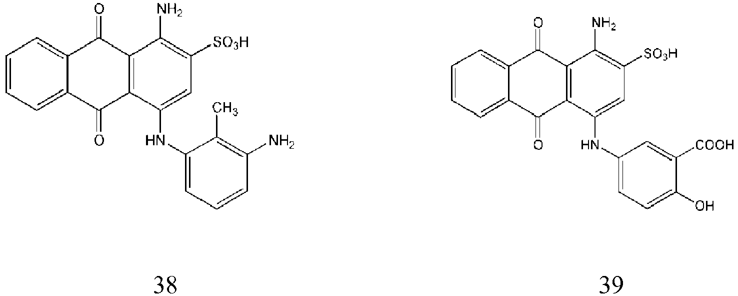 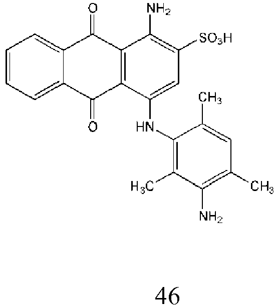 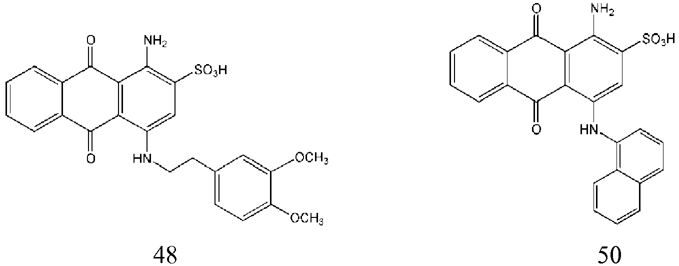 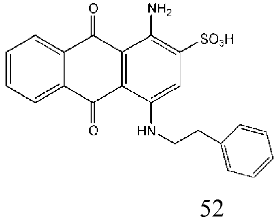 oder ein pharmazeutisch annehmbares Salz davon.

2. Die Verbindung nach Anspruch 1, wobei R⁷ unsubstituiertes C₆ Aryl ist.

3. Die Verbindung nach Anspruch 1 oder 2, wobei Y NH ist und/oder wobei R⁶ für Substituenten steht, die ausgewählt sind aus der Gruppe bestehend aus Wasserstoff, -S(O)₂OR, -C(O)OR, Tetrazolyl, Sulfonyl, Carboxy, Amino, Halogen, C₁-C₄ Alkoxy, C₁-C₄ Alkyl, Hydroxy, und Nitro.

4. Die Verbindung nach einem der Ansprüche 1-3, wobei
(1) R¹² für einen bis vier Substituenten steht, die ausgewählt sind aus der Gruppe bestehend aus Wasserstoff, Halo, Amino, unsubstituiertes C₁-C₄ Alkyl oder Alkoxy, oder Hydroxy; und/oder
(2) mindestens eines von A und B C=O ist oder beide A und B C=O sind; und/oder
(3) R¹ Amino ist; und/oder
(4) R² Tetrazolyl, -S(O)₂OR oder -C(O)OR ist.

5. Eine pharmazeutische Zusammensetzung, umfassend eine Verbindung oder ein Salz nach einem der Ansprüche 1 bis 4 und einen pharmazeutisch annehmbaren Träger oder Hilfsstoff und gegebenenfalls ferner ein zusätzliches Medikament oder Arzneimittel.

6. Eine Verbindung nach einem der Ansprüche 1 (A) oder (B) bis 4 oder ein pharmazeutisch annehmbares Salz davon zur Verwendung in einem Verfahren zur Hemmung der Thrombozytenaggregation oder zur Verwendung bei der Prävention, Linderung und/oder Behandlung einer Erkrankung oder Störung im Zusammenhang mit P2Y12-Rezeptor-Aktivität.

7. Eine Verbindung der Formel III: 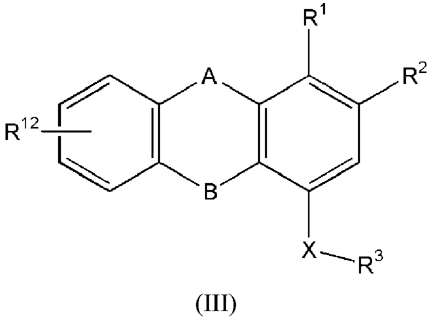 wobei:
A und B C=O sind;
X NH ist;
R¹ -NH₂ ist;
R² aus der Gruppe aus -C(O)OH und -S(O)₂OH ausgewählt ist;
R³ ausgewählt ist aus der Gruppe bestehend aus unsubstituiertes oder substituiertes C₁-C₁₀ Alkyl, unsubstituiertes oder substituiertes C₁-C₁₀ Alkenyl, unsubstituiertes oder substituiertes C₁-C₁₀ Alkynyl, unsubstituiertes oder substituiertes C₃-C₈ Cycloalkyl, unsubstituiertes oder substituiertes C₁-C₁₀ Alkoxy, unsubstituiertes oder substituiertes C₃-C₈ Cycloalkoxy, unsubstituiertes oder substituiertes C₆-C₁₄ Aryl, ein unsubstituiertes oder substituiertes 5- bis 10-gliedriges Heteroaryl, wobei 1 bis 4 Ringatom unabhänging ausgewählt sind aus Stickstoff, Sauerstoff oder Schwefel, ein unsubstituierter oder substituierter 5- bis 10-gliedriger heteroalicyclischer Ring, wobei 1 bis 3 Ringatome unabhängig Stickstoff, Sauerstoff oder Schwefel sind, Alkylaryl, Alkylheteroaryl, ein unsubstituiertes oder substituiertes 7 bis 12-gliedriges bicyclisches Alkyl oder heterocyclischer Ring, wobei 1 bis 3 Ringatome unabhängig Stickstoff, Sauerstoff oder Schwefel sind, ein unsubstituiertes oder substituiertes 10 bis 16-gliedriges tricyclisches Alkyl oder heterocyclischer Ring, wobei 1 bis 3 Ringatome unabhängig Stickstoff, Sauerstoff oder Schwefel sind, 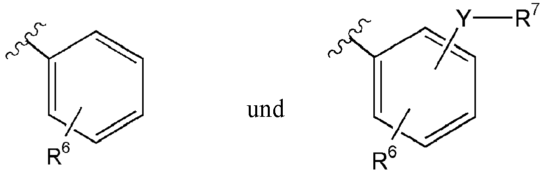 ;
R⁶ für einen bis vier Substituenten steht, die unabhängig ausgewählt sind aus der Gruppe bestehend aus Wasserstoff, Halogen, unsubstituiertes oder substituiertes C₁-C₄ Alkyl, Alkenyl oder Alkynyl, ein unsubstituiertes oder substituiertes 5- bis 10-gliedriges Heteroaryl, wobei 1 bis 4 Ringatome unabhängig ausgewählt sind aus Stickstoff, Sauerstoff oder Schwefel, ein unsubstituierter oder substituierter 5- bis 10-gliedriger heteroalicyclischer Ring, wobei 1 bis 3 Ringatome unabhängig Stickstoff, Sauerstoff oder Schwefel sind, -OR, -NRR', -NO₂, unsubstituiertes oder substituiertes C₁-C₄ Alkoxy, -C(O)R, -C(O)OR, -C(O)NRR',-S(O)₂R, -S(O)₂OR, und -S(O)₂NRR';
Y ausgewählt ist aus der Gruppe bestehend aus Wasserstoff, Halogen, NH, O, S, CH₂, CH₂CH₂, C(O), C(O)O, S(O)₂O, oder unsubstituiertes C₁-C₄ Alkoxy, wobei wenn Y Wasserstoff, Halogen oder Alkoxy ist R⁷ fehlt;
R⁷ ausgewählt ist aus der Gruppe bestehend aus Wasserstoff, Halogen, -OR, unsubstituiertes oder substituiertes C₁-C₁₀ Alkyl, Alkenyl oder Alkynyl, unsubstituiertes oder substituiertes C₁-C₁₀ Alkoxy, unsubstituiertes oder substituiertes C₃-C₈ Cycloalkoxy, unsubstituiertes oder substituiertes C₃-C₈ Cycloalkyl, unsubstituiertes oder substituiertes C₆-C₁₄ Aryl, ein unsubstituiertes oder substituiertes 5- bis 10-gliedriges Heteroaryl, wobei 1 bis 4 Ringatome unabhängig ausgewählt sind aus Stickstoff, Sauerstoff oder Schwefel, ein unsubstituierter oder substituierter 5- bis 10-gliedriger heteroalicyclischer Ring, wobei 1 bis 3 Ringatome unabhängig Stickstoff, Sauerstoff oder Schwefel sind, -C(O)R, -C(O)OR,-C(O)NRR', -NRR', - S(O)₂R, -S(O)₂OR, -S(O)₂NRR', und -P(O)RR';
R¹² für ein bis vier Substituenten steht, die unabhängig ausgewählt sind aus der Gruppe bestehend aus Wasserstoff, Halogen, unsubstituiertes oder substituiertes C₁-C₄ Alkyl, Alkenyl oder Alkynyl, ein unsubstituiertes oder substituiertes 5- bis 10-gliedriges Heteroaryl, wobei 1 bis 4 Ringatome unabhängig ausgewählt sind aus Stickstoff, Sauerstoff oder Schwefel, ein unsubstituierter oder substituierter 5- bis 10-gliedriger heteroalicyclischer Ring, wobei 1 bis 3 Ringatome unabhängig Stickstoff, Sauerstoff oder Schwefel sind, -OR, -NRR', -NO₂, unsubstituiertes oder substituiertes C₁-C₄ Alkoxy, -C(O)R, -C(O)OR, -C(O)NRR', - S(O)₂R, -S(O)₂OR, und -S(O)₂NRR'; und
R und R' unabhängig ausgewählt sind aus der Gruppe bestehend aus Wasserstoff und unsubstituiertes C₁-C₄ Alkyl;
oder einem pharmazeutisch annehmbaren Salz davon zur Verwendung in der Prävention, Linderung und/oder Behandlung einer Erkrankung oder Störung im Zusammenhang mit P2Y 12-Rezeptor-Aktivität,
wenn das C₁-C₁₀ Alkyl substituiert ist, die Substituentengruppe(n) eins oder mehr ist, zum Beispiel eine oder zwei Gruppen, individuell ausgewählt aus der Gruppe bestehend aus C₃-C₈ Cycloalkyl, C₆-C₁₄ Aryl, 5-10 gliedriges Heteroaryl, wobei 1 bis 4 Ringatome unabhängig ausgewählt sind aus Stickstoff, Sauerstoff oder Schwefel, 5-10 gliedriger heteroalicyclischer Ring, wobei 1 bis 3 Ringatome unabhängig Stickstoff, Sauerstoff oder Schwefel sind, Hydroxy, C₁-C₁₀ Alkoxy, C₃-C₈ Cycloalkoxy, Aryloxy, Mercapto, Alkylthio, Arylthio, Cyano, Halo, Carbonyl, Thiocarbonyl, O-carbamyl, N-carbamyl, O-thiocarbamyl, N-thiocarbamyl, C-amido, N-amido, C-carboxy, O-carboxy, Nitro, Silyl, Sulfinyl, Sulfonyl, Amino, und -NR¹⁰ R¹¹ wobei R¹⁰ und R¹¹ unabhängig ausgewählt sind aus der Gruppe bestehend aus Wasserstoff, C₁-C₄ Alkyl, C₃-C₈ Cycloalkyl, C₆-C₁₄ Aryl, Carbonyl, Acetyl, Sulfonyl, Amino, und Trifluoromethanesulfonyl, oder R¹⁰ und R¹¹, zusammen mit dem Stickstoffatom, an das sie gebunden sind, sich vereinen, um einen fünf- oder sechs-gliedrigen heteroalicyclischen Ring zu formen,
wobei wenn das C₃-C₈ Cycloalkyl substituiert ist, die Substituentengruppe(n) eins oder mehr ist, zum Beispiel eine oder zwei Gruppen, individuell ausgewählt aus der Gruppe bestehend aus C₁-C₁₀ Alkyl, C₃-C₈ Cycloalkyl, C₆-C₁₄ Aryl, 5-10 gliedrigen Heteroaryl, wobei 1 bis 4 Ringatome unabhängig ausgewählt sind aus Stickstoff, Sauerstoff oder Schwefel, 5-10 gliedrige heteroalicyclische Ringe, wobei 1 bis 3 Ringatome unabhängig Stickstoff, Sauerstoff oder Schwefel sind, Hydroxy, C₁-C₁₀ Alkoxy, C₃-C₈ Cycloalkoxy, Aryloxy, Mercapto, Alkylthio, Arylthio, Cyano, Halo, Carbonyl, Thiocarbonyl, O-carbamyl, N-carbamyl, O-thiocarbamyl, N-thiocarbamyl, C-amido, N-amido, C-carboxy, O-carboxy, Nitro, Silyl, Sulfinyl, Sulfonyl, Amino, und NR¹⁰ R¹¹ mit R¹⁰ und R¹¹ wie oben definiert,
wobei wenn das C₆-C₁₄ Aryl substituiert ist, die Substituentengruppe(n) eins oder mehr ist, zum Beispiel eine, zwei oder drei Gruppen, individuell ausgewählt aus der Gruppe bestehend aus C₁-C₁₀ Alkyl, C₃-C₈ Cycloalkyl, C₆-C₁₄ Aryl, 5-10 gliedriges Heteroaryl, wobei 1 bis 4 Ringatome unabhängig ausgewählt sind aus Stickstoff, Sauerstoff oder Schwefel, 5-10 gliedrige heteroalicyclische Ringe, wobei 1 bis 3 Ringatome unabhängig Stickstoff, Sauerstoff oder Schwefel sind, Hydroxy, C₁-C₁₀ Alkoxy, C₃-C₈ Cycloalkoxy, Aryloxy, Mercapto, Alkylthio, Arylthio, Cyano, Halo, Trihalomethyl, Carbonyl, Thiocarbonyl, O-carbamyl, N-carbamyl, O-thiocarbamyl, N-thiocarbamyl, C-amido, N-amido, C-carboxy, O-carboxy, Nitro, Silyl, Sulfinyl, Sulfonyl, Amino, und NR¹⁰ R¹¹ mit R¹⁰ und R¹¹ wie oben definiert,
wobei wenn das 5- bis 10-gliedrige Heteroaryl substituiert ist, die Substituentengruppe(n) eins oder mehr ist, zum Beispiel eine oder zwei Gruppen, individuell ausgewählt aus der Gruppe bestehend aus C₁-C₁₀ Alkyl, C₃-C₈ Cycloalkyl, C₆-C₁₄ Aryl, 5-10 gliedriges Heteroaryl, wobei 1 bis 4 Ringatome unabhängig ausgewählt sind aus Stickstoff, Sauerstoff oder Schwefel, 5-10 gliedriger heteroalicyclischer Ring, wobei 1 bis 3 Ringatome unabhängig Stickstoff, Sauerstoff oder Schwefel sind, Hydroxy, C₁-C₁₀ Alkoxy, C₃-C₈ Cycloalkoxy, Aryloxy, Mercapto, Alkylthio, Arylthio, Cyano, Halo, Trihalomethyl, Carbonyl, Thiocarbonyl, O-carbamyl, N-carbamyl, O-thiocarbamyl, N-thiocarbamyl, C-amido, N-amido, C-carboxy, O-carboxy, Nitro, Silyl, Sulfinyl, Sulfonyl, Amino, und -NR¹⁰ R¹¹ mit R¹⁰ und R¹¹ wie oben definiert, und
wobei wenn der 5- bis 10-gliedrige heteroalicyclische Ring substituiert ist, die Substituentengruppe(n) eins oder mehr ist, zum Beispiel eine, zwei oder drei Gruppen, individuell ausgewählt aus der Gruppe bestehend aus C₁-C₁₀ Alkyl, C₃-C₈ Cycloalkyl, C₆-C₁₄ Aryl, 5-10 gliedriges Heteroaryl, wobei 1 bis 4 Ringatome unabhängig ausgewählt sind aus Stickstoff, Sauerstoff oder Schwefel, 5-10 gliedriger heteroalicyclischer Ring, wobei 1 bis 3 Ringatome unabhängig Stickstoff, Sauerstoff oder Schwefel sind, Hydroxy, C₁-C₁₀ Alkoxy, C₃-C₈ Cycloalkoxy, Aryloxy, Mercapto, Alkylthio, Arylthio, Cyano, Halo, Trihalomethyl, Carbonyl, Thiocarbonyl, O-carbamyl, N-carbamyl, O-thiocarbamyl, N-thiocarbamyl, C-amido, N-amido, C-carboxy, O-carboxy, Nitro, Silyl, Sulfinyl, Sulfonyl, Amino, und -NR¹⁰ R¹¹ mit R¹⁰ und R¹¹ wie oben definiert.

8. Die Verbindung zur Verwendung nach Anspruch 7, wobei die Verbindung ausgewählt ist aus der Gruppe bestehend aus: 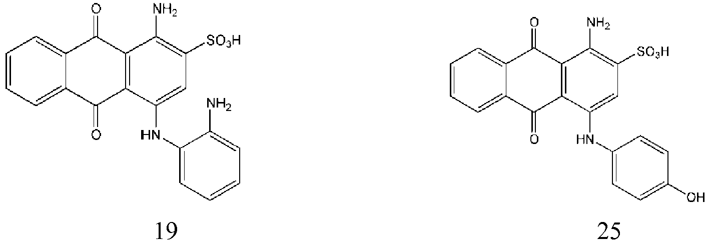 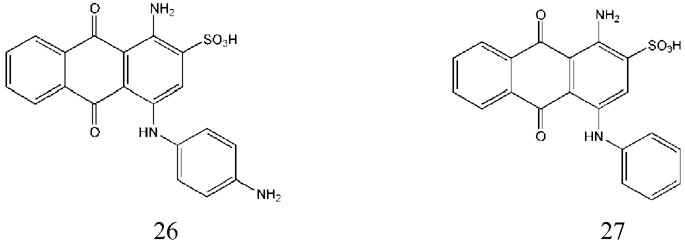 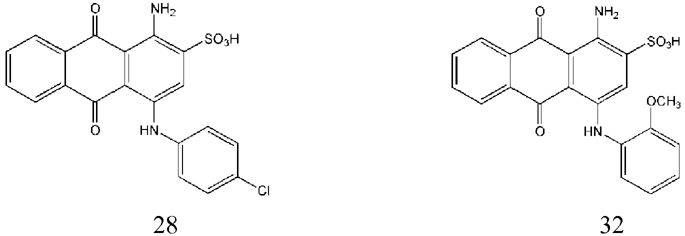 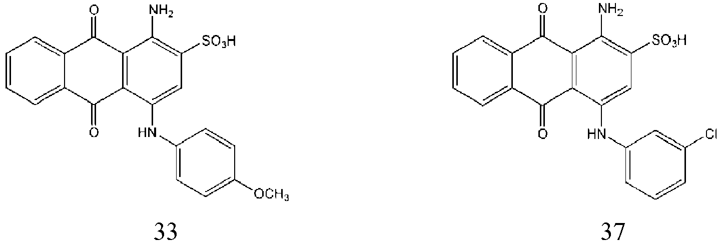 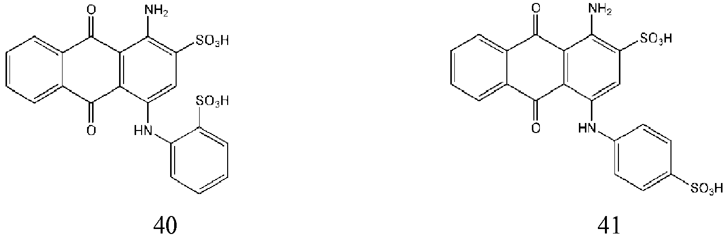 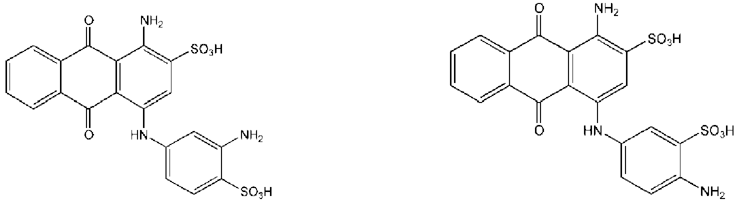 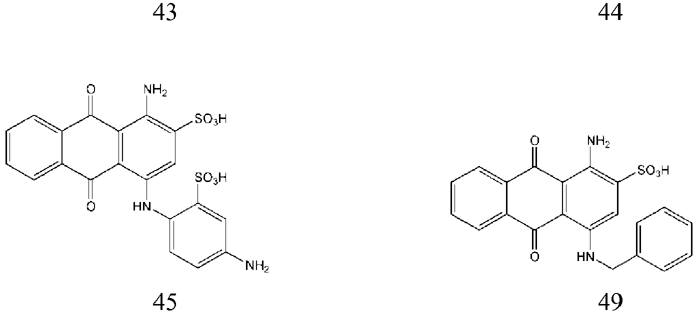 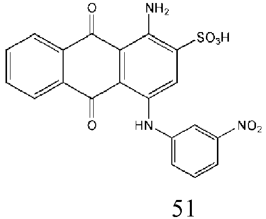 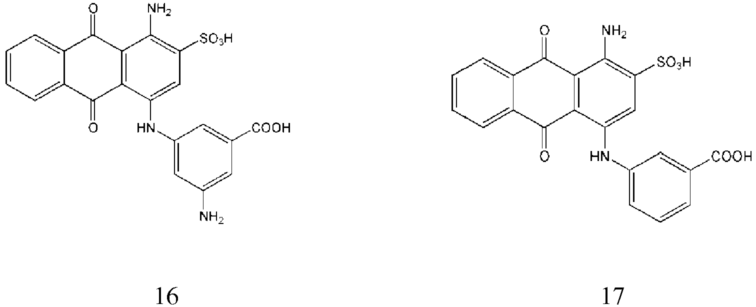 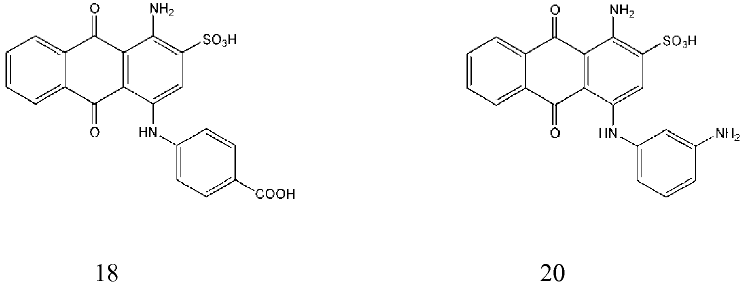 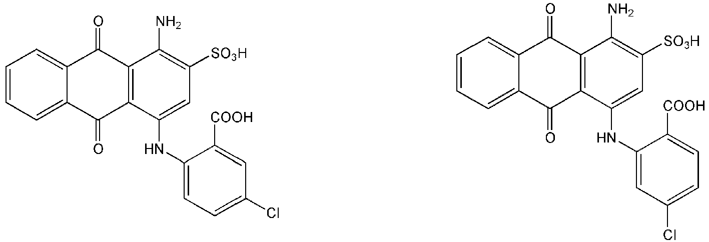 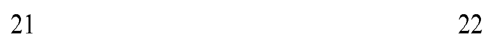 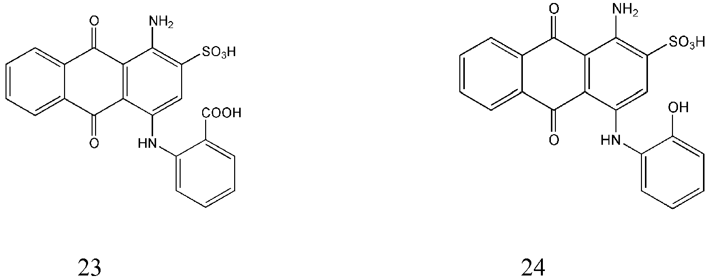 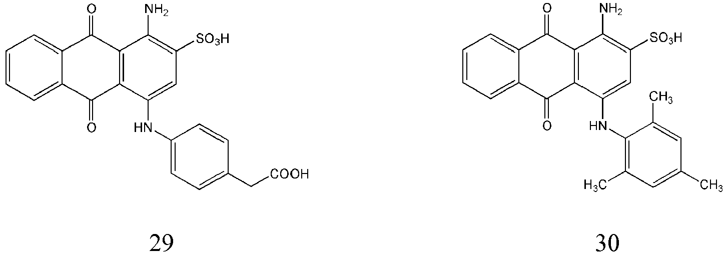 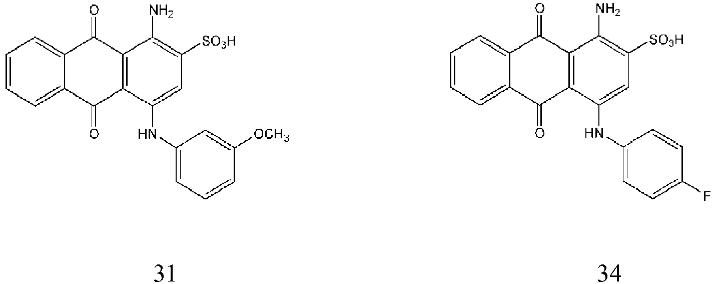 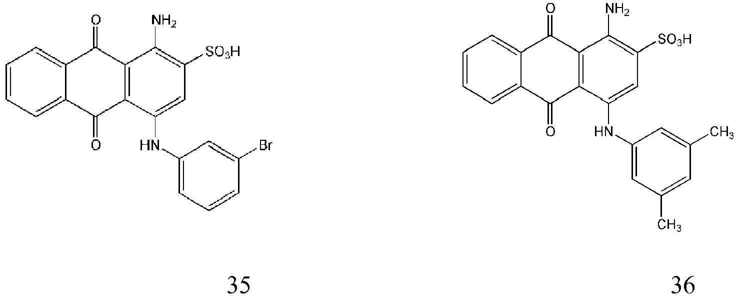 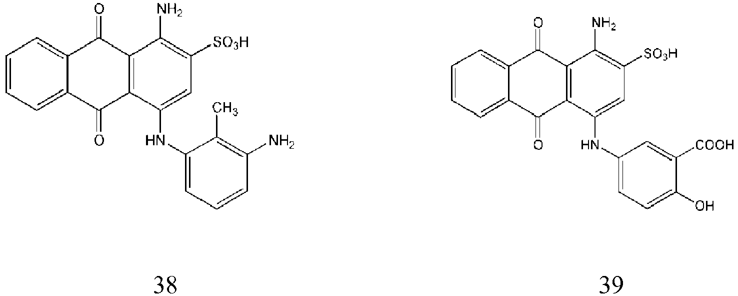 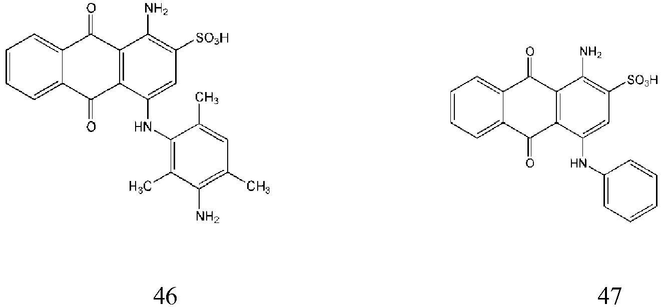 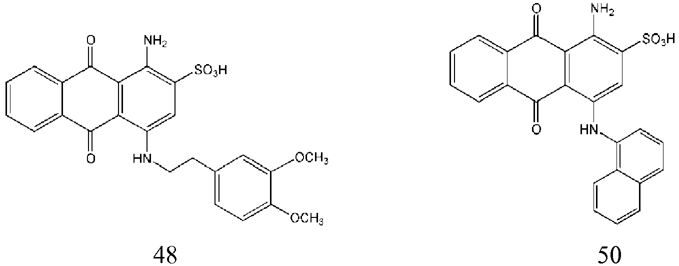 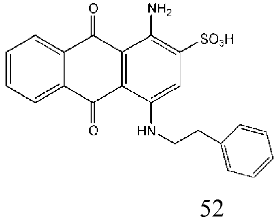 oder ein pharmazeutisch annehmbares Salz davon.

9. Die Verbindung zur Verwendung nach einem der Ansprüche 6 bis 8, wobei die Erkrankung oder Störung aus der Gruppe des Schlaganfalls, des Myokardinfarkts, der Myokardischämie, der Gefäßerkrankungen, der koronaren Herzkrankheit, der peripheren Arterienerkrankung, der Atherosklerose, der zerebrovaskulären Erkrankung, Embolien und ZNS-Störungen ausgewählt ist.

10. Ein *in vitro* Verfahren zur Antagonisierung der P2Y12-Rezeptorfunktion, umfassend den Schritt des in-Kontakt-bringens von Zellen, die einen P2Y12-Rezeptor exprimieren, mit mindestens einer Verbindung nach einem der Ansprüche 1 bis 4 und 6 bis 8.

## Revendications

1. Composé
(A) de formule I : 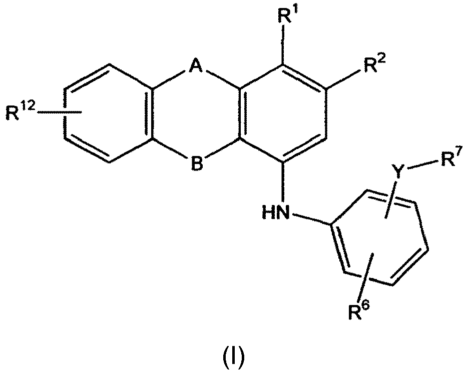 dans laquelle :
A et B sont indépendamment choisis dans l'ensemble constitué par CH₂, O, S, NH, C=O, C=NH, C=S et C=N-OH ;
R¹ est indépendamment choisi dans l'ensemble constitué par l'hydrogène, alkyle en C₁ à C₁₀ substitué ou non substitué, alcényle en C₁ à C₁₀ substitué ou non substitué, alcynyle en C₁ à C₁₀ substitué ou non substitué, cycloalkyle en C₃ à C₈ substitué ou non substitué, alcoxy en C₁ à C₁₀ substitué ou non substitué, cycloalcoxy en C₃ à C₈ substitué ou non substitué, aryle en C₆ à C₁₄ substitué ou non substitué, hétéroaryle à 5 à 10 chaînons substitué ou non substitué dont 1 à 4 atomes de cycle sont indépendamment choisis parmi l'azote, l'oxygène et le soufre, un cycle hétéroalicyclique à 5 à 10 chaînons substitué ou non substitué dont 1 à 3 atomes de cycle sont indépendamment l'azote, l'oxygène ou le soufre, -OR, -C(O)R, -C(O)OR, -C(O)NRR', -NRR', -S(O)₂R, -S(O)₂OR, et -S(O)₂NRR' ;
R² est indépendamment choisi dans l'ensemble constitué par alkyle en C₁ à C₁₀ substitué ou non substitué, alcényle en C₁ à C₁₀ substitué ou non substitué, alcynyle en C₁ à C₁₀ substitué ou non substitué, cycloalkyle en C₃ à C₈ substitué ou non substitué, alcoxy en C₁ à C₁₀ substitué ou non substitué, cycloalcoxy en C₃ à C₈ substitué ou non substitué, aryle en C₆ à C₁₄ substitué ou non substitué, hétéroaryle à 5 à 10 chaînons substitué ou non substitué dont 1 à 4 atomes de cycle sont indépendamment choisis parmi l'azote, l'oxygène et le soufre, un cycle hétéroalicyclique à 5 à 10 chaînons substitué ou non substitué dont 1 à 3 atomes de cycle sont indépendamment l'azote, l'oxygène ou le soufre, -OR, -C(O)R,-C(O)OR, -C(O)NRR', -NRR', -S(O)₂R, -S(O)₂OR, et -S(O)₂NRR' ;
R⁶ représente des substituants indépendamment choisis dans l'ensemble constitué par l'hydrogène, un halogène, alkyle, alcényle ou alcynyle en C₁ à C₄ substitué ou non substitué, hétéroaryle à 5 à 10 chaînons substitué ou non substitué dont 1 à 4 atomes de cycle sont indépendamment choisis parmi l'azote, l'oxygène et le soufre, un cycle hétéroalicyclique à 5 à 10 chaînons substitué ou non substitué dont 1 à 3 atomes de cycle sont indépendamment l'azote, l'oxygène ou le soufre, -OR, -NRR', -NO₂, alcoxy en C₁ à C₄ substitué ou non substitué,-C(O)R, -C(O)OR, -C(O)NRR', -S(O)₂R, -S(O)₂OR et -S(O)₂NRR' ; R⁶ en position méta du cycle aryle étant un groupe tétrazolyle, un acide sulfonique ou un acide carboxylique ou un sel sodique ou potassique respectif ;
Y est choisi dans l'ensemble constitué par NH, O, S, CH₂, CH₂CH₂, C(O), C(O)O et S(O)₂O ;
R⁷ est un aryle en C₆ à C₁₄ non substitué ;
R¹² représente un à quatre substituants indépendamment choisis dans l'ensemble constitué par l'hydrogène, un halogène, alkyle, alcényle ou alcynyle en C₁ à C₄ substitué ou non substitué, un hétéroaryle à 5 à 10 chaînons substitué ou non substitué dont 1 à 4 atomes de cycle sont indépendamment choisis parmi l'azote, l'oxygène et le soufre, un cycle hétéroalicyclique à 5 à 10 chaînons substitué ou non substitué dont 1 à 3 atomes de cycle sont indépendamment l'azote, l'oxygène ou le soufre, -OR, -NRR', -NO₂, alcoxy en C₁ à C₄ substitué ou non substitué, -C(O)R, -C(O)OR, -C(O)NRR', -S(O)₂R, -S(O)₂OR et -S(O)₂NRR' ; et
R et R' sont indépendamment choisis dans l'ensemble constitué par l'hydrogène et un alkyle en C₁ à C₄ non substitué ;
ou un sel pharmaceutiquement acceptable d'un tel composé,
dans lequel, si l'alkyle en C₁ à C₁₀ est substitué, le ou les groupes substituants sont un ou plusieurs, par exemple un ou deux groupes, individuellement choisis dans l'ensemble constitué par cycloalkyle en C₃ à C₈, aryle en C₆ à C₁₄, hétéroaryle à 5 à 10 chaînons dont 1 à 4 atomes de cycle sont indépendamment choisis parmi l'azote, l'oxygène et le soufre, hétéroalicyclique à 5 à 10 chaînons dont 1 à 3 atomes de cycle sont indépendamment l'azote, l'oxygène ou le soufre, hydroxy, alcoxy en C₁ à C₁₀, cycloalcoxy en C₃ à C₈, aryloxy, mercapto, alkylthio, arylthio, cyano, halogéno, carbonyle, thiocarbonyle, O-carbamyle, N-carbamyle, O-thiocarbamyle, N-thiocarbamyle, C-amido, N-amido, C-carboxy, O-carboxy, nitro, silyle, sulfinyle, sulfonyle, amino, et -NR¹⁰R¹¹ où R¹⁰ et R¹¹ sont indépendamment choisis dans l'ensemble constitué par l'hydrogène, alkyle en C₁ à C₄, cycloalkyle en C₃ à C₈, aryle en C₆ à C₁₄, carbonyle, acétyle, sulfonyle, amino, et trifluorométhanesulfonyle, ou bien R¹⁰ et R¹¹, conjointement avec l'atome d'azote auquel ils sont rattachés, se combinent pour former un cycle hétéroalicyclique à cinq ou six chaînons,
dans lequel, si le cycloalkyle en C₃ à C₈ est substitué, le ou les groupes substituants sont un ou plusieurs, par exemple un ou deux groupes, individuellement choisis parmi alkyle en C₁ à C₁₀, cycloalkyle en C₃ à C₈, aryle en C₆ à C₁₄, hétéroaryle à 5 à 10 chaînons dont 1 à 4 atomes de cycle sont indépendamment choisis parmi l'azote, l'oxygène et le soufre, hétéroalicyclique à 5 à 10 chaînons dont 1 à 3 atomes de cycle sont indépendamment l'azote, l'oxygène ou le soufre, hydroxy, alcoxy en C₁ à C₁₀, cycloalcoxy en C₃ à C₈, aryloxy, mercapto, alkylthio, arylthio, cyano, halogéno, carbonyle, thiocarbonyle, O-carbamyle, N-carbamyle, O-thiocarbamyle, N-thiocarbamyle, C-amido, N-amido, C-carboxy, O-carboxy, nitro, silyle, sulfinyle, sulfonyle, amino, et NR¹⁰R¹¹ où R¹⁰ et R¹¹ sont tels que définis ci-dessus,
dans lequel, si l'aryle en C₆ à C₁₄ est substitué, le ou les groupes substituants sont un ou plusieurs, par exemple un, deux ou trois substituants, indépendamment choisis dans l'ensemble constitué par alkyle en C₁ à C₁₀, cycloalkyle en C₃ à C₈, aryle en C₆ à C₁₄, hétéroaryle à 5 à 10 chaînons dont 1 à 4 atomes de cycle sont indépendamment choisis parmi l'azote, l'oxygène et le soufre, hétéroalicyclique à 5 à 10 chaînons dont 1 à 3 atomes de cycle sont indépendamment l'azote, l'oxygène ou le soufre, hydroxy, alcoxy en C₁ à C₁₀, cycloalcoxy en C₃ à C₈, aryloxy, mercapto, alkylthio, arylthio, cyano, halogéno, trihalogénométhyle, carbonyle, thiocarbonyle, O-carbamyle, N-carbamyle, O-thiocarbamyle, N-thiocarbamyle, C-amido, N-amido, C-carboxy, O-carboxy, nitro, silyle, sulfinyle, sulfonyle, amino, et NR¹⁰R¹¹ où R¹⁰ et R¹¹ sont tels que définis ci-dessus,
dans lequel, si l'hétéroaryle à 5 à 10 chaînons est substitué, le ou les groupes substituants sont un ou plusieurs, par exemple un ou deux substituants, indépendamment choisis dans l'ensemble constitué par alkyle en C₁ à C₁₀, cycloalkyle en C₃ à C₈, aryle en C₆ à C₁₄, hétéroaryle à 5 à 10 chaînons dont 1 à 4 atomes de cycle sont indépendamment choisis parmi l'azote, l'oxygène et le soufre, hétéroalicyclique à 5 à 10 chaînons dont 1 à 3 atomes de cycle sont indépendamment l'azote, l'oxygène ou le soufre, hydroxy, alcoxy en C₁ à C₁₀, cycloalcoxy en C₃ à C₈, aryloxy, mercapto, alkylthio, arylthio, cyano, halogéno, trihalogénométhyle, carbonyle, thiocarbonyle, O-carbamyle, N-carbamyle, O-thiocarbamyle, N-thiocarbamyle, C-amido, N-amido, C-carboxy, O-carboxy, nitro, silyle, sulfinyle, sulfonyle, amino, et -NR¹⁰R¹¹ où R¹⁰ et R¹¹ sont tels que définis ci-dessus, et
dans lequel, si le cycle hétéroalicyclique à 5 à 10 chaînons est substitué, le ou les groupes substituants sont un ou plusieurs, par exemple un, deux ou trois substituants, indépendamment choisis dans l'ensemble constitué par alkyle en C₁ à C₁₀, cycloalkyle en C₃ à C₈, aryle en C₆ à C₁₄, hétéroaryle à 5 à 10 chaînons dont 1 à 4 atomes de cycle sont indépendamment choisis parmi l'azote, l'oxygène et le soufre, hétéroalicyclique à 5 à 10 chaînons dont 1 à 3 atomes de cycle sont indépendamment l'azote, l'oxygène ou le soufre, hydroxy, alcoxy en C₁ à C₁₀, cycloalcoxy en C₃ à C₈, aryloxy, mercapto, alkylthio, arylthio, cyano, halogéno, trihalogénométhyle, carbonyle, thiocarbonyle, O-carbamyle, N-carbamyle, O-thiocarbamyle, N-thiocarbamyle, C-amido, N-amido, C-carboxy, O-carboxy, nitro, silyle, sulfinyle, sulfonyle, amino, et -NR¹⁰R¹¹ où R¹⁰ et R¹¹ sont tels que définis ci-dessus ;
(B) choisi dans l'ensemble constitué par : 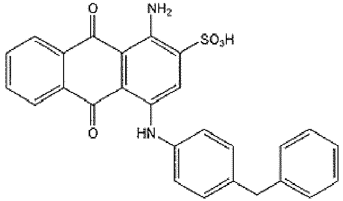 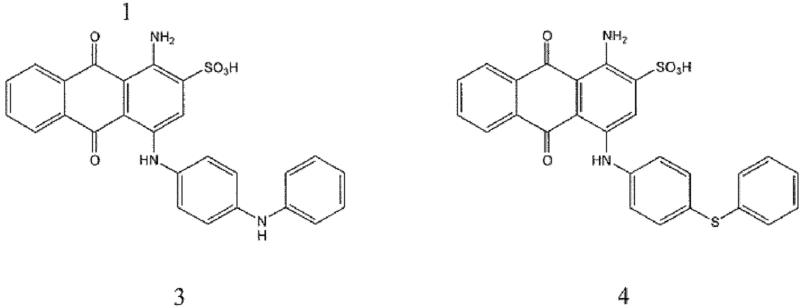 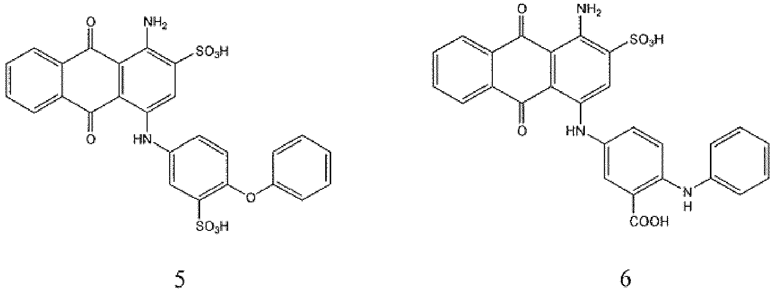 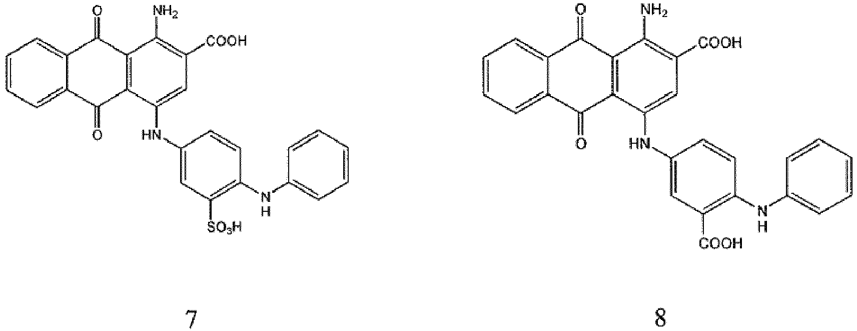 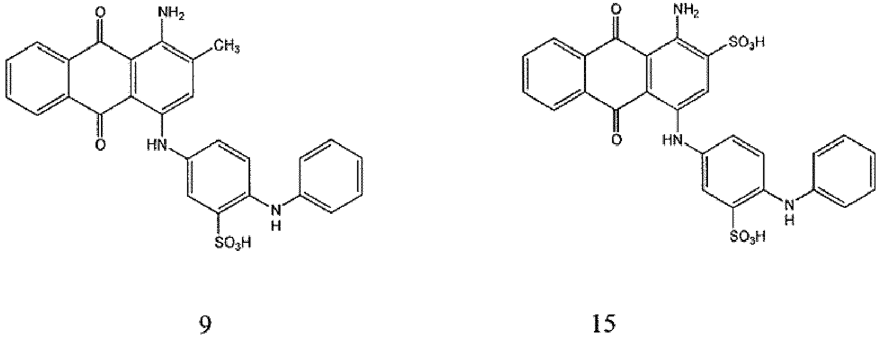 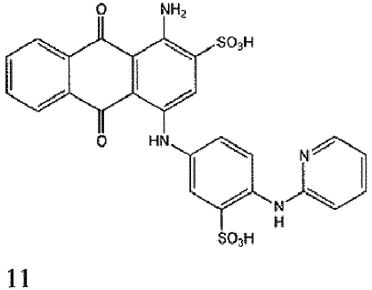 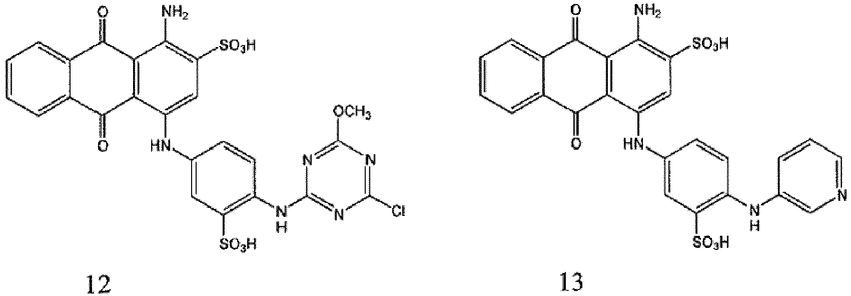 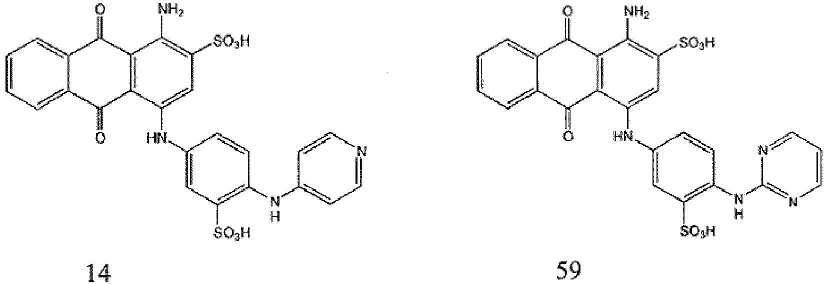 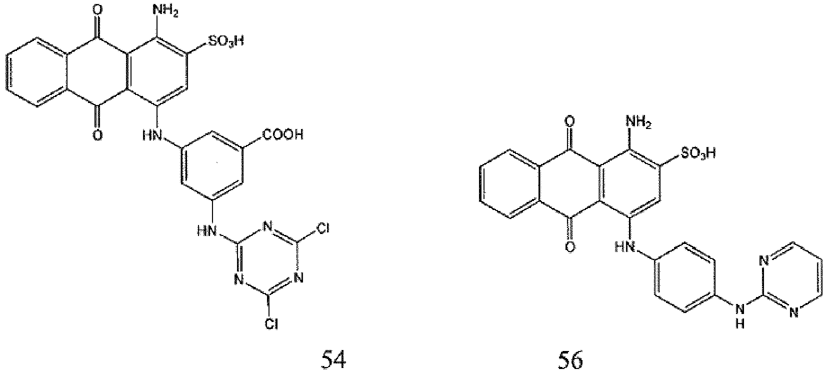 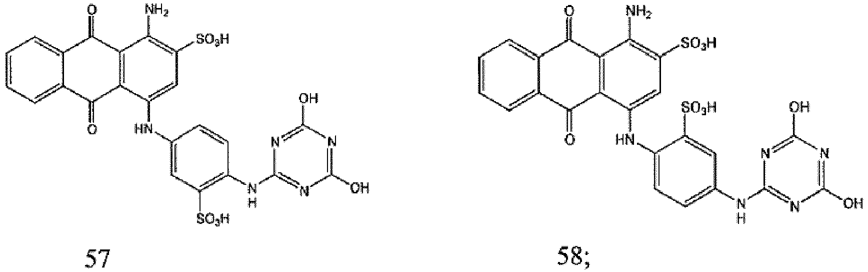 ou leurs sels pharmaceutiquement acceptables ; ou
(C) choisi dans l'ensemble constitué par : 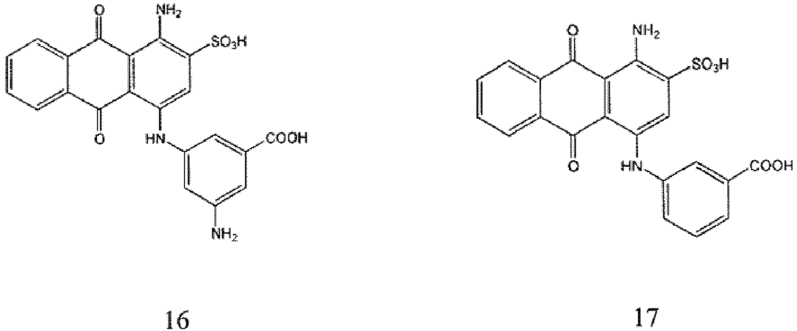 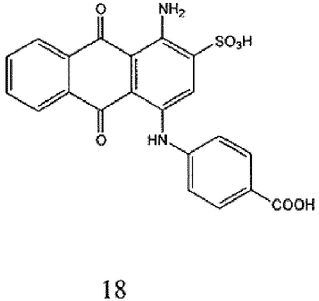 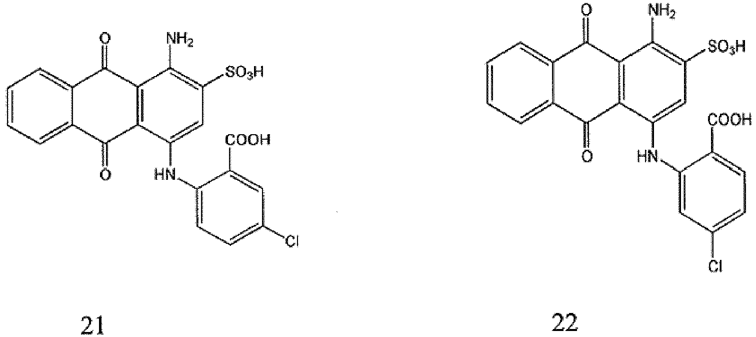 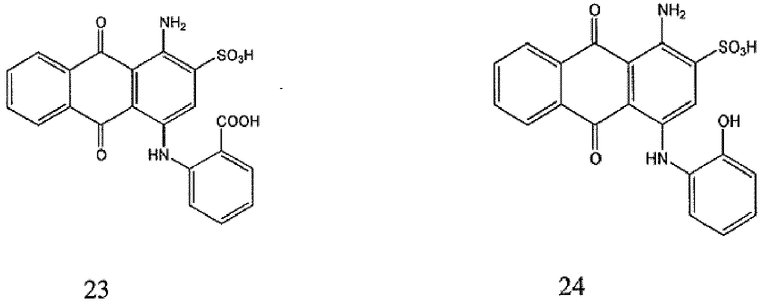 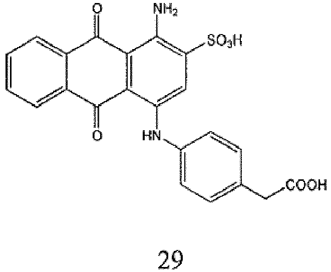 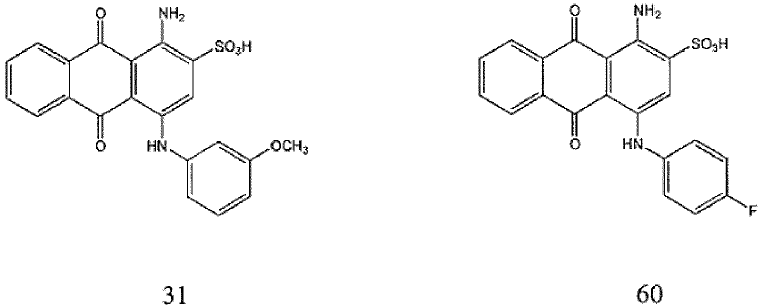 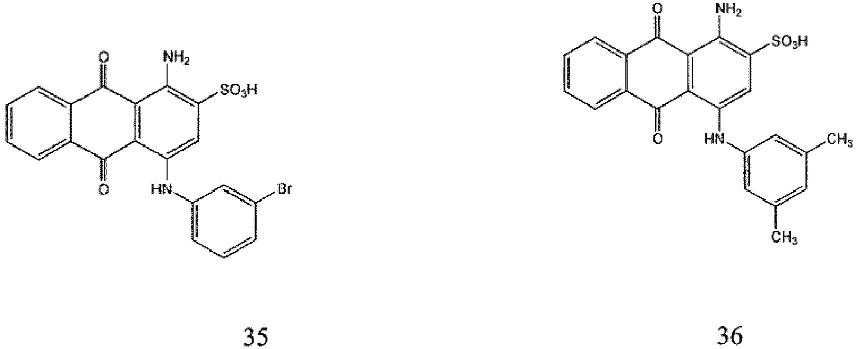 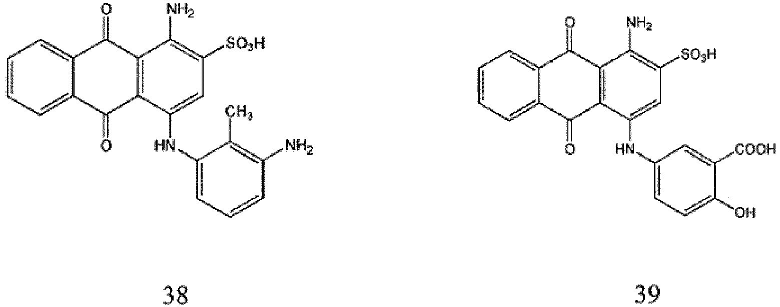 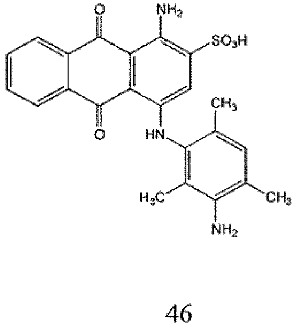 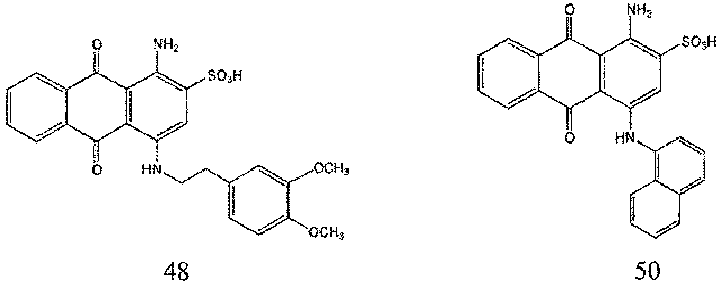 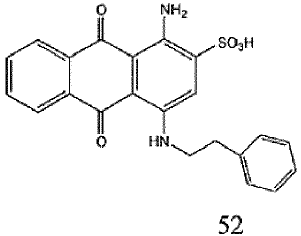 ou leurs sels pharmaceutiquement acceptables.

2. Composé selon la revendication 1, dans lequel R⁷ est un aryle en C₆ non substitué.

3. Composé selon la revendication 1 ou 2, dans lequel Y est NH et/ou dans lequel R⁶ représente des substituants choisis dans l'ensemble constitué par l'hydrogène,-S(O)₂OR, -C(O)OR, tétrazolyle, sulfonyle, carboxy, amino, alcoxy en C₁ à C₄, alkyle en C₁ à C₄, hydroxy, et nitro.

4. Composé selon l'une quelconque des revendications 1 à 3, dans lequel
(1) R¹² représente un à quatre substituants choisis dans l'ensemble constitué par l'hydrogène, halogéno, amino, alkyle ou alcoxy en C₁ à C₄ non substitué, et hydroxy ; et/ou
(2) au moins l'un parmi A et B est C=O ou bien A et B sont tous deux C=O ; et/ou
(3) R¹ est amino ; et/ou
(4) R² est tétrazolyle, -S(O)₂OR ou -C(O)OR.

5. Composition pharmaceutique comprenant un composé ou sel selon l'une quelconque des revendications 1 à 4 et un véhicule ou excipient pharmaceutiquement acceptable et comprenant éventuellement en outre un médicament ou un agent pharmaceutique additionnel.

6. Composé selon l'une quelconque des revendications 1 (A) ou (B) à 4 ou un sel pharmaceutiquement acceptable de celui-ci, pour une utilisation dans un procédé d'inhibition de l'agrégation plaquettaire ou pour une utilisation dans la prévention, le soulagement et/ou le traitement d'une maladie ou d'un trouble lié à l'activité du récepteur P2Y₁₂.

7. Composé de formule III : 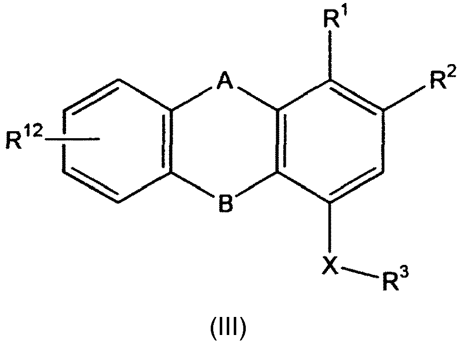 dans laquelle :
A et B sont C=O ;
XestNH;
R¹ est -NH₂ ;
R² est choisi dans l'ensemble constitué par -C(O)OH et -S(O)₂OH ;
R³ est choisi dans l'ensemble constitué par alkyle en C₁ à C₁₀ substitué ou non substitué, alcényle en C₁ à C₁₀ substitué ou non substitué, alcynyle en C₁ à C₁₀ substitué ou non substitué, cycloalkyle en C₃ à C₈ substitué ou non substitué, alcoxy en C₁ à C₁₀ substitué ou non substitué, cycloalcoxy en C₃ à C₈ substitué ou non substitué, aryle en C₆ à C₁₄ substitué ou non substitué, hétéroaryle à 5 à 10 chaînons substitué ou non substitué dont 1 à 4 atomes de cycle sont indépendamment choisis parmi l'azote, l'oxygène et le soufre, un cycle hétéroalicyclique à 5 à 10 chaînons substitué ou non substitué dont 1 à 3 atomes de cycle sont indépendamment l'azote, l'oxygène ou le soufre, alkylaryle, alkylhétéroaryle, un cycle hétérocyclique ou alkyle bicyclique à 7 à 12 chaînons substitué ou non substitué dont 1 à 3 chaînons de cycle sont indépendamment l'azote, l'oxygène ou le soufre, un cycle hétérocyclique ou alkyle tricyclique à 10 à 16 chaînons substitué ou non substitué dont 1 à 3 chaînons de cycle sont indépendamment l'azote, l'oxygène ou le soufre, 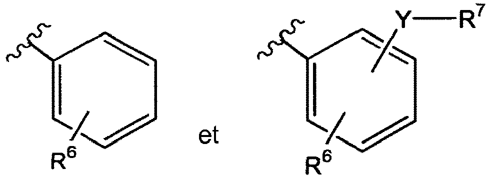
R⁶ représente un à quatre substituants indépendamment choisis dans l'ensemble constitué par l'hydrogène, un halogène, alkyle, alcényle ou alcynyle en C₁ à C₄ substitué ou non substitué, hétéroaryle à 5 à 10 chaînons substitué ou non substitué dont 1 à 4 atomes de cycle sont indépendamment choisis parmi l'azote, l'oxygène et le soufre, un cycle hétéroalicyclique à 5 à 10 chaînons substitué ou non substitué dont 1 à 3 atomes de cycle sont indépendamment l'azote, l'oxygène ou le soufre, -OR, -NRR', -NO₂, alcoxy en C₁ à C₄ substitué ou non substitué, -C(O)R, -C(O)OR, -C(O)NRR', -S(O)₂R, -S(O)₂OR et -S(O)₂NRR' ;
Y est choisi dans l'ensemble constitué par l'hydrogène, un halogène, NH, O, S, CH₂, CH₂CH₂, C(O), C(O)O, S(O)₂O, ou alcoxy en C₁ à C₄ non substitué et, quand Y est l'hydrogène, un halogène ou un alcoxy, alors R⁷ est manquant ;
R⁷ est choisi dans l'ensemble constitué par l'hydrogène, un halogène, -OR, alkyle, alcényle ou alcynyle en C₁ à C₁₀ substitué ou non substitué, alcoxy en C₁ à C₁₀ substitué ou non substitué, cycloalcoxy en C₃ à C₈ substitué ou non substitué, cycloalkyle en C₃ à C₈ substitué ou non substitué, aryle en C₆ à C₁₄ substitué ou non substitué, hétéroaryle à 5 à 10 chaînons substitué ou non substitué dont 1 à 4 atomes de cycle sont indépendamment choisis parmi l'azote, l'oxygène et le soufre, hétéroalicyclique à 5 à 10 chaînons substitué ou non substitué dont 1 à 3 atomes de cycle sont indépendamment l'azote, l'oxygène ou le soufre, -C(O)R,-C(O)OR, -C(O)NRR', -NRR', -S(O)₂R, -S(O)₂OR, -S(O)₂NRR', et -P(O)RR' ;
R¹² représente un à quatre substituants indépendamment choisis dans l'ensemble constitué par l'hydrogène, un halogène, alkyle, alcényle ou alcynyle en C₁ à C₄ substitué ou non substitué, hétéroaryle à 5 à 10 chaînons substitué ou non substitué dont 1 à 4 atomes de cycle sont indépendamment choisis parmi l'azote, l'oxygène et le soufre, un cycle hétéroalicyclique à 5 à 10 chaînons substitué ou non substitué dont 1 à 3 atomes de cycle sont indépendamment l'azote, l'oxygène ou le soufre, -OR, -NRR', -NO₂, alcoxy en C₁ à C₄ substitué ou non substitué, -C(O)R, -C(O)OR, -C(O)NRR', -S(O)₂R, -S(O)₂OR et -S(O)₂NRR' ; et
R et R' sont indépendamment choisis dans l'ensemble constitué par l'hydrogène et alkyle en C₁ à C₄ non substitué ;
ou un sel pharmaceutiquement acceptable de celui-ci, pour une utilisation dans la prévention, le soulagement et/ou le traitement d'une maladie ou d'un trouble lié à l'activité du récepteur P2Y₁₂,
dans lequel, si l'alkyle en C₁ à C₁₀ est substitué, le ou les groupes substituants sont un ou plusieurs, par exemple un ou deux groupes, individuellement choisis dans l'ensemble constitué par cycloalkyle en C₃ à C₈, aryle en C₆ à C₁₄, hétéroaryle à 5 à 10 chaînons dont 1 à 4 atomes de cycle sont indépendamment choisis parmi l'azote, l'oxygène et le soufre, hétéroalicyclique à 5 à 10 chaînons dont 1 à 3 atomes de cycle sont indépendamment l'azote, l'oxygène ou le soufre, hydroxy, alcoxy en C₁ à C₁₀, cycloalcoxy en C₃ à C₈, aryloxy, mercapto, alkylthio, arylthio, cyano, halogéno, carbonyle, thiocarbonyle, O-carbamyle, N-carbamyle, O-thiocarbamyle, N-thiocarbamyle, C-amido, N-amido, C-carboxy, O-carboxy, nitro, silyle, sulfinyle, sulfonyle, amino, et -NR¹⁰R¹¹ où R¹⁰ et R¹¹ sont indépendamment choisis dans l'ensemble constitué par l'hydrogène, alkyle en C₁ à C₄, cycloalkyle en C₃ à C₈, aryle en C₆ à C₁₄, carbonyle, acétyle, sulfonyle, amino, et trifluorométhanesulfonyle, ou bien R¹⁰ et R¹¹, conjointement à l'atome d'azote auquel ils sont rattachés, se combinent pour former un cycle hétéroalicyclique à cinq ou six chaînons, dans lequel, si le cycloalkyle en C₃ à C₈ est substitué, le ou les groupes substituants sont un ou plusieurs, par exemple un ou deux groupes, individuellement choisis parmi alkyle en C₁ à C₁₀, cycloalkyle en C₃ à C₈, aryle en C₆ à C₁₄, hétéroaryle à 5 à 10 chaînons dont 1 à 4 atomes de cycle sont indépendamment choisis parmi l'azote, l'oxygène et le soufre, hétéroalicyclique à 5 à 10 chaînons dont 1 à 3 atomes de cycle sont indépendamment l'azote, l'oxygène ou le soufre, hydroxy, alcoxy en C₁ à C₁₀, cycloalcoxy en C₃ à C₈, aryloxy, mercapto, alkylthio, arylthio, cyano, halogéno, carbonyle, thiocarbonyle, O-carbamyle, N-carbamyle, O-thiocarbamyle, N-thiocarbamyle, C-amido, N-amido, C-carboxy, O-carboxy, nitro, silyle, sulfinyle, sulfonyle, amino, et NR¹⁰R¹¹ où R¹⁰ et R¹¹ sont tels que définis ci-dessus,
dans lequel, si l'aryle en C₆ à C₁₄ est substitué, le ou les groupes substituants sont un ou plusieurs, par exemple un, deux ou trois substituants, indépendamment choisis dans l'ensemble constitué par alkyle en C₁ à C₁₀, cycloalkyle en C₃ à C₈, aryle en C₆ à C₁₄, hétéroaryle à 5 à 10 chaînons dont 1 à 4 atomes de cycle sont indépendamment choisis parmi l'azote, l'oxygène et le soufre, hétéroalicyclique à 5 à 10 chaînons dont 1 à 3 atomes de cycle sont indépendamment l'azote, l'oxygène ou le soufre, hydroxy, alcoxy en C₁ à C₁₀, cycloalcoxy en C₃ à C₈, aryloxy, mercapto, alkylthio, arylthio, cyano, halogéno, trihalogénométhyle, carbonyle, thiocarbonyle, O-carbamyle, N-carbamyle, O-thiocarbamyle, N-thiocarbamyle, C-amido, N-amido, C-carboxy, O-carboxy, nitro, silyle, sulfinyle, sulfonyle, amino, et NR¹⁰R¹¹ où R¹⁰ et R¹¹ sont tels que définis ci-dessus,
dans lequel, si l'hétéroaryle à 5 à 10 chaînons est substitué, le ou les groupes substitué sont un ou plusieurs, par exemple un ou deux substituants, indépendamment choisis dans l'ensemble constitué par alkyle en C₁ à C₁₀, cycloalkyle en C₃ à C₈, aryle en C₆ à C₁₄, hétéroaryle à 5 à 10 chaînons dont 1 à 4 atomes de cycle sont indépendamment choisis parmi l'azote, l'oxygène et le soufre, hétéroalicyclique à 5 à 10 chaînons dont 1 à 3 atomes de cycle sont indépendamment l'azote, l'oxygène ou le soufre, hydroxy, alcoxy en C₁ à C₁₀, cycloalcoxy en C₃ à C₈, aryloxy, mercapto, alkylthio, arylthio, cyano, halogéno, trihalogénométhyle, carbonyle, thiocarbonyle, O-carbamyle, N-carbamyle, O-thiocarbamyle, N-thiocarbamyle, C-amido, N-amido, C-carboxy, O-carboxy, nitro, silyle, sulfinyle, sulfonyle, amino, et -NR¹⁰R¹¹ où R¹⁰ et R¹¹ sont tels que définis ci-dessus, et
dans lequel, si le cycle hétéroalicyclique à 5 à 10 chaînons est substitué, le ou les groupes substituants sont un ou plusieurs, par exemple un, deux ou trois substituants, indépendamment choisis dans l'ensemble constitué par alkyle en C₁ à C₁₀, cycloalkyle en C₃ à C₈, aryle en C₆ à C₁₄, hétéroaryle à 5 à 10 chaînons dont 1 à 4 atomes de cycle sont indépendamment choisis parmi l'azote, l'oxygène et le soufre, hétéroalicyclique à 5 à 10 chaînons dont 1 à 3 atomes de cycle sont indépendamment l'azote, l'oxygène ou le soufre, hydroxy, alcoxy en C₁ à C₁₀, cycloalcoxy en C₃ à C₈, aryloxy, mercapto, alkylthio, arylthio, cyano, halogéno, trihalogénométhyle, carbonyle, thiocarbonyle, O-carbamyle, N-carbamyle, O-thiocarbamyle, N-thiocarbamyle, C-amido, N-amido, C-carboxy, O-carboxy, nitro, silyle, sulfinyle, sulfonyle, amino, et -NR¹⁰R¹¹ où R¹⁰ et R¹¹ sont tels que définis ci-dessus.

8. Composé pour une utilisation selon la revendication 7, qui est choisi dans l'ensemble constitué par : 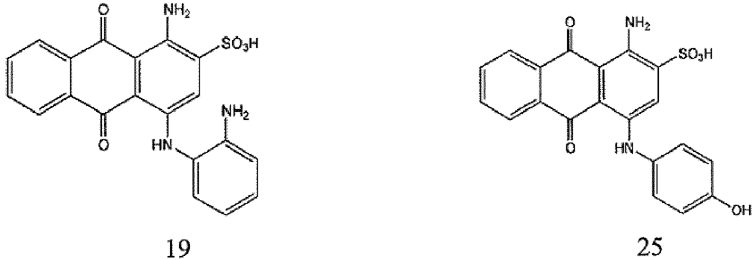 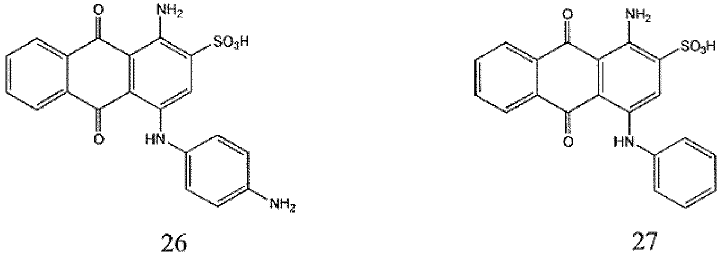 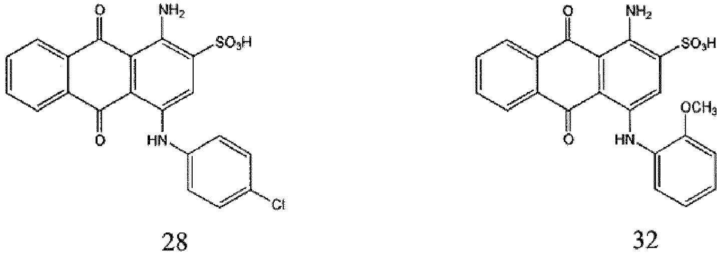 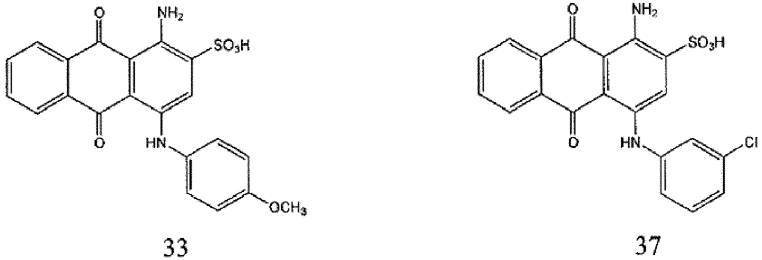 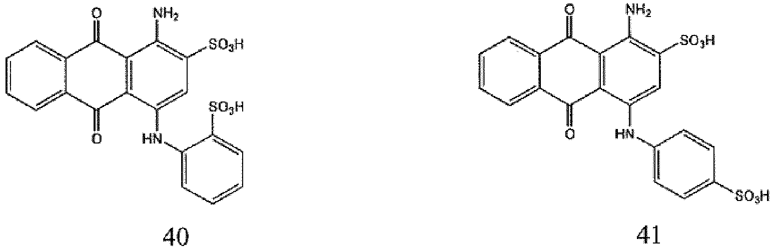 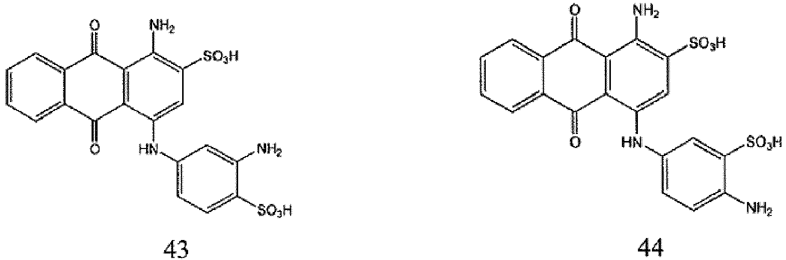 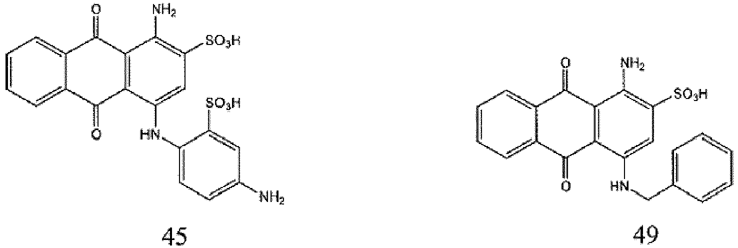 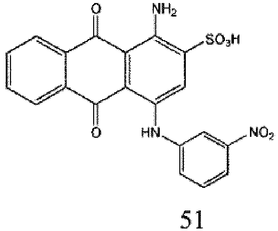 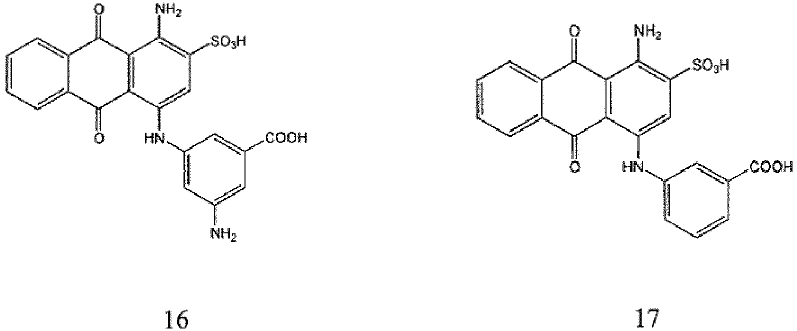 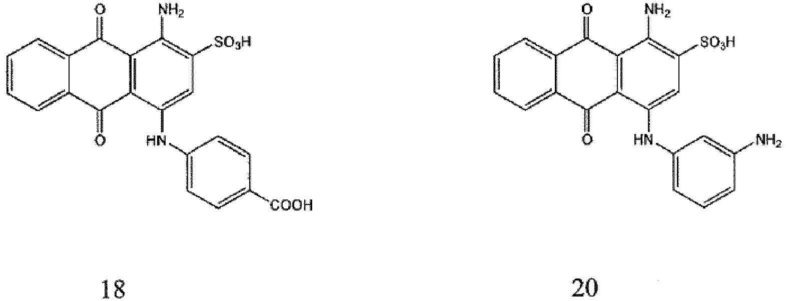 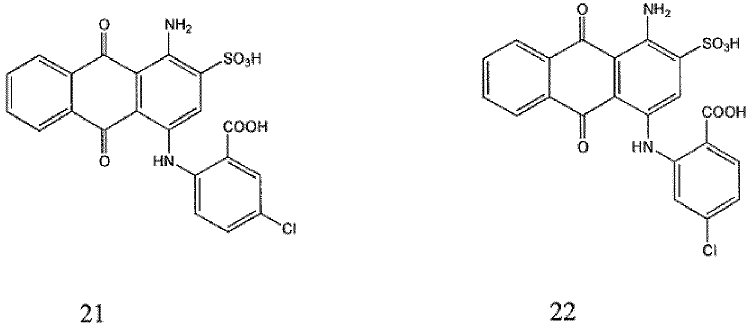 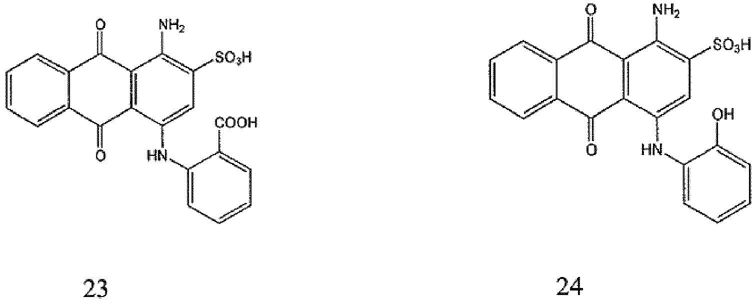 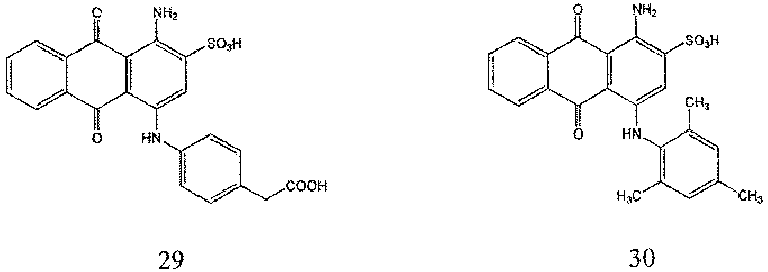 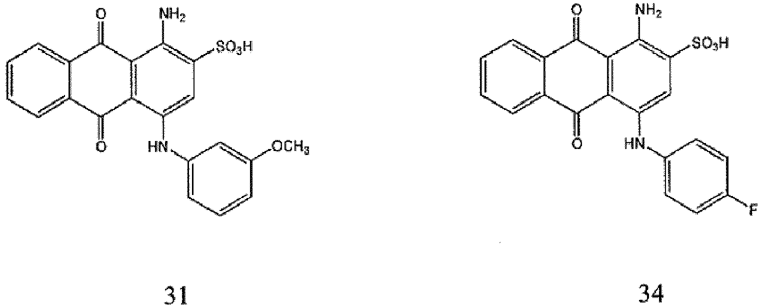 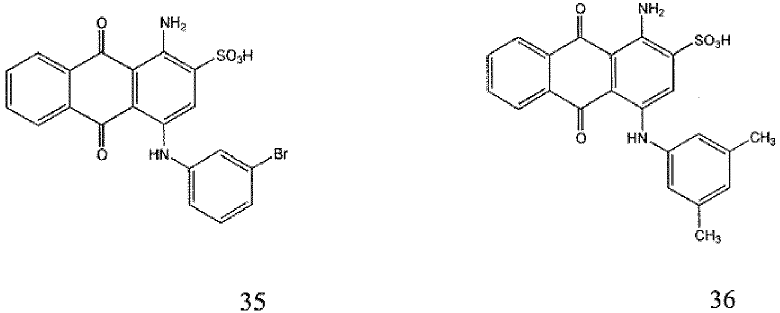 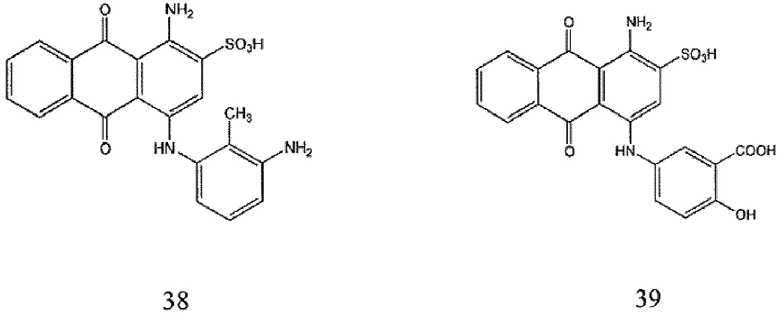 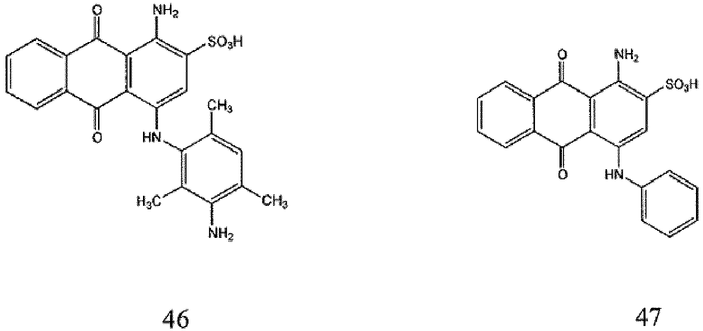 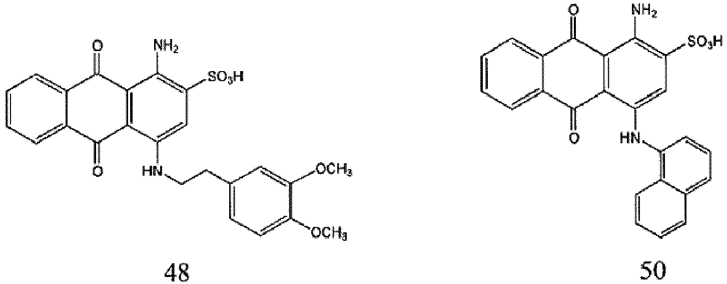 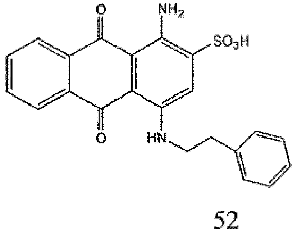 ou leurs sels pharmaceutiquement acceptables.

9. Composé pour une utilisation selon l'une quelconque des revendications 6 à 8, dans lequel la maladie ou le trouble est choisi dans l'ensemble constitué par un accident vasculaire, un infarctus du myocarde, une ischémie myocardique, les maladies vasculaires, une maladie coronarienne, une maladie artérielle périphérique, l'athérosclérose, une maladie cérébrovasculaire, les embolies, et les troubles du SNC.

10. Procédé in vitro pour réaliser un effet antagoniste de la fonction du récepteur P2Y₁₂, comprenant l'étape de mise en contact de cellules exprimant un récepteur P2Y₁₂ avec au moins un composé tel que défini dans l'une quelconque des revendications 1 à 4 et 6 à 8.
